# EUROPEAN PATENT SPECIFICATION

(11) **EP 2 831 060 B1**
(45) Date of publication and mention of the grant of the patent: **04.05.2016**
(21) Application number: 13712263.6
(22) Date of filing: 26.03.2013
(51) Int. Cl.: C07D 401/14, C07D 413/14, A61K 31/506, C07D 403/14, C07D 409/14, C07D 417/04, C07D 417/14, A61P 31/12

(54) **NOVEL 4-METHYL-DIHYDROPYRIMIDINES FOR THE TREATMENT AND PROPHYLAXIS OF HEPATITIS B VIRUS INFECTION**
NEUARTIGE 4-METHYL-DIHYDROPYRIMIDINE ZUR BEHANDLUNG UND PROPHYLAXE VON HEPATITIS-B-VIRUS-INFEKTIONEN
NOUVEAUX 4-MÉTHYL-DIHYDROPYRIMIDINES POUR LE TRAITEMENT ET LA PROPHYLAXIE DU VIRUS DE L'HÉPATITE B

(30) Priority: 31.03.2012 WO PCT/CN2012/073388; 08.02.2013 WO PCT/CN2013/071575
(43) Date of publication of application: 04.02.2015
(73) Proprietor: F.Hoffmann-La Roche AG, 4070 Basel (CH)
(72) Inventor: GUO, Lei, Shanghai 200122 (CN); LIN, Xianfeng, Shanghai 201203 (CN); LIU, Haixia, Shanghai 201203 (CN); QIU, Zongxing, Shanghai 200333 (CN); SHEN, Hong, Shanghai 201203 (CN); TANG, Guozhi, Shanghai 201314 (CN); WU, Guolong, Shanghai 201318 (CN); ZHANG, Weixing, Shanghai 201100 (CN); ZHU, Wei, Shanghai 200235 (CN)
(74) Representative: Zhuang Plodeck, Jianping
(86) International application number: PCT/EP2013/056371
(87) International publication number: WO 2013/144129

(56) References cited:
- WO-A1-01/68640
- WO-A1-99/01438
- WO-A1-2013/019967
- DERES KARL ET AL: "Inhibition of hepatitis B virus replication by drug-induced depletion of nucleocapsids", SCIENCE, AMERICAN ASSOCIATION FOR THE ADVANCEMENT OF SCIENCE, US, vol. 299, no. 5608, 7 February 2003 (2003-02-07), pages 893-896, XP009100473, ISSN: 1095-9203, DOI: 10.1126/SCIENCE.1077215

## Description

The present invention relates to organic compounds useful for therapy and/or prophylaxis in a human, and in particular to Hepatitis B virus (HBV) inhibitors by targeting on HBV capsid useful for treating HBV infection.

### FIELD OF THE INVENTION

HBV is a species of the hepadnaviridae family of viruses. HBV is a serious public health problem worldwide, with more than 400 million people especially in Asia-pacific regions chronically infected by this small enveloped DNA virus. Although most individuals seem to resolve the infection following acute symptoms, 15-40% of HBV patients will finally develop clinical diseases during their lifespan, most notably, hepatitis, liver cirrhosis, and hepatocellular carcinoma. Every year 500,000 to 1 million people die from the end stage of liver diseases caused by HBV infection.

HBV lifecycle begins with the binding of the "Dane" particle with an unidentified receptor on the surface of hepatocyte. Following entry, viral genome is delivered into nucleus where a covalently closed circular DNA (cccDNA) is formed through DNA repair of viral relaxed circular DNA. Unlike the mechanisms of most other DNA viruses, HBV cccDNA replicates through the retrotranscription of a 1.1-genome unit-length RNA copy (pregenomic RNA). Viral pregenomic RNA interacts with other two viral components, capsid protein and polymerase, as well as some host factors, to form capsid particles where viral DNA replication occurs. Most copies of the encapsidated genome then efficiently associate with the envelope proteins for virion assembly and secretion; a minority of these genomes are shunted to the nucleus, where they are converted to cccDNA.

Currently, there are two types of anti-HBV agents on the market, nucleoside (tide) analogs targeting viral polymerase (lamivudine, adefovir, tenofovir, telbivudine and entecavir) and interferon modulating host immune functions. Mutations in the primary sequence of the polymerase that confer resistance to lamivudine and adefovir have been identified clinically and underlie a rebound of serum virus titers that 70% of treated patients experience within 3 years of the start of lamivudine therapy. Although resistance to telbivudine, adefovir, and entecavir occurs more rarely, it has been recorded. Interferon alpha is the other major therapy available for hepatitis B, but it is limited by a poor long-term response and debilitating side effects. Some viral genotypes do not show good responses to interferon therapy. Now, the standard of clinic cure of HBV infection is the loss and/or seroconversion of HBsAg. The majority (around or more than 90%) of treated patients fail to achieve this goal. This drawback is mainly due to the presence of a stable pool of viral cccDNA in nucleus that doesn't replicate itself, therefore, shows no accessibility to nucleoside (tide) analogs.

Hence, there is certainly a medical need for treatments with improved characteristics, and for a diversity of approaches in the development of therapies for HBV infection.

HBV capsid protein plays essential roles in HBV replication. HBV has an icosahedral core comprising of 240 copies of the capsid (or core) protein. The predominant biological function of capsid protein is to act as a structural protein to encapsidate pre-genomic RNA and form immature capsid particles in the cytoplasm. This step is prerequisite for viral DNA replication. The HBV capsid spontaneously self-assembles from many copies of core dimers present in the cytoplasm. It has been shown that the formation of a trimeric nucleus and the subsequent elongation reactions occur by adding one dimeric subunit at a time until it is complete. Besides this function, capsid protein regulates viral DNA synthesis through different phosphorylation status of its C-terminal phosphorylation sites. When a near full-length relaxed circular DNA is formed through reverse-transcription of viral pregenomic RNA, an immature capsid becomes a mature capsid. On one hand, capsid protein might facilitate the nuclear translocation of viral relaxed circular genome by means of the nuclear localization signals located in the Arginine-rich domain of the C-terminal region of capsid protein. In nucleus, as a component of viral cccDNA minichromosome, capsid protein could play a structural and regulatory role in the functionality of cccDNA minichromosomes. Capsid protein also interacts with viral large envelope protein in endoplasmic reticulum and triggers the release of intact viral particles from hepatocytes.

There has been a couple of capsid related anti-HBV inhibitors reported. For example, phenylpropenamide derivatives, including compounds named AT-61 and AT-130 (Feld J. et al. Antiviral Research 2007, 168-177), and a class of thiazolidin-4-ones from Valeant R&D (WO2006/033995), have been shown to inhibit pgRNA packaging. A recent study suggested that phenylpropenamides are, in fact, accelerators of HBV capsid assembly, and their actions result in the formation of empty capsids. These very interesting results illustrate the importance of the kinetic pathway in successful virus assembly.

Heteroaryldihydropyrimidines or HAP, including compounds named Bay 41-4109, Bay 38-7690 and Bay 39-5493, were discovered in a tissue culture-based screening (Deres K. et al. Science 2003, 893). These HAP analogs act as synthetic allosteric activators and are able to induce aberrant capsid formation that leads to degradation of the core protein. HAP analogs also reorganized core protein from preassembled capsids into noncapsid polymers, presumably by interaction of HAP with dimers freed during capsid 'breathing', the transitory breaking of individual intersubunit bonds. Bay 41-4109 was administered to HBV infected transgenic mouse or humanized mouse models and demonstrated *in vivo* efficacy with HBV DNA reduction (Deres K. et al. Sciertce 2003, 893; Brezillon N. etal. PLoS ONE 2011, e25096.). It was also shown that bis- ANS, a small molecule that acts as a molecular 'wedge' and interferes with normal capsid-protein geometry and capsid formation (Zlotnick A. et al. J. Virol. 2002, 4848-4854).

International patent application WO01/68640 A1 describes dihydropyrimidines and the use thereof for treatment of hepatitis B infections.

### SUMMARY OF THE INVENTION

Objects of the present invention are novel compounds of formula I, their manufacture, medicaments based on a compound in accordance with the invention and their production as well as the use of compounds of formula I for the treatment or prophylaxis of HBV infection.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 1 to 4 carbon atoms, for example methyl, ethyl, propyl, isopropyl, 1-butyl, 2-butyl, *tert*-butyl and the like. Particular "C₁₋₆alkyl" groups are methyl, ethyl, isopropyl, *tert*-butyl*.*

The term "cycloalkyl", alone or in combination, refers to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular cycloalkyl groups are cyclopropyl, cyclopentyl and cyclohexyl.

The term "C₁₋₆alkoxy" alone or in combination signifies a group C₁₋₆alkyl-O-, wherein the "C₁₋₆alkyl" is as defined above; for example methoxy, ethoxy, propoxy, isopropoxy, *n*-butoxy, i-butoxy, 2-butoxy, *t*-butoxy and the like. Particular C₁₋₆alkoxy groups are methoxy and ethoxy and more particularly methoxy.

The term "C₂₋₆alkoxy" alone or in combination signifies a group C₂₋₆alkyl-O-, wherein the "C₂₋₆alkyl" alone or in combination signifies a saturated, linear- or branched chain alkyl group containing 2 to 6, particularly 2 to 4 carbon atoms; for example ethoxy, propoxy, isopropoxy, *n-*butoxy, *i*-butoxy, 2-butoxy, *t*-butoxy and the like.

The term "C₁₋₂alkoxy" alone or in combination refers to methoxy or ethoxy.

The term "C_{y}H_{2y}" alone or in combination signifies a saturated, linear- or branched chain alkyl group containing 1 to 6, particularly 1 to 4 carbon atoms.

The term "amino", alone or in combination, refers to primary (-NH₂), secondary (-NH-) or tertiary amino

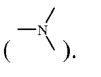

The term "carbonyl" alone or in combination refers to the group -C(O)-.

The term "carboxy" alone or in combination refers to the group -COOH.

The term "cyano" alone or in combination refers to the group -CN.

The term "halogen" means fluorine, chlorine, bromine or iodine. Halogen is particularly fluorine or chlorine, more particularly fluorine.

The term "hydroxy" alone or in combination refers to the group -OH.

The term "sulfonyl" alone or in combination refers to the group -S(O)₂-.

The term "morpholinyl" alone or in combination refers to the group

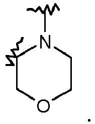

The term "pyrrolidinyl" alone or in combination refers to the group

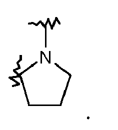

The term "piperidinyl" alone or in combination refers to the group

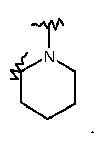

The term "tautomers isomers" refers to constitutional isomers of organic compounds that readily interconvert by a chemical reaction called tautomerization. This reaction commonly results in the formal migration of a hydrogen atom or proton, accompanied by a switch of a single bond and adjacent double bond. For example, compounds of general formular (I) 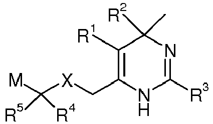 and its tautomers isomer

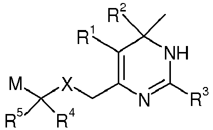

The term "pharmaceutically acceptable salt" refers to conventional acid-addition salts or base-addition salts that retain the biological effectiveness and properties of the compounds of formula I and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et. al., Organic Process Research & Development 2000, 4, 427-435; or in Ansel, H., et. al., In: Pharmaceutical Dosage Forms and Drug Delivery Systems, 6th ed. (1995), pp. 196 and 1456-1457. Particular are the sodium salts of the compounds of formula I.

Compounds of the general formula I which contain one or several chiral centers can either be present as racemates, diastereomeric mixtures, or optically active single isomers. The racemates can be separated according to known methods into the enantiomers. Particularly, diastereomeric salts which can be separated by crystallization are formed from the racemic mixtures by reaction with an optically active acid such as e.g. D- or L-tartaric acid, mandelic acid, malic acid, lactic acid or camphorsulfonic acid.

### INHIBITORS OF HEPATITIS B VIRUS

The present invention provides (i) novel compounds having the general formula I: 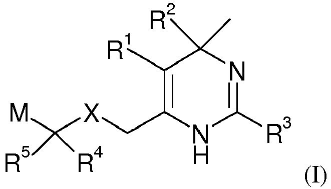 wherein
R¹ is C₁₋₂alkoxycarbonyl or cyano;
R² is phenyl, which is substituted by halogen;
R³ is thiazolyl, thienyl, imidazolyl, isoxazolyl or pyridinyl; which is unsubstituted or substituted by halogen or C₁₋₆alkyl;
X is oxygen or -NR⁷;
R⁴ and R⁵ are independently selected from hydrogen, C₁₋₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷, form a pyrrolidinyl, morpholinyl or piperidinyl ring, which ring is unsubstituted or substituted by fluoro;
M is C₁₋₆alkoxycarbonyl, carboxy, diC₁₋₆alkylaminoC₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 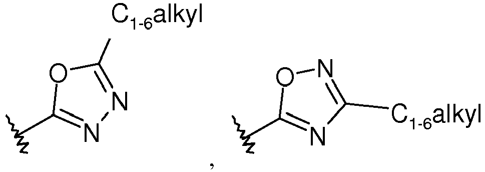 or 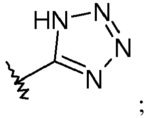
R⁷ is C₁₋₆alkyl or trifluoroC₁₋₆alkyl;
y is 1-6;
or pharmaceutically acceptable salts, or tautomerism isomers thereof.

Another embodiment of present invention is (ii) a compound of formula I, wherein
R¹ is C₁₋₂alkoxycarbonyl or cyano;
R² is phenyl, which is once or twice substituted by halogen;
R³ is 2-thiazolyl, which is unsubstituted or once substituted by C₁₋₆alkyl or halogen; or 2-thienyl or 2-pyridinyl, which is once substituted by halogen; or 2-imidazolyl, which is once substituted by C₁₋₆alkyl; or 3-isoxazolyl, which is unsubstituted or once substituted by C₁₋₆alkyl;
X is oxygen or -NR⁷;
R⁴ and R⁵ are independently selected from hydrogen, C₁₋₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷ together form a morpholinyl; or pyrrolidinyl or piperidinyl, which is substituted by fluoro;
M is C₁₋₆alkoxycarbonyl, carboxy, diC₁₋₆alkylamino-C₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 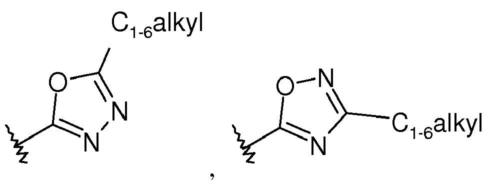 or 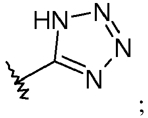
R⁷ is C₁₋₆alkyl or trifluoroC₁₋₆alkyl;
y is 1-6;
or pharmaceutically acceptable salts, or tautomerism isomers thereof.

Further embodiment of present invention is (iii) a compound of formula I, wherein
R¹ is methoxycarbonyl, ethoxycarbonyl or cyano;
R² is phenyl substituted once or twice by fluoro; 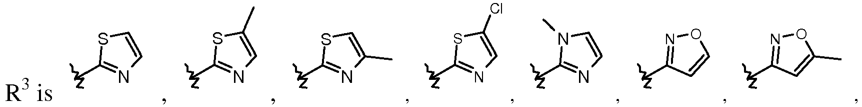 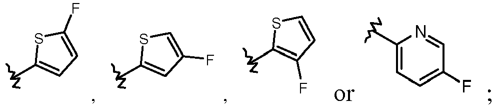
X is oxygen or -NR⁷;
R⁴ and R⁵ are independently selected from hydrogen, methyl and trifluoromethyl; or
R⁴ and R⁵ together with the carbon atom to which they are attached form cyclopropyl; or when X is -NR⁷, one of R⁴ and R⁵ is hydrogen or methyl, and the other one with the carbon atom to which it is attached and -NR⁷ form 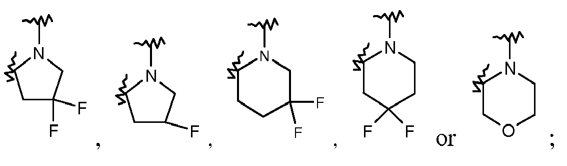
M is methoxycarbonyl, carboxy, dimethylaminoethoxycarbonyl, aminocarbonyl, dimethylaminocarbonyl, methylaminocarbonyl, methylsulfonylaminocarbonyl, 2-thiazolylaminocarbonyl, hydroxymethyl, hydroxypropyl, 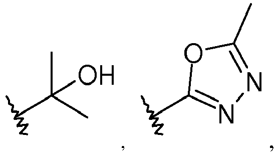 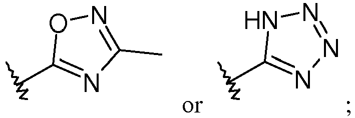
R⁷ is methyl or trifluoroethyl;
or pharmaceutically acceptable salts, or tautomerism isomers thereof.

Another embodiment of present invention is (iv) a compound of formula I or a pharmaceutically acceptable salt or tautomerism isomers thereof, wherein
R¹ is Cₗ₋₂alkoxycarbonyl;
R² is phenyl which is once substituted by halogen;
R³ is 2-thiazolyl;
X is oxygen;
R⁴ and R⁵ are independently selected from hydrogen, C₁₋₆alkyl and trifluoroC₁₋₆alkyl;
M is C₁₋₆alkoxycarbonyl or carboxy.

Further embodiment of present invention is (v) a compound of formula I or a pharmaceutically acceptable salt or tautomerism isomers thereof, wherein
R¹ is methyoxycarbonyl;
R² is 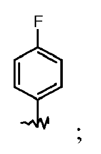
R³ is 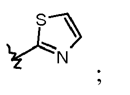
X is oxygen;
R⁴ and R⁵ are independently selected from hydrogen, methyl and trifluoromethyl;
M is methoxycarbonyl or carboxy.

Another embodiment of present invention is (vi) a compound of formula I or a pharmaceutically acceptable salt or tautomerism isomers thereof, wherein
R¹ is C₁₋₂alkoxycarbonyl;
R² is phenyl which is once substituted by halogen;
R³ is 2-thiazolyl;
X is -N-C₁₋₆alkyl or -N-trifluoroC₁₋₆alkyl;
R⁴ is hydrogen;
R⁵ is hydrogen;
or R⁴ and R⁵ together with the carbon atom to which they are attached, form a 3 to 7 membered cycloalkyl;
M is carboxy.

Further embodiment of present invention is (vii) a compound of formula I or a pharmaceutically acceptable salt or tautomerism isomers thereof, wherein
R¹ is methoxycarbonyl;
R² is 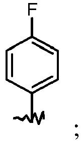
R³ is 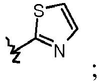
X is -NCH₃ or 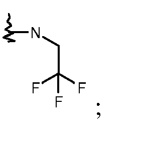
R⁴ is hydrogen;
R⁵ is hydrogen;
or R⁴ and R ⁵ together with the carbon atom to which they are attached, form cyclopropyl;
M is carboxy.

Another embodiment of present invention is (viii) a compound of formula I or a pharmaceutically acceptable salt or tautomerism isomers thereof, wherein
R¹ is C₁₋₂alkoxycarbonyl or cyano;
R² is phenyl which is once or twice substituted by halogen;
R³ is 2-thiazolyl; or 2-pyridinyl, which is once substituted by halogen; or 2-imidazolyl, which is once substituted by C₁₋₆alkyl;
X is -NR⁷;
one of R⁴ and R⁵ is hydrogen, and the other one with the carbon atom to which it is attached and -NR⁷ together form a morpholinyl;
M is C₁₋₆alkoxycarbonyl, carboxy or hydroxy-C_{y}H_{2y}-;
y is 1-6.

Further embodiment of present invention is (ix) a compound of formula I or a pharmaceutically acceptable salt or tautomerism isomers thereof, wherein
R¹ is methoxycarbonyl, ethoxycarbonyl or cyano;
R² is 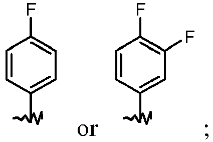
R³ is 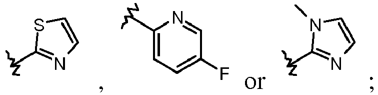
one of R⁴ and R⁵ is hydrogen, and the other one with the carbon atom to which it is attached and -NR⁷ form 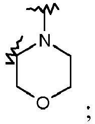
M is methoxycarbonyl, carboxy or hydroxymethyl-.

Another embodiment of present invention is (x) a compound of formula I or a pharmaceutically acceptable salt or tautomerism isomers thereof, wherein
R¹ is C₁₋₂alkoxycarbonyl or cyano;
R² is phenyl which is once or twice substituted by halogen;
R³ is 2-thiazolyl, which is unsubstituted or once substituted by C₁₋₆alkyl or halogen; or 2-thienyl or 2-pyridinyl, which is once substituted by halogen; or 2-imidazolyl, which is once substituted by C₁₋₆alkyl; or 3-isoxazolyl, which is unsubstituted or once substituted by C₁₋₆alkyl;
X is -NR⁷;
one of R⁴ and R⁵ is hydrogen or C₁₋₆alkyl, and the other one with the carbon atom to which it is attached and -NR⁷ together form a pyrrolidinyl or piperidinyl, which is substituted by fluoro;
M is C₁₋₆alkoxycarbonyl, carboxy, diC₁₋₆alkylaminoC₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 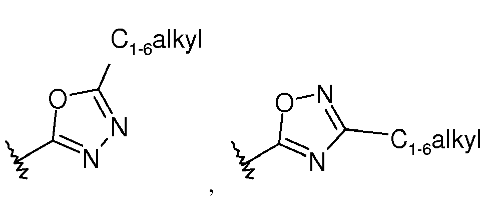 or 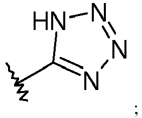
y is 1-6.

Further embodiment of present invention is (xi) a compound of formula I or a pharmaceutically acceptable salt or tautomerism isomers thereof, wherein
R¹ is methoxycarbonyl, ethoxycarbonyl or cyano;
R² is 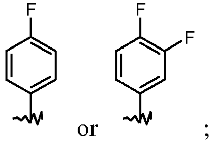
R³ is 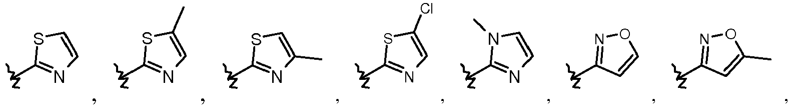 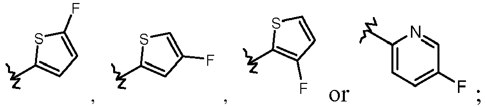
X is -NR⁷;
one of R⁴ and R⁵ is hydrogen or methyl, and the other one with the carbon atom to which it is attached and -NR⁷ form 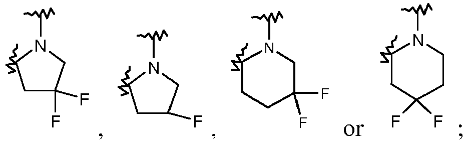
M is methoxycarbonyl, carboxy, dimethylaminoethoxycarbonyl, aminocarbonyl, dimethylaminocarbonyl, methylaminocarbonyl, methylsulfonylaminocarbonyl, 2-thiazolylaminocarbonyl, hydroxymethyl, hydroxypropyl,

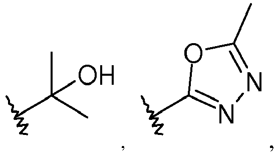

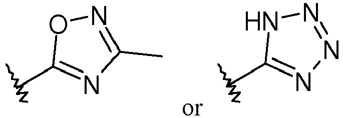

Another embodiment of present invention is (xii) a compound of formula I' or a pharmaceutically acceptable salt or tautomerism isomers thereof, 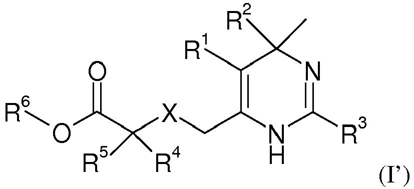 wherein
R¹ is C₁₋₂alkoxycarbonyl or cyano;
R² is phenyl, which is substituted by halogen;
R³ is 2-thiazolyl which is unsubstituted or substituted by C₁₋₆alkyl or 2-pyridinyl, which is substituted by halogen;
X is oxygen or -NR⁷;
R⁴ and R⁵ are independently selected from hydrogen, C₁-₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷ together form a morpholinyl; or pyrrolidinyl substituted by fluoro;
R⁶ is hydrogen or C₁₋₆alkyl;
R⁷ is C₁₋₆alkyl.

Further embodiment of present invention is (xiii) a compound of formula I' or a pharmaceutically acceptable salt or tautomerism isomers thereof, wherein
R¹ is methoxycarbonyl or cyano;
R² is phenyl substituted once or twice by fluoro;
R³ is 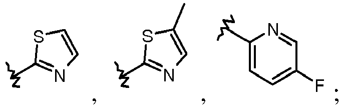
X is oxygen or -NR⁷;
R⁴ and R⁵ are independently selected from hydrogen, methyl and trifluoromethyl; or
R⁴ and R⁵ together with the carbon atom to which they are attached form cyclopropyl; or when X is -NR⁷, one of R⁴ and R⁵ is hydrogen or methyl, and the other one with the carbon atom to which it is attached and -NR⁷ form 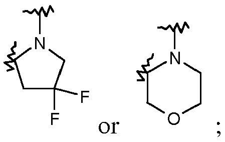
R⁶ is hydrogen or methyl;
R⁷ is methyl.

Still further embodiment of present invention is (xiv) a compound of formula I' or a pharmaceutically acceptable salt or tautomerism isomers thereof, wherein
R¹ is C₁₋₂alkoxycarbonyl;
R² is phenyl which is substituted by halogen;
R³ is 2-thiazolyl;
X is oxygen;
R⁴ and R⁵ are independently selected from hydrogen, C₁₋₆alkyl and trifluoroC₁₋₆alkyl;
R⁶ is hydrogen or C₁₋₆alkyl.

More further embodiment of present invention is (xv) a compound of formula I' or a pharmaceutically acceptable salt or tautomerism isomers thereof, wherein
R¹ is methyoxycarbonyl;
R² is 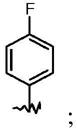
R³ is 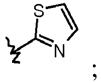
X is oxygen;
R⁴ and R⁵ are independently selected from hydrogen, methyl and trifluoromethyl;
R⁶ is hydrogen or methyl.

Another further embodiment of present invention is (xvi) a compound of formula I' or a pharmaceutically acceptable salt or tautomerism isomers thereof, wherein
R¹ is C₁₋₂alkoxycarbonyl;
R² is phenyl which is substituted by halogen;
R³ is 2-thiazolyl;
X is NC₁₋₆alkyl;
R⁴ and R⁵, together with the carbon atom to which they are attached, form a 3 to 7 membered cycloalkyl;
R⁶ is hydrogen.

Further embodiment of present invention is (xvii) a compound of formula I' or a pharmaceutically acceptable salt or tautomerism isomers thereof, wherein
R¹ is methoxycarbonyl;
R² is 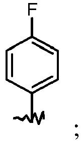
R³ is 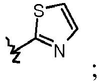
X is -NCH₃;
R⁴ and R⁵, together with the carbon atom to which they are attached, form cyclopropyl;
R⁶ is hydrogen.

More further embodiment of present invention is (xviii) a compound of formula I' or a pharmaceutically acceptable salt or tautomerism isomers thereof, wherein
R¹ is C₁₋₂alkoxycarbonyl or cyano;
R² is phenyl which is substituted by halogen;
R³ is 2-thiazolyl; or 2-pyridinyl, which is substituted by halogen;
X is -NR⁷;
one of R⁴ and R^{S} is hydrogen or C₁₋₆alkyl, and the other one with the carbon atom to which it is attached and -NR⁷ together form a morpholinyl;
R⁶ is hydrogen or C₁₋₆alkyl.

Further embodiment of present invention is (xix) a compound of formula I' or a pharmaceutically acceptable salt or tautomerism isomers thereof, wherein
R¹ is methoxycarbonyl or cyano;
R² is 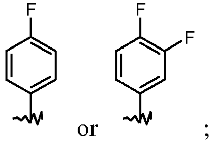
R³ is 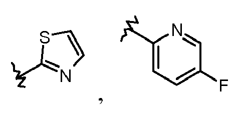
one of R⁴ and R⁵ is hydrogen or methyl, and the other one with the carbon atom to which it is attached and -NR⁷ form 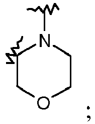
R⁶ is hydrogen or methyl.

Still further embodiment of present invention is (xx) a compound of formula I' or a pharmaceutically acceptable salt or tautomerism isomers thereof, wherein
R¹ is C₁₋₂alkoxycarbonyl or cyano;
R² is phenyl which is substituted by halogen;
R³ is 2-thiazolyl which is unsubstituted or substituted by C₁₋₆alkyl or 2-pyridinyl, which is substituted by halogen;
X is -NR⁷;
one of R⁴ and R⁵ is hydrogen or C₁₋₆alkyl, and the other one with the carbon atom to which it is attached and -NR⁷ together form a pyrrolidinyl which is substituted by fluoro;
R⁶ is hydrogen or C₁₋₆alkyl.

Another further embodiment of present invention is (xxi) a compound of formula I' or a pharmaceutically acceptable salt or tautomerism isomers thereof, wherein
R¹ is methoxycarbonyl or cyano;
R² is 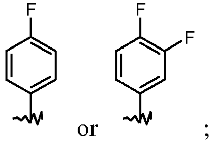
R³ is 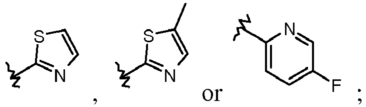
X is -NR⁷;
one of R⁴ and R⁵ is hydrogen or methyl, and the other one with the carbon atom to which it is attached and -NR⁷ form 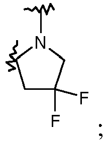
R⁶ is hydrogen or methyl.

Particular compounds of formula I, including their activity data, NMR data and MS data are summarized in the following Table 1 and 2.

**Table 1: Structure, name and activity data of particular compounds**

| Example No. | Structure | Name | HepDe19 EC50 (µM) | CC50 (µM) |
|---|---|---|---|---|
| 1 | 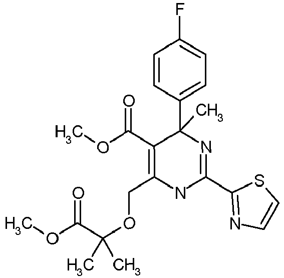 | 4-(4-Fluoro-phenyl)-6-(1-methoxycarbonyl-1-methyl-ethoxymethyl)-4-methyl-2-thiazol-2-yl-1,4-dihydro-pyrimidine-5-carboxylic acid methyl ester | 26.29 | >100 |
| 2 | 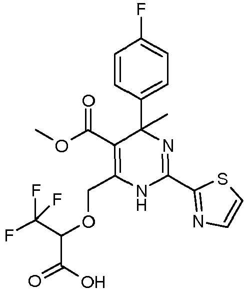 | 6-(1-Carboxy-2,2,2-trifluoro-ethoxymethyl)-4-(4-fluoro-phenyl)-4-methyl-2-thiazol-2-yl-1,4-dihydro-pyrimidine-5-carboxylic acid methyl ester | 24.43 | >100 |
| 3 | 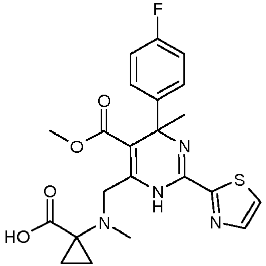 | 6-{[(1-Carboxy-cyclopropyl)-methyl-amino]-methyl}-4-(4-fluoro-phenyl)-4-methyl-2-thiazol-2-yl-1,4-dihydro-pyrimidine-5-carboxylic acid methyl ester | 21.31 | >100 |
| 4 | 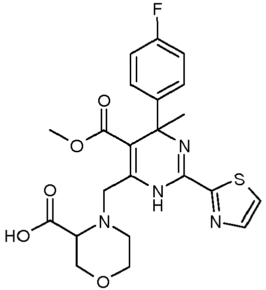 | 4-[6-(4-Fluoro-phenyl)-5-methoxycarbonyl-6-methyl-2-thiazol-2-yl-3,6-dihydro-pyrimidin-4-ylmethyl]-morpholine-3-carboxylic acid | 1.46 | >100 |
| 5 | 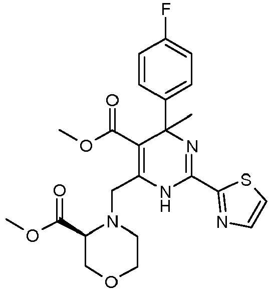 | 4-[6-(4-Fluoro-phenyl)-5-methoxycarbonyl-6-methyl-2-thiazol-2-yl-3,6-dihydro-pyrimidin-4-ylmethyl]-morpholine-(S)-3-carboxylic acid methyl ester | 0.7 | >100 |
| 6 | 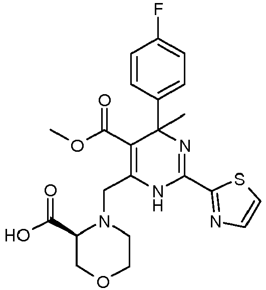 | 4-[6-(4-Fluoro-phenyl)-5-methoxycarbonyl-6-methyl-2-thiazol-2-yl-3,6-dihydro-pyrimidin-4-ylmethyl]-morpholine-(S)-3-carboxylic acid | 1.2 | >100 |
| 7 | 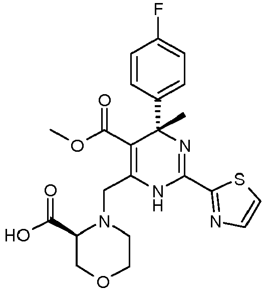 | (S)-4-[6-(4-Fluorophenyl)-5-methoxycarbonyl-6-methyl-2-thiazol-2-yl-3,6-dihydro-pyrimidin-4-ylmethyl]-morpholine-(S)-3-carboxylic acid | 0.77 | >100 |
| 8 | 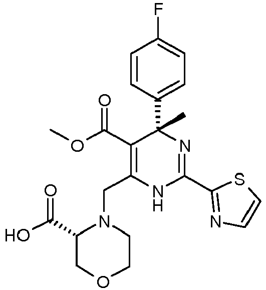 | (S)-4-[6-(4-Fluorophenyl)-5-methoxycarbonyl-6-methyl-2-thiazol-2-yl-3,6-dihydro-pyrimidin-4-ylmethyl]-morpholine-(R)-3-carboxylic acid | 3.01 | >100 |
| 9 | 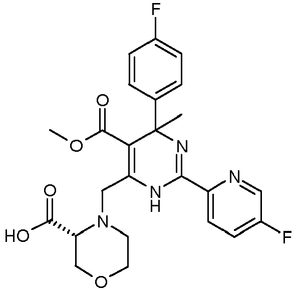 | 4-[6-(4-Fluoro-phenyl)-2-(5-fluoro-pyridin-2-yl)-5-methoxycarbonyl-6-methyl-3,6-dihydro-pyrimidin-4-ylmethyl]-morpholine-(R)-3-carboxylic acid | 4.18 | >100 |
| 10 | 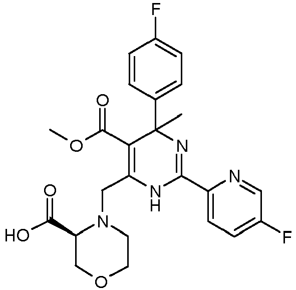 | 4-[6-(4-Fluoro-phenyl)-2-(5-fluoro-pyridin-2-yl)-5-methoxycarbonyl-6-methyl-3,6-dihydro-pyrimidin-4-ylmethyl]-morpholine-(S)-3-carboxylic acid | 7.04 | >100 |
| 11 | 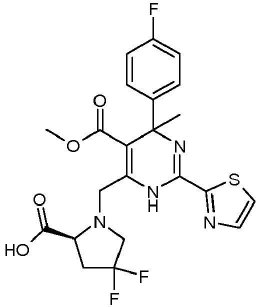 | 6-(2-(S)-Carboxy-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 0.46 | 95.5 |
| 12 | 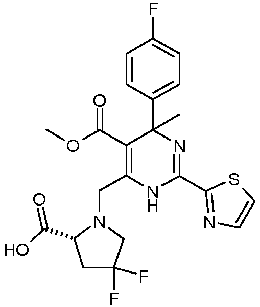 | 6-(2-(R)-Carboxy-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 6.59 | 82 |
| 13 | 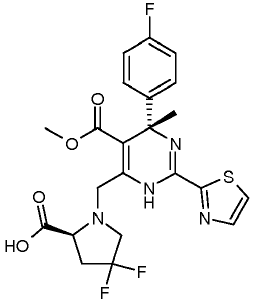 | (S)-6-((S)-2-Carboxy-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 0.15 | >100 |
| 14 | 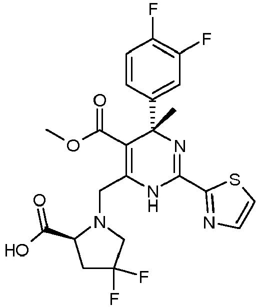 | (S)-6-((S)-2-Carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid methyl ester | 0.2 | >100 |
| 15 | 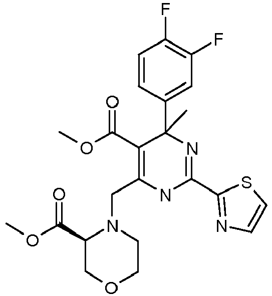 | 4-[(S)-6-(3,4-Difluorophenyl)-5-methoxycarbonyl-6-methyl-2-thiazol-2-yl-3,6-dihydro-pyrimidin-4-ylmethyl]-morpholine-3-carboxylic acid methyl ester | 0.56 | >100 |
| 16 | 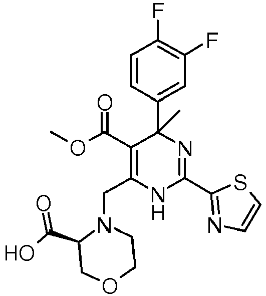 | 4-[6-(3,4-Difluoro-phenyl)-5-methoxycarbonyl-6-methyl-2-thiazol-2-yl-3,6-dihydro-pyrimidin-4-ylmethyl]-morpholine-3-carboxylic acid | 0.25 | >100 |
| 17 | 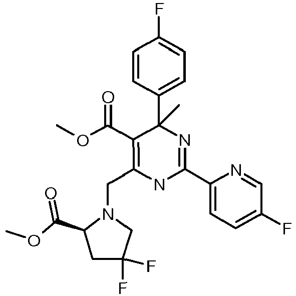 | 6-(4,4-Difluoro-2-methoxycarbonyl-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester | 0.59 | 90 |
| 18 | 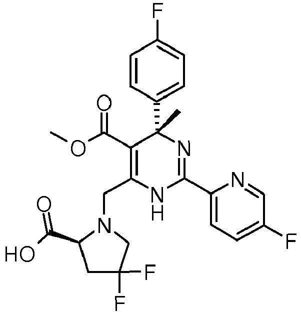 | 6-(2-Carboxy-4,4-difluoro-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester | 0.57 | 25 |
| 19 | 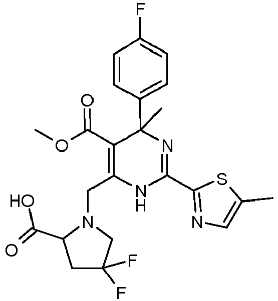 | 6-(2-Carboxy-4,4-difluoro-pyrrolidin-1-ylmethyl)-4-(4-fluorophenyl)-4-methyl-2-(5-methyl-thiazol-2-yl)-1,4-dihydro-pyrimidine-5-carboxylic acid methyl ester | 16.7 | >100 |
| 20 | 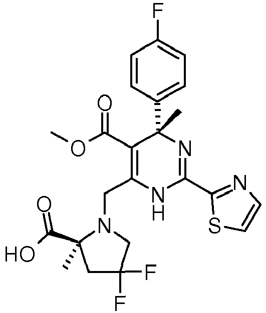 | (S)-6-((S)-2-Carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester | 2.8 | >100 |
| 21 | 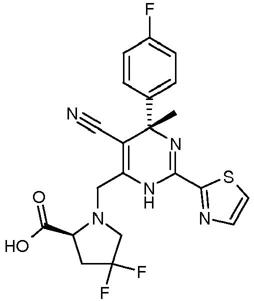 | (S)-1-[(S)-5-Cyano-6-(4-fluoro-phenyl)-6-methyl-2-thiazol-2-yl-3,6-dihydro-pyrimidin-4-ylmethyl]-4,4-difluoro-pyrrolidine-2-carboxylic acid | 21 | >100 |
| 22 | 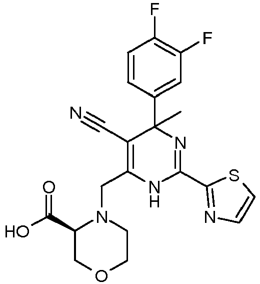 | (S)-4-[5-Cyano-6-(3,4-difluoro-phenyl)-6-methyl-2-thiazol-2-yl-3,6-dihydro-pyrimidin-4-ylmethyl]-morpholine-3-carboxylic acid | 20.4 | >100 |
| 23 | 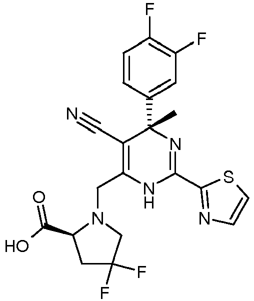 | (S)-1-[(S)-5-Cyano-6-(3,4-difluoro-phenyl)-6-methyl-2-thiazol-2-yl-3,6-dihydro-pyrimidin-4-ylmethyl]-4,4-difluoro-pyrrolidine-2-carboxylic acid | 7 | >100 |
| 24 | 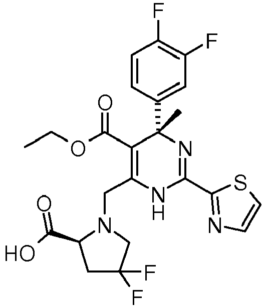 | (*S*)-6-((*S*)-2-Carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester | 0.8 | 100 |
| 25 | 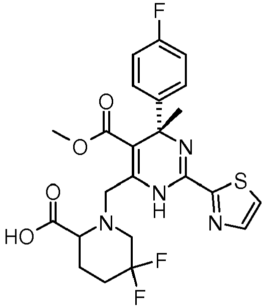 | (*S*)-6-(2-Carboxy-5,5-difluoro-piperidin-1-ylmethyl) -4-(4-fluoro-phenyl)-4-methyl-2-thiazol-2-yl-1,4-dihydro-pyrimidine-5-carboxylic acid methyl ester | 1.09 | 100 |
| 26 | 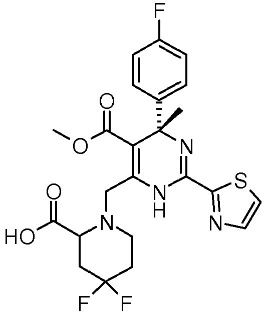 | (*S*)-6-(2-Carboxy-4,4-difluoro-piperidin-1-ylmethyl)4-(4-fluorophenyl)-4-methyl-2-thiazol-2-yl-1,4-dihydro-pyrimidine-5-carboxylic acid methyl ester | 1.97 | 100 |
| 27 | 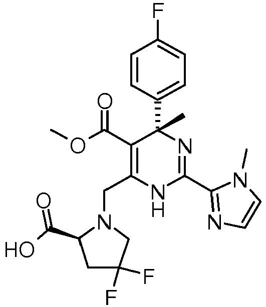 | (*S*)-6-((*S*)-2-Carboxy-4,4-difluoro-pyrrolidin-1-ylmethyl)-4-(4-fluorophenyl)-4-methyl-2-(1-methyl-1*H*-imidazol-2-yl)-1,4-dihydro-pyrimidine-5-carboxylic acid methyl ester | 2.72 | 55.8 |
| 28 | 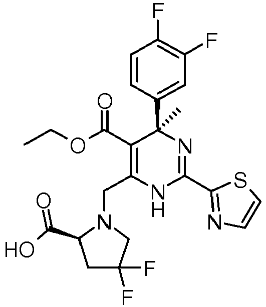 | (*R*)-6-((*S*)-2-Carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester | 7.48 | 100 |
| 29 | 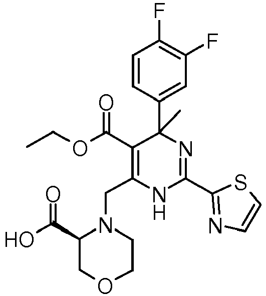 | (*S*)-4-[6-(3,4-Difluorophenyl)-5-ethoxycarbonyl-6-methyl-2-thiazol-2-yl-3,6-dihydro-pyrimidin-4-ylmethyl] -morpholine-3-carboxylic acid | 3.98 | 100 |
| 30 | 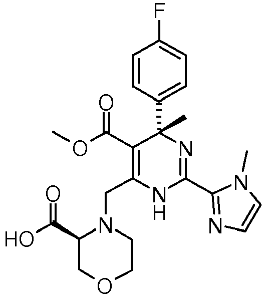 | (*S*)-4-[(*S*)-6-(4-Fluorophenyl)-5-methoxycarbonyl-6-methyl-2-(1-methyl-1H-imidazol-2-yl)-3,6-dihydro-pyrimidin-4-ylmethyl]-morpholine-3-carboxylic acid | 6.77 | 100 |
| 31 | 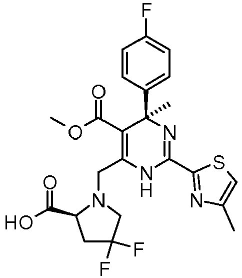 | (*R*)-6-((*S*)-2-Carboxy-4,4-difluoro-pyrrolidin-1-ylmethyl)-4-(4-fluorophenyl)-4-methyl-2-(4-methyl-thiazol-2-yl)-1,4-dihydro-pyrimidine-5-carboxylic acid methyl ester | 1.67 | 100 |
| 32 | 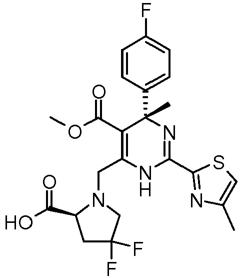 | (*S*)-6-((*S*)-2-Carboxy-4,4-difluoro-pyrrolidin-1-ylmethyl)-4-(4-fluorophenyl)-4-methyl-2-(4-methyl-thiazol-2-yl)-1,4-dihydro-pyrimidine-5-carboxylic acid methyl ester | 0.13 | 75 |
| 33 | 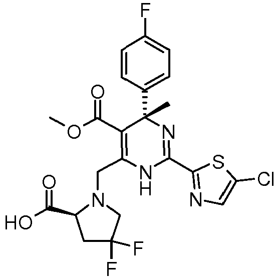 | (*S*)-6-((*S*)-2-Carboxy-4,4-difluoro-pyrrolidin-1-ylmethyl)-2-(5-chloro-thiazol-2-yl)-4-(4-fluorophenyl)-4-methyl-1,4-dihydro-pyrimidine-5-carboxylic acid methyl ester | 2.07 | 80 |
| 34 | 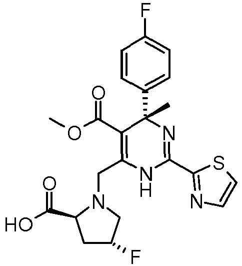 | (*S*)-6-((2*S*,4*R*)-2-Carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester | 2.84 | 100 |
| 35 | 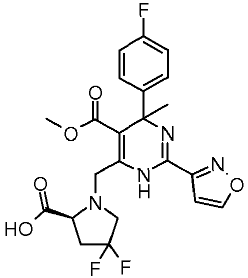 | 6-((*S*)-2-Carboxy-4,4-difluoro-pyrrolidin-1-ylmethyl)-4-(4-fluorophenyl)-2-isoxazol-3-yl-4-methyl-1,4-dihydro-pyrimidine-5-carboxylic acid methyl ester | 15 | 100 |
| 36 | 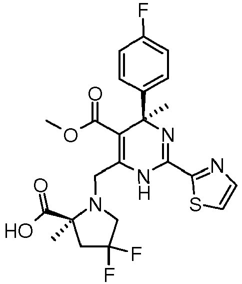 | (*R*)-6-((*S*)-2-Carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester | 7.49 | 100 |
| 37 | 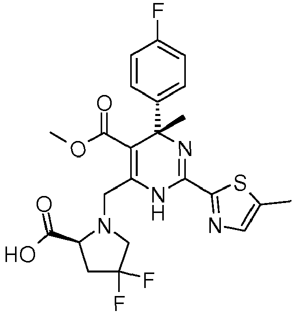 | (*S*)-6-((*S*)-2-Carboxy-4,4-difluoro-pyrrolidin-1-ylmethyl)-4-(4-fluorophenyl)-4-methyl-2-(5-methyl-thiazol-2-yl)-1,4-dihydro-pyrimidine-5-carboxylic acid methyl ester | 0.47 | 85 |
| 38 | 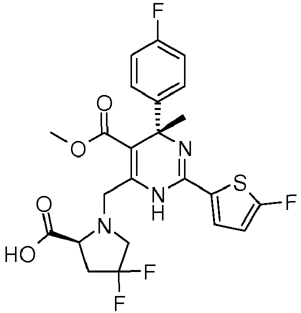 | (*S*)-6-((*S*)-2-Carboxy-4,4-difluoro-pyrrolidin-1-ylmethyl)-4-(4-fluoro-phenyl)-2-(5-fluoro-thiophen-2-yl)-4-methyl-1,4-dihydro-pyrimidine-5-carboxylic acid methyl ester | 2.03 | |
| 39 | 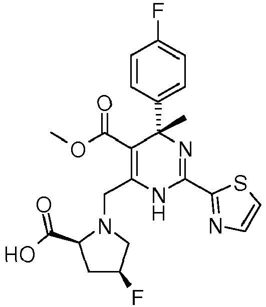 | (*S*)-6-((2*S*,4*S*)-2-Carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester | 10.5 | 100 |
| 40 | 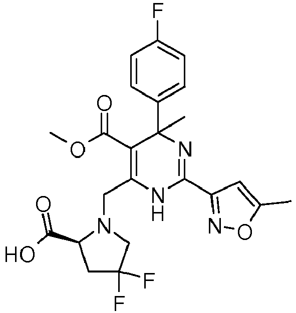 | 6-((*S*)-2-Carboxy-4,4-difluoro-pyrrolidin-1-ylmethyl)-4-(4-fluorophenyl)-4-methyl-2-(5-methyl-isoxazol-3-yl)-1,4-dihydro-pyrimidine-5-carboxylic acid methyl ester | 5.39 | 100 |
| 41 | 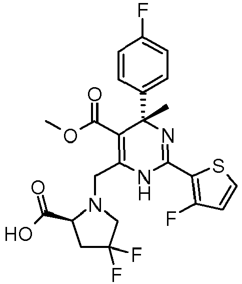 | (*S*)-6-((*S*)-2-Carboxy-4,4-difluoro-pyrrolidin-1-ylmethyl)-4-(4-fluorophenyl)-2-(3-fluoro-thiophen-2-yl)-4-methyl-1,4-dihydro-pyrimidine-5-carboxylic acid methyl ester | 0.285 | 100 |
| 42 | 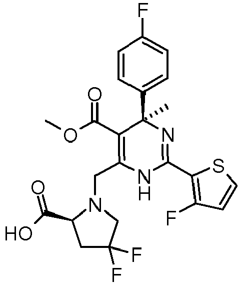 | (*R*)-6-((*S*)-2-Carboxy-4,4-difluoro-pyrrolidin-1-ylmethyl)-4-(4-fluorophenyl)-2-(3-fluoro-thiophen-2-yl)-4-methyl-1,4-dihydro-pyrimidine-5-carboxylic acid methyl ester | 6.81 | 100 |
| 43 | 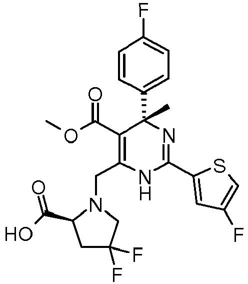 | (*S*)-6-((*S*)-2-Carboxy-4,4-difluoro-pyrrolidin-1-ylmethyl)-4-(4-fluorophenyl)-2-(4-fluoro-thiophen-2-yl)-4-methyl-1,4-dihydro-pyrimidine-5-carboxylic acid methyl ester | 0.131 | 100 |
| 44 | 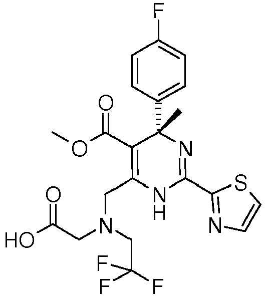 | (*S*)-6-{[Carboxymethyl-(2,2,2-trifluoro-ethyl)-amino]-methyl}-4-(4-fluoro-phenyl)-4-methyl-2-thiazol-2-yl-1,4-dihydro-pyrimidine-5-carboxylic acid methyl ester | 21.5 | 100 |
| 45 | 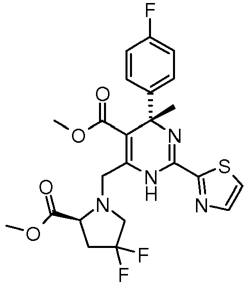 | (*S*)-6-((*S*)-4,4-Difluoro-2-methoxycarbonyl-pyrrolidin-1-ylmethyl)-4-(4-fluoro-phenyl)-4-methyl-2-thiazol-2-yl-1,4-dihydro-pyrimidine-5-carboxylic acid methyl ester | 0.783 | 100 |
| 46 | 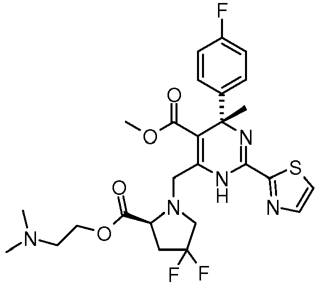 | (*S*)-6-[(*S*)-2-(2-Dimethylaminoethoxycarbonyl)-4,4-difluoro-pyrrolidin-1-ylmethyl]-4-(4-fluoro-phenyl)-4-methyl-2-thiazol-2-yl-1,4-dihydro-pyrimidine-5-carboxylic acid methyl ester | 2.13 | 100 |
| 47 | 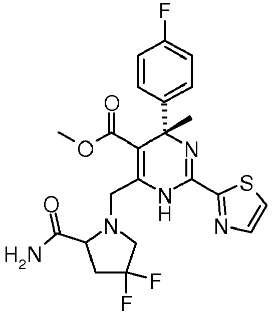 | (S)-6-(2-Carbamoyl-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 0.048 | 100 |
| 48 | 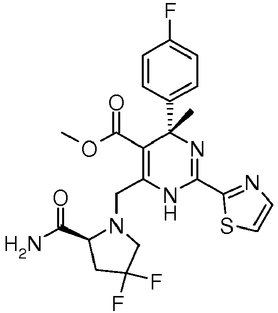 | (*S*)-6-((*S*)-2-Carbamoyl-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 0.08 | 100 |
| 49 | 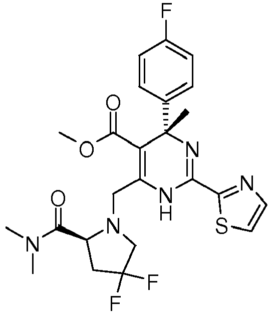 | *(S)-6-((S)-2-*Dimethylcarbamoyl-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester | 0.29 | 100 |
| 50 | 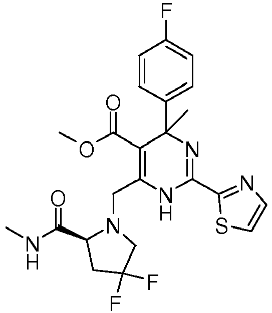 | 6-((S)-4,4-Difluoro-2-methylcarbamoyl-pyrrolidin-1-ylmethyl)-4-(4-fluoro-phenyl)-4-methyl-2-thiazol-2-yl-1,4-dihydro-pyrimidine-5-carboxylic acid methyl ester | 0.606 | 100 |
| 51 | 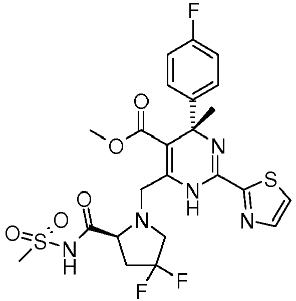 | (*S*)-6-((*S*)-4,4-Difluoro-2-methanesulfonylaminoca rbonyl-pyrrolidin-1-ylmethyl)-4-(4-fluorophenyl)-4-methyl-2-thiazol-2-yl-1,4-dihydro-pyrimidine-5-carboxylic acid methyl ester | 18.16 | 90 |
| 52 | 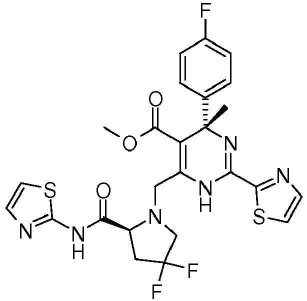 | (*S*)-6-[(*S*)-4,4-Difluoro-2-(thiazol-2-ylcarbamoyl)-pyrrolidin-1-ylmethyl]-4-(4-fluorophenyl)-4-methyl-2-thiazol-2-yl-1,4-dihydro-pyrimidine-5-carboxylic acid methyl ester | 5.426 | 4.07 |
| 53 | 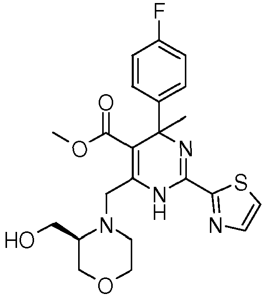 | 4-(4-Fluoro-phenyl)-6-((*R*)-3-hydroxymethyl-morpholin-4-ylmethyl)-4-methyl-2-thiazol-2-yl-1,4-dihydro-pyrimidine-5-carboxylic acid methyl ester | 1.75 | 100 |
| 54 | 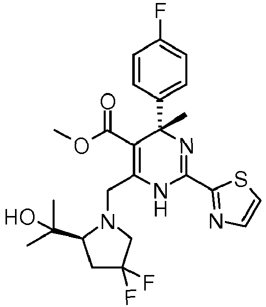 | (*S*)-6-[(*S*)-4,4-Difluoro-2-(1-hydroxy-1-methyl-ethyl)-pymolidin-1-ylmethyl]-4-(4-fluorophenyl)-4-methyl-2-thiazol-2-yl-1,4-dihydro-pyrimidine-5-carboxylic acid methyl ester | 0.7 | 50.4 |
| 55 | 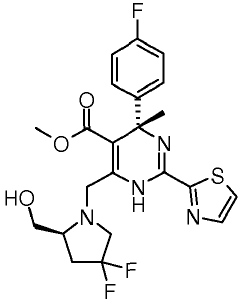 | (*S*)-6-((*S*)-4,4-Difluoro-2-hydroxymethyl-pyrrolidin-1-ylmethyl)-4-(4-fluoro-phenyl)-4-methyl-2-thiazol-2-yl-1,4-dihydro-pyrimidine-5-carboxylic acid methyl ester | 0.067 | 100 |
| 56 | 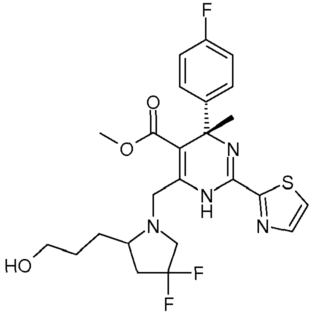 | (*S*)-6-[4,4-Difluoro-2-(3-hydroxy-propyl)-pyrrolidin-1-ylmethyl] -4-(4-fluoro-phenyl)-4-methyl-2-thiazol-2-yl-1,4-dihydro-pyrimidine-5-carboxylic acid methyl ester | 0.66 | 90 |
| 57 | 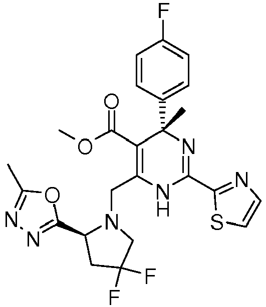 | (*S*)-6-[(*S*)-4,4-Difluoro-2-(5-methyl-[1,3,4]oxadiazol-2-yl)-pyrrolidin-1-ylmethyl]-4-(4-fluoro-phenyl)-4-methyl-2-thiazol-2-yl-1,4-dihydro-pyrimidine-5-carboxylic acid methyl ester | 1.558 | 100 |
| 58 | 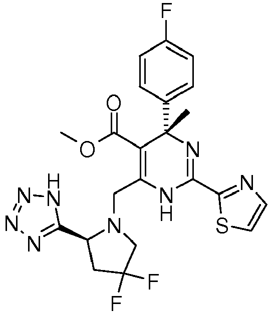 | (*S*)-6-[(*S*)-4,4-Difluoro-2-(1H-tetrazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester | 1.017 | 100 |
| 59 | 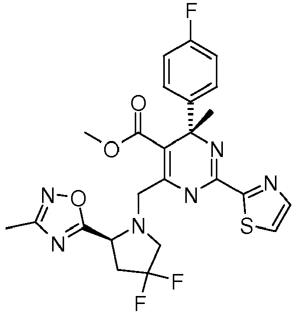 | (*S*)-6-[(*S*)-4,4-Difluoro-2-(3-methyl-[1,2,4]oxadiazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester | 1.238 | 65.3 |

**Table 2: NMR and MS data of particular compounds**

| Example No. | 1H NMR data | MW |
|---|---|---|
| 1 | 1H NMR (MeOD-d₄, 400MHz), 7.95 (d, 1H, J = 3.2 Hz), 7.75 (d, 1H, J = 3.2 Hz), 7.49-7.45 (m, 2H), 7.04 (t, 2H, J = 8.8 Hz), 4.71-4.62 (m, 2H), 3.80 (s, 3H), 3.43 (s, 3H), 1.92 (s, 3H), 1.57 (s, 6H). | MS: calc'd 462 (MH+), exp 462 (MH+). |
| 2 | 1H NMR (MeOD-d₄, 400MHz), 7.94 (d, 1H, J = 2.8 Hz), 7.93 (d, 1H, J = 2.8 Hz), 7.53-7.49 (m, 2H), 7.07-7.03 (m, 2H), 4.85-4.78 (m, 2H), 4.40-4.37 (m, 1H), 3.44 (s, 3H), 1.94 (s, 3H). | MS: calc'd 488 (MH+), exp 488 (MH+). |
| 3 | 1H NMR (MeOD-d₄, 400MHz), 8.12 (d, 1H, J = 3.2 Hz), 8.06 (d, 1H, J = 3.2 Hz), 7.63-7.60 (m, 2H), 7.15 (t, 2H, J = 8.8 Hz), 4.30 (s, 2H), 3.51 (s, 3H), 2.79 (s, 3H), 2.10 (s, 3H), 1.52-1.51 (m, 2H), 1.38-1.37 (m, 2H). | MS: calc'd 459 (MH+), exp 459 (MH+). |
| 4 | 1H NMR (CD3OD, 500 MHz) δ 8.10 (d, 1H), 8.03 (d, 1H), 7.64-7.61 (m, 2H), 7.15-7.12 (m, 2H), 4.32-4.31 (m, 2H), 4.18-4.14 (m, 2H), 3.99-3.72 (m, 3H), 3.51-3.49 (m, 4H), 3.02 (m, 1H), 2.11 (d, 3H). | LC-MS: calc'd 475 (MH+), exp 475 (MH+). |
| 5 | 1H NMR (MeOD-d4, 400 MHz), 7.95 (d, 1H, J = 3.2 Hz), 7.74 (d, 1H, J = 3.2 Hz), 7.48-7.45 (m, 2H), 7.03 (t, 2H, J = 8.8 Hz), 4.05-3.87 (m, 4H), 3.78-3.71 (m, 5H), 3.49-3.45 (m, 1H), 3.42 (s, 3H), 3.14-3.07 (m, 1H), 2.52-2.42 (m, 1H), 1.99 (s, 3H). | MS: calc'd (MH+) 489 exp (MH+) 489. |
| 6 | 1H NMR (MeOD-d4, 400 MHz), 7.95 (d, 1H, J = 3.2 Hz), 7.74 (d, 1H, J = 3.2 Hz), 7.48-7.45 (m, 2H), 7.03 (t, 2H, J = 8.8 Hz), 4.35-4.21 (m, 2H), 4.17-4.05 (m, 2H), 3.96-3.84 (m, 2H), 3.75-3.70 (m, 1H), 3.58-3.47 (m, 4H), 2.97-2.89 (m, 1H), 1.99 (s, 3H). | MS: calc'd (MH+) 475 exp (MH+) 475. |
| 7 | 1H NMR (MeOD-d4, 400 MHz), 8.07 (d, 1H, J = 3.2 Hz), 7.97 (d, 1H, J = 3.2 Hz), 7.62-7.59 (m, 2H), 7.13 (t, 2H, J = 8.8 Hz), 4.31 (s, 2H), 4.17-4.05 (m, 2H), 3.96-3.84 (m, 3H), 3.62-3.55 (m, 1H), 3.51 (s, 3H), 3.03-2.99 (m, 1H), 2.09 (s, 3H). | MS: calc'd (MH+) 475 exp (MH+) 475. |
| 8 | 1H NMR (MeOD-d4, 400 MHz), 8.07 (d, 1H, J = 3.2 Hz), 7.97 (d, 1H, J = 3.2 Hz), 7.62-7.59 (m, 2H), 7.13 (t, 2H, J = 8.8 Hz), 4.28 (dd, 2H, J1 = 33.4 Hz, J2 = 16.4 Hz), 4.17-4.05 (m, 2H), 3.96-3.84 (m, 3H), 3.62-3.50 (m, 4H), 3.03-2.99 (m, 1H), 2.09 (s, 3H). | MS: calc'd (MH+) 475 exp (MH+) 475. |
| 9 | 1H NMR (MeOD-d4, 400 MHz), 8.60 (d, 1H, J = 2.8 Hz), 8.53-8.50 (m, 1H), 7.80-7.78 (m, 1H), 7.59-7.56 (m, 2H), 7.11-7.07 (m, 2H), 4.11-4.01 (m, 3H), 3.99-3.97 (m, 1H), 3.88-3.84 (m, 1H), 3.82-3.80 (m, 1H), 3.59-3.56 (m, 1H), 3.49 (s, 3H), 3.44-3.38 (m, 1H), 2.89-2.80 (m, 1H), 2.05 (s, 3H). | MS: calc'd (MH+) 487 exp (MH+) 487. |
| 10 | 1H NMR (MeOD-d4, 400 MHz), 8.60 (d, 1H, J = 2.8 Hz), 8.55-8.53 (m, 1H), 7.83-7.79 (m, 1H), 7.60-7.57 (m, 2H), 7.13-7.08 (m, 2H), 4.17-4.08 (m, 3H), 4.01-3.97 (m, 1H), 3.91-3.84 (m, 1H), 3.82-3.80 (m, 1H), 3.60-3.58 (m, 1H), 3.50 (s, 3H), 3.44-3.38 (m, 1H), 2.89-2.80 (m, 1H), 2.06 (s, 3H). | MS: calc'd (MH+) 487 exp (MH+) 487. |
| 11 | 1H NMR (CD3OD, 500 MHz) δ 8.15-8.15 (m, 2H), 7.67-7.62 (m, 2H), 7.19-7.14 (m, 2H), 4.16-3.90 (m, 3H), 3.65-3.61 (m, 1H), 3.51 (d, 3H), 3.25-3.20 (m, 1H), 2.86-2.82 (m, 1H), 2.63-2.61 (m, 1H), 2.15 (d, 3H). | LC-MS: calc'd 495 (MH+), exp 495 (MH+). |
| 12 | 1H NMR (CD3OD, 500 MHz) δ 7.96 (s, 1H), 7.77 (s, 1H), 7.52 (d, 2H), 7.06 (d, 2H), 4.00-3.95 (m, 3H), 3.63-3.61 (m, 1H), 3.46 (s, 3H), 3.20-3.18 (m, 1H),2.79 (m, 1H), 2.67 (m, 1H), 1.95 (s, 3H). | LC-MS: calc'd 495 (MH+), exp 495 (MH+). |
| 13 | 1H NMR (MeOD-d4, 400 MHz), 8.21 (s, 2H), 7.67-7.65 (m, 2H), 7.21-7.17 (m, 2H,), 4.16 (d, 1H, J = 15.6 Hz), 4.02 (t, 1H, J = 8.0 Hz), 3.93 (d, 1H, J = 15.6 Hz), 3.72-3.61 (m, 1H), 3.53 (s, 3H), 3.29-3.19 (m, 1H), 2.91-2.78 (m, 1H), 2.59-2.55 (m, 1H), 2.17 (s, 3H). | MS: calc'd (MH+) 495 exp (MH+) 495. |
| 14 | 1H NMR (MeOD-d4, 400 MHz), 8.11 (d, 1H, J = 3.2 Hz), 8.04 (d, 1H, J = 3.2 Hz), 7.55-7.49 (m, 1H), 7.40-7.38 (m, 1H), 7.34-7.29 (m, 1H,), 4.15 (d, 1H, J = 15.6 Hz), 4.02 -3.98(m, 2H), 3.74-3.61 (m, 1H), 3.53 (s, 3H), 3.30-3.23 (m, 1H), 2.91-2.78 (m, 1H), 2.65-2.49 (m, 1H), 2.08 (s, 3H). | MS: calc'd (MH+) 513.1, exp (MH+) 513.1 |
| 15 | 1H NMR (MeOD-d4, 400 MHz), 8.17 (d, 1H, J = 3.2 Hz), 8.10 (d, 1H, J = 3.2 Hz), 7.62-7.53 (m, 1H), 7.45-7.39 (m, 1H), 7.37-7.25 (m, 1H,), 4.25-4.18 (m, 2H), 4.10-4.06(m, 2H), 3.96-3.94 (m, 1H), 3.89-3.86 (m, 2H), 3.84 (s, 3H), 3.55 (s, 3H), 3.50-3.40 (m, 1H), 2.90-2.87 (m, 1H), 2.08 (s, 3H). | MS: calc'd (MH+) 507, exp (MH+) 507. |
| 16 | 1H NMR (MeOD-d4, 400 MHz), 8.06 (d, 1H, J = 3.2 Hz), 7.95 (d, 1H, J = 3.2 Hz), 7.53-7.51 (m, 1H), 7.40-7.37 (m, 1H), 7.31-7.25 (m, 1H,), 4.39-4.28 (m, 2H), 4.15-4.12 (m, 2H), 3.58-3.50 (m, 4H), 3.05-3.01 (m, 1H), 2.05 (s, 3H). | MS: calc'd (MH+) 493 exp (MH+) 493. |
| 17 | 1H NMR (MeOD-d4, 400 MHz), 8.80 (d, 1H, J = 2.8 Hz), 8.43-8.40 (m, 1H), 8.01-7.98 (m, 1H), 7.70-7.66 (m, 2H), 7.24-7.18 (m, 2H), 4.16-3.94 (m, 3H), 3.84-3.83 (m, 3H), 3.61-3.55 (m, 4H), 3.22-3.18 (m, 1H), 2.91-2.78 (m, 1H), 3.61-3.48 (m, 1H), 2.24-2.21 (m, 3H). | MS: calc'd (MH+) 521 exp (MH+) 521 |
| 18 | 1H NMR (MeOD-d4, 400 MHz), 8.77 (d, 1H, J = 2.8 Hz), 8.46-8.42 (m, 1H), 8.00-7.95 (m, 1H), 7.70-7.66 (m, 2H), 7.23-7.19 (m, 2H), 4.16 (d, 1H, J = 16 Hz), 3.98 (t, 1H, J = 8.8 Hz), 3.88 (d, 1H, J = 16 Hz), 3.60-3.56 (m, 4H), 3.28-3.15 (m, 1H), 2.93-1.79 (m, 1H), 2.58-44 (m, 1H), 2.21 (s, 3H). | MS: calc'd (MH+) 507 exp (MH+) 507. |
| 19 | ¹H NMR (400 MHz, MeOH-d4) 7.87 (s, 1 H), 7.47 - 7.51 (m, 2 H), 7.01 - 7.06 (m, 2 H), 3.92 - 4.30 (m, 2 H), 3.49 (s, 3 H), 3.40 (m, 2H), 2.35 - 2.90 (m, 3 H), 2.36 (s, 3 H), 1.85 (s, 3 H). | MS: calc'd (MH+) 509 exp (MH+) 509. |
| 20 | ¹H NMR (400 MHz, MeOH-d4) 8.02 - 8.18 (m, 2 H), 7.54 - 7.65 (m, 2 H), 7.08 - 7.20 (m, 2 H), 3.88 - 4.14 (m, 2 H), 3.49 (s, 3 H), 2.75 - 2.90 (m, 2 H), 2.41 - 2.57 (m, 2 H), 2.09 (s, 3 H), 1.56 (s, 3 H). | MS: calc'd (MH+) 509 exp (MH+) 509. |
| 21 | 1H NMR (MeOD-d4, 400 MHz), 7.98 (d, 1H, J = 3.2 Hz), 7.81 (d, 1H, J = 3.2 Hz), 7.57-7.53 (m, 2H), 7.14 (t, 2H, J = 8.8 Hz), 3.92-3.74 (m, 3H), 3.49-3.41 (m, 1H), 3.18-3.08 (m, 1H), 2.81-2.69 (m, 1H), 2.55-2.49 (m, 1H), 1.89 (s, 3H). | MS: calc'd (M++H) 462, exp (M++H) 462. |
| 22 | 1H NMR (MeOD-d4, 400 MHz), 8.03 (d, 1H, J = 3.2 Hz), 7.88 (d, 1H, J = 3.2 Hz), 7.45-7.42 (m, 1H), 7.36-7.30 (m, 2H), 4.16-4.01(m, 4H), 3.96-3.82 (m, 3H), 3.43-3.37 (m, 1H), 2.93-2.86 (m, 1H), 1.92 (s, 3H). | MS: calc'd (MH+) 460 exp (MH+) 460. |
| 23 | 1H NMR (MeOD-d4, 400 MHz), 7.98 (d, 1H, J = 3.2 Hz), 7.81 (d, 1H, J = 3.2 Hz), 7.44-7.39 (m, 1H), 7.35-7.27 (m, 2H), 3.92-3.74 (m, 3H), 3.49-3.41 (m, 1H), 3.18-3.08 (m, 1H), 2.81-2.69 (m, 1H), 2.52-2.46 (m, 1H), 1.86 (s, 3H). | MS: calc'd (MH+) 480 exp (MH+) 480. |
| 24 | 1H NMR (MeOD-d₄, 400 MHz), 7.95 (d, *J* = 3.01 Hz, 1 H), 7.73 (d, *J* = 3.26 Hz, 1 H), 7.37 (ddd, *J* = 12.17, 7.78, 1.88 Hz, 1 H), 7.28 (br. s., 1 H), 7.17- 7.26 (m, 1 H), 3.85 - 4.06 (m, 4 H) 3.69 (t, J=8.16 Hz, 1 H) 3.48 - 3.61 (m, 1 H) 2.99 - 3.18 (m, 1 H) 2.61 - 2.78 (m, 1 H) 2.39 - 2.58 (m, 1 H) 1.89 (s, 3 H)1.04 (t, *J*= 7.03 Hz, 3 H). | MS: calc'd (MH+) 527 exp (MH+) 527. |
| 25 | ¹H NMR (MeOD-d₄, 400 MHz), 8.25 (s, 2 H), 7.58 - 7.75 (m, 2 H), 7.19 (t, *J* = 8.66 Hz, 2 H), 3.93 - 4.19 (m, 2 H), 3.75 (d, *J* = 1.25 Hz, 1 H), 3.53 (s, 3 H), 3.35 -3.46 (m, 1 H), 2.88 - 3.10 (m, 1 H), 1.85 - 2.41 (m, 7 H). | MS: calc'd (MH+) 509 exp (MH+) 509. |
| 26 | ¹H NMR (MeOD-d₄, 400 MHz), 7.97 (d, *J* = 3.01 Hz, 1 H), 7.72 (d, *J* = 3.01 Hz, 1 H), 7.49 (dd, *J* = 8.66, 5.40 Hz, 2 H), 7.04 (t, *J* = 8.78 Hz, 2 H), 3.72- 3.87 (m, 2 H), 3.44 (s, 3 H), 3.25 - 3.31 (m, 1 H), 3.14 (d, *J* = 12.05 Hz, 1 H), 2.47 - 2.60 (m, 1 H), 1.97 - 2.41 (m, 4 H), 1.89 (s, 3 H). | MS: calc'd (MH+) 509 exp (MH+) 509. |
| 27 | ¹H NMR (MeOD-d₄, 400 MHz), 7.53 (dd, *J* = 8.66, 5.40 Hz, 2 H), 7.19 (s, 1 H), 6.98 - 7.11 (m, 3 H), 3.77 - 3.96 (m, 5 H), 3.51 - 3.65 (m, 2 H), 3.46(s, 3 H), 3.01 (td, *J* = 15.12, 11.42 Hz, 1 H), 2.64 (qd, *J* = 12.84, 8.16 Hz, 1 H), 2.32 - 2.50 (m, 1 H), 1.84 - 2.00 (m, 3 H). | MS: calc'd (MH+) 492 exp (MH+) 492. |
| 28 | ¹H NMR (MeOD-d₄, 400 MHz), 7.95 (d, *J* = 3.01 Hz, 1 H), 7.72 (d, *J* = 3.26 Hz, 1 H), 7.36 (ddd, *J* = 12.30, 7.78, 2.26 Hz, 1 H), 7.27 (br. s., 1 H), 7.21(dd, *J =* 10.29, 8.28 Hz, 1 H), 3.80 - 4.02 (m, 4 H), 3.44 - 3.58 (m, 2 H), 2.87 (td, *J* = 15.56, 11.04 Hz, 1H), 2.54 - 2.73 (m, 1 H), 2.35 - 2.52 (m, 1 H), 1.89 (s, 3 H), 1.03 (t, *J* = 7.03 Hz, 3 H). | MS: calc'd (MH+) 527 exp (MH+) 527. |
| 29 | ¹H NMR (MeOD-d₄, 400 MHz), 8.10 (d, *J*= 2.76 Hz, 1 H), 7.99 - 8.06 (m, 1 H), 7.52 - 7.63 (m, 1 H), 7.44 (d, J = 7.03 Hz, 1 H), 7.22 - 7.38 (m, 1 H), 5.36 (t, *J*= 4.64 Hz, 1 H), 4.30 - 4.54 (m, 2 H), 4.09 - 4.22 (m, 3 H), 3.86 - 4.06 (m, 4 H), 3.55 - 3.73 (m, 1 H), 1.98 - 2.15 (m, 3 H), 0.92 (t, *J*= 6.78 Hz, 3 H). | MS: calc'd (MH+) 507 exp (MH+) 507. |
| 30 | ¹H NMR (MeOD-d₄, 400 MHz), 7.52 (dd, *J* = 8.66, 5.40 Hz, 2 H), 7.21 (s, 1 H), 6.98 - 7.11 (m, 3 H), 3.72 - 4.06 (m, 10 H) 3.48 (s, 3 H), 3.12 - 3.23 (m, 1 H), 2.61 (ddd, *J =* 11.54, 8.16, 3.14 Hz, 1 H), 1.93 (s, 3 H). | MS: calc'd (MH+) 472 exp (MH+) 472. |
| 31 | ¹H NMR (CDCl₃, 400 MHz), 7.47 - 7.59 (m, 2 H), 7.18 (s, 1 H), 7.01 - 7.12 (m, 2 H), 4.12 (d, *J* = 14.56 Hz, 1 H), 3.98 (dd, *J* = 9.79, 6.02 Hz, 1 H), 3.61 (d, *J* = 14.31 Hz, 1 H), 3.53 (d, *J* = 10.54 Hz, 1 H), 3.47 (s, 3 H), 3.12 (br. s., 1 H), 2.71 - 2.90 (m, 1 H), 2.50 - 2.67 (m, 1 H), 2.46 (s, 3 H), 1.98 - 2.07 (m, 3 H). | MS: calc'd (MH+) 509 exp (MH+) 509. |
| 32 | ¹H NMR (CDCl₃, 400 MHz), 7.49 - 7.61 (m, 2 H), 7.17 (s, 1 H), 7.05 - 7.13 (m, 2 H), 4.14 (d, *J* = 14.31 Hz, 1 H), 3.92 (dd, *J* = 9.66, 6.15 Hz, 1 H), 3.49 - 3.58 (m, 2 H), 3.47 (s, 3 H), 3.18 - 3.33 (m, 1 H), 2.81 (dd, *J* = 14.68, 9.66 Hz, 1 H), 2.57 (dd, *J* = 12.30, 6.02 Hz, 1 H), 2.46 (s, 3 H), 2.01 (s, 3 H). | MS: calc'd (MH+) 509 exp (MH+) 509. |
| 33 | ¹H NMR (CDCl₃, 400 MHz), 7.71 (s, 1 H), 7.41 - 7.58 (m, 2 H), 7.00 - 7.17 (m, 2 H), 4.12 - 4.21 (m, 1 H), 3.93 - 4.03 (m, 1 H), 3.70 - 3.78 (m, 1 H), 3.54 (s, 4 H), 3.15 - 3.30 (m, 1 H), 2.75 - 2.90 (m, 1 H), 2.52 - 2.69 (m, 1 H), 2.15 (s, 3 H). | MS: calc'd (MH+) 529 exp (MH+) 529. |
| 34 | ¹H NMR (MeOD-d₄, 400 MHz), 7.93 - 8.01 (m, 1 H), 7.79 - 7.89 (m, 1 H), 7.55 - 7.67 (m, 2 H), 7.06 - 7.17 (m, 2 H), 5.45 - 5.58 (m, 1 H), 5.31 - 5.45 (m, 1 H), 3.81 - 4.12 (m, 2 H), 3.48 (s, 3 H), 2.70 - 2.94 (m, 2 H), 2.31 - 2.55 (m, 2 H), 1.96 - 2.10 (m, 3 H). | MS: calc'd (MH+) 477 exp (MH+) 477. |
| 35 | ¹H NMR (MeOD-d₄, 400 MHz), 8.80 - 8.87 (m, 1 H), 7.48 - 7.59 (m, 2 H), 7.10 (s, 3 H), 3.77 - 4.10 (m, 3 H), 3.48 - 3.62 (m, 1 H), 3.46 (d, *J* = 4.27 Hz, 3 H), 3.00 - 3.23 (m, 1 H), 2.70 - 2.87 (m, 1 H), 2.42 - 2.64 (m, 1 H), 1.98 (d, *J* = 3.51 Hz, 3 H). | MS: calc'd (MH+) 479 exp (MH+) 479. |
| 36 | ¹H NMR (MeOD-d₄, 400 MHz), 8.14 - 8.17 (m, 1 H), 8.10 - 8.13 (m, 1 H), 7.58 - 7.65 (m, 2 H), 7.11 - 7.19 (m, 2 H), 3.99 - 4.06 (m, 2 H), 3.52 (s, 4 H), 3.35 - 3.38 (m, 1 H), 2.75 - 2.89 (m, 1 H), 2.41 - 2.56 (m, 1 H), 2.12 (s, 3 H), 1.56 (s, 3 H). | MS: calc'd (MH+) 509 exp (MH+) 509. |
| 37 | ¹H NMR (MeOD-d₄, 400 MHz), 7.62 (s, 1H), 7.50 (dd, *J* = 5.40, 8.66 Hz, 2H), 7.05 (t, *J* = 8.78 Hz, 2H), 3.90 (d, *J = 5.77* Hz, 2H), 3.65 (t, *J* = 8.28 Hz, 1H), 3.39 - 3.58 (m, 7H), 3.05 (d, *J* = 11.54 Hz, 1H), 2.68 (dd, *J* = 7.91, 13.18 Hz, 1H), 2.48 - 2.58 (m, 4H). | MS: calc'd (MH+) 509 exp (MH+)509. |
| 38 | ¹H NMR (MeOD-d₄, 400MHz), 7.62 (t, *J* = 3.9 Hz, 1 H), 7.46 (dd, *J* = 8.7, 5.4 Hz, 2 H), 7.02 (t, *J* = 8.8 Hz, 2 H), 6.59 (dd, *J* = 4.3, 1.8 Hz, 1 H),3.86 (d, *J* = 14.3 Hz, 2 H), 3.75 (t, *J* = 6.5 Hz, 1 H), 3.41 - 3.62 (m, 6 H), 3.06 (q, *J* = 7.3 Hz, 2 H), 2.61 (dd, *J* = 16.1, 7.5 Hz, 1 H), 2.26 - 2.45 (m, 1 H). | MS: calc'd (MH+)512 exp (MH+) 512. |
| 39 | ¹H NMR (MeOD-d₄, 400MHz), 7.96 (d, *J* = 3.0 Hz, 1 H), 7.85 (d, *J* = 3.3 Hz, 1 H), 7.65 (dd, *J* = 8.8, 5.3 Hz, 2 H), 7.13 (t, *J =* 8.8 Hz, 2 H), 5.29 - 5.52 (m, 1 H), 4.62 (d, *J* = 14.1 Hz, 1 H), 4.39 (d, *J* = 9.5 Hz, 1 H), 4.08 - 4.27 (m, 2 H), 3.44 - 3.69 (m, 5 H), 2.54 - 2.97 (m, 3 H). | MS: calc'd (MH+)477 exp (MH+) 477. |
| 40 | ¹H NMR (MeOD-d₄, 400MHz), 7.51 (dd, *J=* 8.2, 5.4 Hz, 2 H), 7.06 (q, *J* = 8.8 Hz, 2 H), 6.61 (d, *J* = 4.0 Hz, 1 H), 3.72 - 3.96 (m, 2 H), 3.40 - 3.63 (m, 6 H), 2.82 - 3.12 (m, 2 H), 2.57 - 2.76 (m, 1 H), 2.35 - 2.52 ppm (m, 5 H). | MS: calc'd (MH+)493 exp (MH+) 493. |
| 41 | ¹H NMR (CDCl₃, 400 MHz), 7.53 (m, 2H), 7.43 (m, 1H), 7.08 (m, 2H), 6.85 (d, 1H, *J* = 5.6 Hz), 4.08 (d, 1H, *J* = 14.2 Hz), 3.86 (1H, m), 3.57 (d, 1H, *J* = 14.2 Hz), 3.50 (1H, m), 3.45 (3H, s), 3.20 (1H, m), 2.77 (1H, m), 2.54 (1H, m), 1.97 (3H, s). | MS: calc'd (MH+) 512 exp (MH+) 512. |
| 42 | ¹H NMR (CDCl₃, 400 MHz), 7.52 (m, 2H), 7.42 (m, 1H), 7.06 (m, 2H), 6.84 (d, 1H, *J* = 5.6 Hz), 4.05 (d, 1H, *J =* 14.2 Hz), 3.91 (m, 1H), 3.64 (d, 1H, *J* = 14.2 Hz), 3.47 (m, 1H), 3.45 (3H, s), 3.10 (m, 1H), 2.78 (m, 1H), 2.55 (m, 1H), 1.98 (s, 3H). | MS: calc'd (MH+) 512 exp (MH+) 512. |
| 43 | ¹H NMR (CDCl₃, 400 MHz), 7.70 (s, 1H), 7.45 (m, 2H), 7.00 (m, 3H), 4.07 (d, 1H, *J* = 15.0 Hz), 3.72 (m, 1H), 3.55 (s, 3H), 3.54-3.34 (m, 2H), 3.07 (m, 1H), 2.68 (m, 1H), 2.32 (m, 1H), 2.01 (s, 3H). | MS: calc'd (MH+) 512 exp (MH+) 512. |
| 44 | ¹H NMR (MeOD-d₄, 400 MHz),7.95 (d, *J* = 3.26 Hz, 1 H), 7.75 (d, *J* = 3.26 Hz, 1 H), 7.44 - 7.54 (m, 2 H), 7.05 (t, *J =* 8.78 Hz, 2 H), 3.90 - 4.09 (m, 2H), 3.63 (s, 2 H), 3.45 (s, 3 H), 1.91 (s, 3H). | MS: calc'd (MH+) 501 exp (MH+) 501. |
| 45 | ¹H NMR (MeOD-d₄, 400 MHz), 8.23 (d, *J* = 0.75 Hz, 2 H), 7.61 - 7.70 (m, 2H) ,7.19 (t, *J =* 8.66 Hz, 2 H), 4.06 - 4.16 (m, 1 H), 4.02 (t, *J* = 8.03 Hz, 1H), 3.92 (d, *J =* 15.81 Hz, 1 H), 3.82 (s, 3 H), 3.59 (d, *J =* 10.79 Hz, 1 H), 3.53 (s, 3 H), 3.12 - 3.28 (m, 1 H), 2.72 - 2.89 (m, 1 H), 2.43 - 2.61 (m, 1 H), 2.16 (s, 3 H). | MS: calc'd (MH+) 509 exp (MH+) 509. |
| 46 | ¹H NMR (MeOD-d₄, 400 MHz), 7.94 (d, *J* = 3.01 Hz, 1 H), 7.74 (d, *J* = 3.01 Hz, 1 H), 7.49 (br. s., 2 H), 7.05 (t, *J =* 8.78 Hz, 2 H), 4.33 (t, *J* = 5.65 Hz, 2 H), 3.94 (s, 3 H), 3.42 - 3.56 (m, 5 H), 2.66 - 2.82 (m, 4 H), 2.33 (s, 6 H), 1.90 (s, 3 H). | MS: calc'd (MH+) 566 exp (MH+) 566. |
| 47 | ¹H NMR (MeOD-d₄, 400 MHz), 7.95 (br. s., 1 H) 7.69 - 7.84 (m, 1 H) 7.61 (dd, *J* = 8.28, 5.52 Hz, 1 H) 7.48 (dd, *J* = 8.03, 5.52 Hz, 1 H) 6.99 - 7.17(m, 2 H) 3.39 - 3.88 (m, 6 H) 2.91 - 3.30 (m, 2 H) 2.62 - 2.86 (m, 1 H) 2.22 - 2.53 (m, 1 H) 1.81 - 2.02 (m, 3 H). | MS: calc'd (MH+) 494 exp (MH+) 494. |
| 48 | ¹H NMR(MeOD-d₄, 400 MHz), 8.13 - 8.00 (m, 1 H) 7.98 - 7.82 (m, 1 H) 7.59 (br. s., 2 H) 7.13 (s, 2 H) 3.96 - 3.82 (m, 1 H) 3.80 -3.64 (m, 2 H) 3.62 - 3.44 (m, 4 H) 3.24 - 3.07 (m, 1 H) 2.84 - 2.66 (m, 1 H) 2.49 - 2.26 (m, 1 H) 2.02 (s, 3 H). | MS: calc'd (MH+) 494 exp (MH+) 494.1. |
| 49 | ¹H NMR(MeOD-d₄, 400 MHz), 8.10 -7.98 (m, 1 H) 7.94 - 7.76 (m, 1 H) 7.62 - 7.43 (m, 2 H) 7.10 (s, 2 H) 4.27 (s, 1 H) 3.95 (s, 1 H) 3.76 -3.53 (m, 2 H) 3.47 (s, 3 H) 3.09 (s, 4 H) 3.00 (s, 3 H) 2.91 - 2.75 (m, 1H) 2.43 - 2.21 (m, 1 H) 1.97 (s, 3 H). | MS: calc'd (MH+) 522 exp (MH+) 522.0. |
| 50 | ¹H NMR(MeOD-d₄, 400 MHz), 8.05 - 7.89 (m, 1 H) 7.87 - 7.69 (m, 1 H) 7.66 - 7.33 (m, 2 H) 7.07 (br. s., 2 H) 3.92 - 3.59 (m, 3 H) 3.57 - 3.39 (m, 4 H) 3.27 - 2.95 (m, 1 H) 2.90 - 2.58 (m, 4 H) 2.39 -2.23 (m, 1 H) 2.02 - 1.77 (m, 3H). | MS: calc'd (MH+) 508 exp (MH+) 508.2. |
| 51 | ¹H NMR(MeOD-d₄, 400 MHz), 8.14 - 8.03 (m, 1 H) 8.00 - 7.91(m, 1 H) 7.65 - 7.51 (m, 2 H) 7.11 (s, 2 H) 4.03 - 3.81 (m, 3 H) 3.63 (d, *J* = 11.04 Hz, 1 H) 3.50 (s, 3 H) 3.28 - 3.10 (m, 1 H) 291 - 2.73 (m, 1 H) 2.68 (s, 3 H) 2.49 (qd, *J* = 14.01, 7.15 Hz, 1 H) 2.06 (s, 3 H). | MS: calc'd (MH+) 572 exp (MH+) 572.2. |
| 52 | ¹H NMR(MeOD-d₄, 400 MHz), 8.00 -7.84 (m, 1 H) 7.82 - 7.62 (m, 1 H) 7.60 - 7.29(m, 3 H) 7.13 (br. s., 1 H) 7.03 (br. s., 2 H) 4.10 - 3.76 (m, 3 H) 3.72 -3.55 (m, 1 H) 3.45 (s, 3 H) 3.25 - 3.08 (m, 1 H) 2.95 - 2.73 (m, 1 H) 2.63 - 2.35 (m, 1 H) 1.97 - 1.64 (m, 3 H). | MS: calc'd (MH+) 577 exp (MH+) 577.3. |
| 53 | ¹H NMR (MeOD-d₄, 400 MHz), 7.86 - 8.06 (m, 2 H) 7.63 (dd, *J* = 8.53, 5.27 Hz, 2 H) 7.13 (t, *J* = 8.16 Hz, 2 H) 4.41 - 4.73 (m, 2 H) 4.02 - 4.24 (m,2 H) 3.87 - 3.98 (m, 3 H) 3.72-3.65 (m, 2 H) 3.35 - 3.55 (m, 5 H) 2.09 (d, *J* = 4.52 Hz, 3 H). | MS: calc'd (MH+) 461 exp (MH+) 461. |
| 54 | ¹H NMR (CDCl₃, 400 MHz), 7.81 - 7.91 (m, 1 H), 7.38 - 7.66 (m, 3 H), 7.03 (br. s., 2 H), 4.10 - 4.28 (m, 1 H), 3.88 - 4.09 (m, 1 H), 3.45 - 3.59 (m, 3 H), 3.29 - 3.45 (m, 1 H), 3.23 (t, *J* = 8.41 Hz, 1 H), 2.87 - 3.09 (m, 1 H), 2.41 (dd, *J* = 14.31, 7.28 Hz, 1 H), 2.19 (s, 1 H), 1.86 - 2.00 (m, 3 H), 1.18 - 1.33 (m, 6 H). | MS: calc'd (MH+) 509 exp (MH+) 509. |
| 55 | ¹H NMR (CDCl₃, 400 MHz), 7.86 (d, 1H, *J* = 3.1 Hz), 7.52 (m, 3H), 7.02 (m, 2H), 4.04 (m, 1H), 3.79 (m, 2H), 3.52 (s, 3H), 3.52 (m, 2H), 3.16 (m, 1H), 3.00 (m, 1H), 2.40 (m, 2H), 1.98 (s, 3H). | MS: calc'd (MH+) 481 exp (MH+) 481. |
| 56 | ¹H NMR (MeOD-d₄, 400 MHz), 7.95 (d, *J* = 4.0 Hz, 1H), 7.75 (d, *J* = 4.0 Hz, 1H), 7.51-7.47 (m, 2H), 7.05 (t, *J* = 8.0 Hz, 2H), 3.96 (d, *J* = 16 Hz, 1H), 3.74 (d, *J* = 16 Hz, 1H), 3.58 (t, *J* = 6.0 Hz, 2H), 3.50-3.40 (m, 1H), 3.48 (s, 3H), 2.98-2.85 (m, 2H), 2.57-2.45 (m, 1H), 2.13-2.05 (m, 1H), 1.92-1.85 (m, 1H), 1.90 (s, 3H), 1.63-1.49 (m, 3H). | MS: calc'd (MH+) 509 exp (MH+) 509. |
| 57 | ¹H NMR(MeOD-d4, 400 MHz), 7.97 (br. s., 1 H) 7.83 - 7.68 (m, 1 H) 7.44 (br. s., 2 H) 7.03 (br. s., 2 H) 4.55 - 4.37 (m, 1 H) 3.97 (d, *J* = 3.26 Hz, 2 H) 3.67 - 3.53 (m, 1 H) 3.45 (s, 3 H) 3.27 - 3.13(m, 1 H) 2.96 - 2.66 (m, 2 H) 2.36 (s, 3 H) 1.84 (s, 3 H). | MS: calc'd (MH+) 533 exp (MH+) 533.2. |
| 58 | ¹H NMR(MeOD-d₄, 400 MHz), 8.02 - 7.92 (m, 1 H) 7.77 (d, *J* = 3.01 Hz, 1 H) 7.48 (dd, *J* = 8.53, 5.27 Hz, 2 H) 7.05 (t, *J* = 8.66 Hz, 2 H) 4.50 (t, *J* = 8.28 Hz, 1 H) 3.72 - 3.57(m, 2 H) 3.41 (s, 3 H) 3.24 - 3.08 (m, 2 H) 2.80 (td, *J* = 15.75, 7.15 Hz, 2 H) 1.85 (s, 3 H). | MS: calc'd (MH+) 519 exp (MH+) 519.1. |
| 59 | ¹H NMR(MeOD-d₄, 400 MHz), 7.95 (d, *J* = 3.01 Hz, 1 H) 7.74 (d, *J* = 3.26 Hz, 1 H) 7.47 (dd, *J* = 8.66, 5.40 Hz, 2 H) 7.04 (t, *J=* 8.78 Hz, 2 H) 4.53 (t, *J* = 7.91 Hz, 1 H) 3.95 (d, *J* = 8.53 Hz, 2 H) 3.60 (d, *J* = 10.79 Hz, 1 H) 3.50 -3.39 (m, 3 H) 3.26 (br. s., 1 H) 2.99 - 2.85(m, 1 H) 2.78 - 2.64 (m, 1 H) 2.35 (s, 3 H) 1.90 - 1.81 (m, 3 H). | MS: calc'd (MH+) 533 exp (MH+) 533.2. |

More particular compounds of formula I include the following:
4-[6-(4-fluoro-phenyl)-5-methoxycarbonyl-6-methyl-2-thiazol-2-yl-3,6-dihydro-pyrimidin-4-ylmethyl]-morpholine-3-carboxylic acid;
4-[6-(4-fluoro-phenyl)-5-methoxycarbonyl-6-methyl-2-thiazol-2-yl-3,6-dihydro-pyrimidin-4-ylmethyl]-morpholine-(*S*)-3-carboxylic acid;
(*S*)-4-[6-(4-fluoro-phenyl)-5-methoxycarbonyl-6-methyl-2-thiazol-2-yl-3,6-dihydro-pyrimidin-4-ylmethyl]-morpholine-(*S*)-3-carboxylic acid;
(*S*)-4-[6-(4-fluoro-phenyl)-5-methoxycarbonyl-6-methyl-2-thiazol-2-yl-3,6-dihydro-pyrimidin-4-ylmethyl]-morpholine-(*R*)-3-carboxylic acid;
4-[6-(4-fluoro-phenyl)-2-(5-fluoro-pyridin-2-yl)-5-methoxycarbonyl-6-methyl-3,6-dihydro-pyrimidin-4-ylmethyl]-morpholine-(*R*)-3-carboxylic acid;
4-[6-(4-fluoro-phenyl)-2-(5-fluoro-pyridin-2-yl)-5-methoxycarbonyl-6-methyl-3,6-dihydro-pyrimidin-4-ylmethyl]-moipholine-(*S*)-3-carboxylic acid;
6-(2-(*S*)-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester;
6-(2-(R)-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-6-((*S*)-2-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-6-((*S*)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid methyl ester;
4-[6-(3,4-difluoro-phenyl)-5-methoxycarbonyl-6-methyl-2-thiazol-2-yl-3,6-dihydro-pyrimidin-4-ylmethyl]-morpholine-3-carboxylic acid;
6-(4,4-difluoro-2-methoxycarbonyl-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester;
6-(2-carboxy-4,4-difluoro-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester;
6-((*S*)-2-carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester;
6-(2-carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-1-[(*S*)-5-cyano-6-(3,4-difluoro-phenyl)-6-methyl-2-thiazol-2-yl-3,6-dihydro-pyrimidin-4-ylmethyl]-4,4-difluoro-pyrrolidine-2-carboxylic acid;
(*S*)-6-((*S*)-2-Carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester;
(*S*)-6-(2-Carboxy-5,5-difluoro-piperidin-1-ylmethyl)-4-(4-fluoro-phenyl)-4-methyl-2-thiazol-2-yl-1,4-dihydro-pyrimidine-5-carboxylic acid methyl ester;
(*S*)-6-(2-Carboxy-4,4-difluoro-piperidin-1-ylmethyl)-4-(4-fluoro-phenyl)-4-methyl-2-thiazol-2-yl-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-4-methyl-2-(1-methyl-1*H*-imidazol-2-yl)-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-4-methyl-2-(4-methyl-thiazol-2-yl)-1,4-dihydro-pyrimidine-5-carboxylic acid methyl ester;
(*S*)-6-((*S*)-2-Carboxy-4,4-difluoro-pyrrolidin-1-ylmethyl)-2-(5-chloro-thiazol-2-yl)-4-(4-fluoro-phenyl)-4-methyl-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-2-(5-fluoro-thiophen-2-yl)-4-methyl-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-2-(3-fluoro-thiophen-2-yl)-4-methyl-1,4-dihydro-pyrimidine-5-carboxylic acid methyl ester;
(*S*)-6-((*S*)-2-Carboxy-4,4-difluoro-pyrrolidin-1-ylmethyl)-4-(4-fluoro-phenyl)-2-(4-fluoro-thiophen-2-yl)-4-methyl-1,4-dihydro-pyrimidine-5-carboxylic acid methyl ester;
(*S*)-6-((*S*)-4,4-Difluoro-2-methoxycarbonyl-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-6-(2-Carbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-6-((*S*)-2-Dimethylcarbamoyl-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester;
(S)-6-[(S)-4,4-Difluoro-2-(thiazol-2-ylcarbamoyl)-pyrrolidin-1-ylmethyl]-4-(4-fluorophenyl)-4-methyl-2-thiazol-2-yl-1,4-dihydro-pyrimidine-5-carboxylic acid methyl ester;
(*S*)-6-[(*S*)-4,4-Difluoro-2-(1-hydroxy-1-methyl-ethyl)-pyrrolidin-1-ylmethyl]-4-(4-fluoro-phenyl)-4-methyl-2-thiazol-2-yl-1,4-dihydro-pyrimidine-5-carboxylic acid methyl ester;
(*S*)-6-((*S*)-4,4-Difluoro-2-hydroxymethyl-pyrrolidin-1-ylmethyl)-4-(4-fluoro-phenyl)-4-methyl-2-thiazol-2-yl-1,4-dihydro-pyrimidine-5-carboxylic acid methyl ester;
(*S*)-6-[(*S*)-4,4-Difluoro-2-(5-methyl-[1,3,4]oxadiazol-2-yl)-pyrrolidin-1-ylmethyl]-4-(4-fluoro-phenyl)-4-methyl-2-thiazol-2-yl-1,4-dihydro-pyrimidine-5-carboxylic acid methyl ester;
(*S*)-6-[(*S*)-4,4-Difluoro-2-(1*H*-tetrazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester; and
(*S*)-6-[(*S*)-4,4-Difluoro-2-(3-methyl-[1,2,4] oxadiazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester.

It is very important for a drug to have a moderate or low clearance, as this often lead to a good oral bioavailability and high exposure in target organ. Reducing the clearance of a compound or drug could then potentially reduce drastically the daily dose required for efficacy and therefore give a much better safety profile as well. From the examples below, it has been found a marked increase of metabolic stability and liver exposure of 4-methyldihydropyrimidines of current invention.

4-Hydrogen-dihydropyrimidines such as Bay 41-4109 can be oxidized to pyrimidine product **XLVI** when treated with human, rat or mouse liver microsomes. In this experiment, pooled liver microsomes (20 mg/ml) from human, male Wister rat and male CD-1 mouse were obtained from BD Bioscience (Franklin Lakes, NJ USA). Incubation reaction mixtures contained a final concentration of 0.1M sodium phosphate buffer (pH 7.4), 0.5 mg/ml microsomal protein, 5 µM of the tested compounds and 1 mM NADPH in a total volume of 400 µl. The incubations were done for 60 minutes and 300 µl of the mixtures was transferred to 150 µl of ice cold methanol to terminate reactions. After vortexes for 3 minutes and centrifuged at 4000 rpm at 4°C for 10 minutes, the clear supernatant was used directly for analysis. The samples were analyzed by Applied Biosystems API 3200 Q TRAP LC/MS/MS system using electrospray ionization mode.

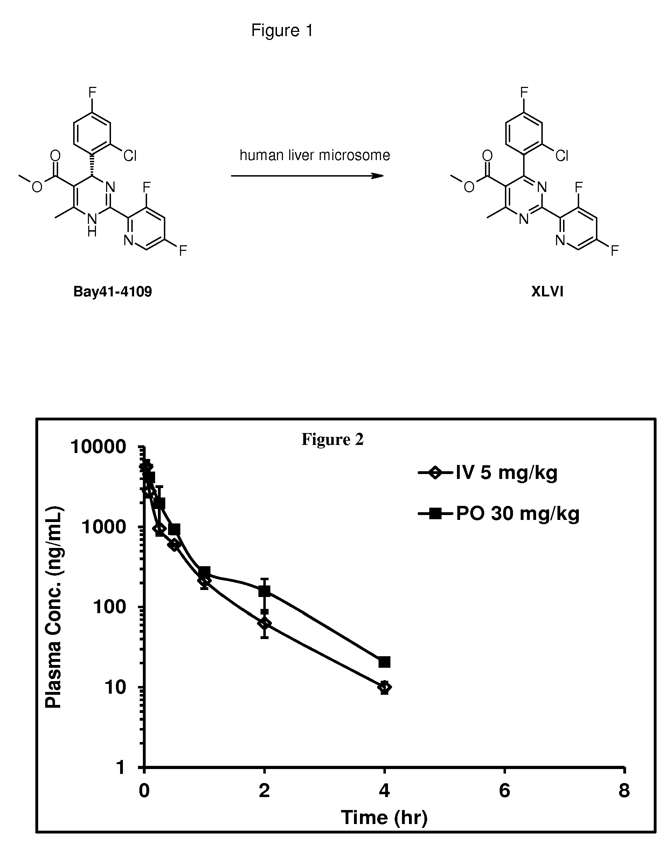

Pyrimidine product **XLVI** was the major metabolite in the *in vitro* clearance tests (Figure 1), and it was inactive to HBV DNA reduction in HepDE19 cell based assays with EC₅₀ value above 100 µM. On the other hand, the 4-methyl-dihydropyrimidines series in this invention do not have the aromatization issues of the core structure.

HBV viruses infect hepatocyte cells and replicate in the liver. To have effective viral suppression, it is important for an anti-HBV drug to have sufficient exposure in the target organ. The following findings highlight increased metabolic stability and high liver exposure of 4-methyldihydropyrimidine analogs in this invention.

In this experiment, fresh mouse liver sample was homogenized by adding saline (1 g liver tissue: 5 mL saline) immediately after collection. After centrifuging for 10 minutes at 14000 rpm, the pooled supernatant was used to prepare liver homogenate solutions. The effective compounds concentrations in liver homogenate were 100, 300, and 1000 ng/mL. Then, they were incubated at rt. After incubation time of 0, 15 and 30 minutes, 180 µl aliquots of MeOH was added into 20 µl of homogenate, respectively. All these samples were vortex mixed for 5 minutes at 1500 rpm and centrifuged for 10 minutes at 14000 rpm. The supernatants were transferred into a 96-well plate for LC-MS/MS analysis. The results were summarized and showed in Table 3.

**Table 3. The percentage of Bay 41-4109 and Example 13 remaining in mouse liver homogenate.**

| **Compound** | **Concentration (ng/mL)** | **Peak Area*** | **Incubation Time (min)** | **Percentage to 0 min** |
|---|---|---|---|---|
| | 100 | 9263 | 15 | 1.84% |
| | | 504715 | 0 | 100% |
| **Bay41-4109** | 300 | N.D. | 30 | 0% |
| | | 1363649 | 0 | 100% |
| | 1000 | 7868 | 30 | 0.16% |
| | | 93431 | 15 | 1.90% |
| | | 4930207 | 0 | 100% |
| | 100 | 58644 | 15 | 119% |
| | | 49480 | 0 | 100% |
| **Example 13** | 300 | 120162 | 30 | 108% |
| | | 111014 | 0 | 100% |
| | 1000 | 368388 | 30 | 86.0% |
| | | 375366 | 15 | 87.6% |
| | | 428458 | 0 | 100% |

| | | | | |
|---|---|---|---|---|
| *: the data directly calculated by LC/MS/MS, the relative standard variation is at 20%. **: not detected | | | | |

By comparing the peak area of individual samples at 15 and 30 min to the one at 0 min from same concentration level, the stability of Bay41-4109 and Example **13** in CD-1 mouse liver homogenate was evaluated.

It can be obviously concluded that Bay41-4109 is not stable in liver homogenate treated with saline. About 2% of compound was detected after 15 minutes room temperature incubation, at three different concentration levels. In samples incubated for 30 minutes, only 0.16% can be found (at 1000 ng/mL. Not detected in 100 ng/mL and 300 ng/mL samples due to instrument sensitivity).

It can be concluded that Example **13** is stable in in liver homogenate treated with saline.

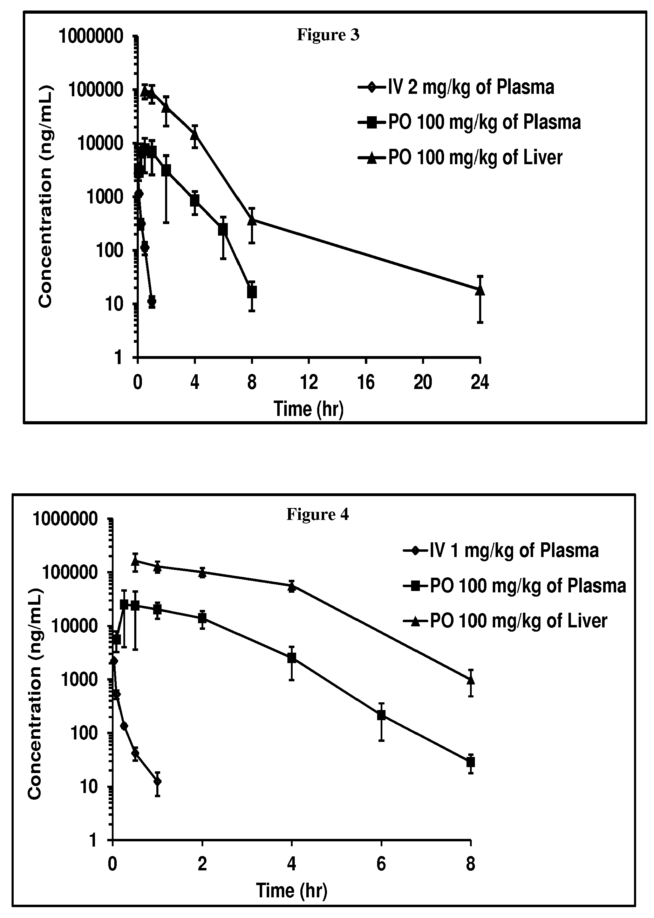

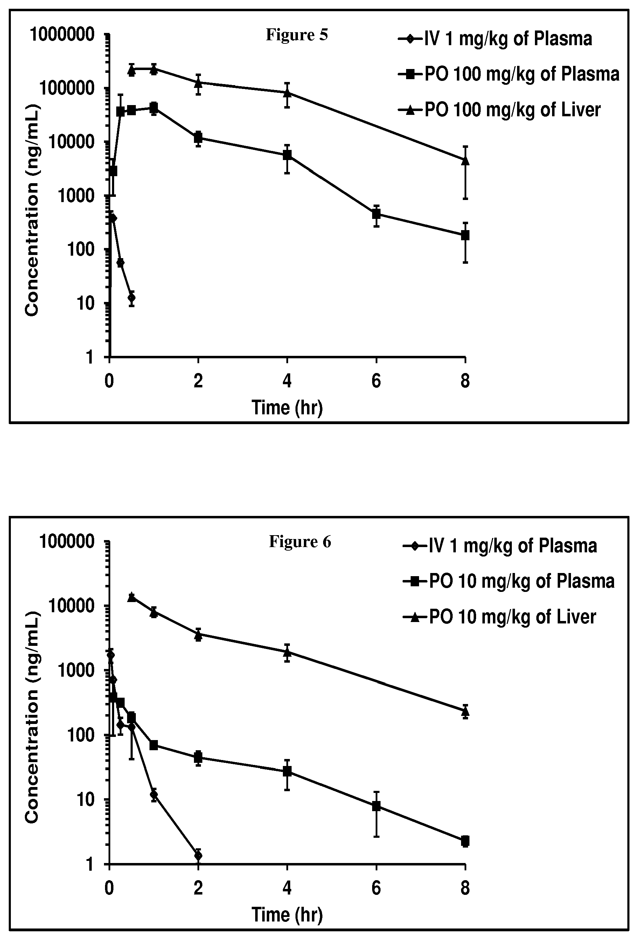

The *in vivo* DMPK of selected compounds were evaluated in male ICR mice following intravenous (or *i*.*v*.) or oral (or *p.o.)* administration. In single dose pharmacokinetics (SDPK) studies, compounds were dissolved in 6% Solutol solution (Solutol: Ethanol, 1:1, v/v), and 94% 0.9% saline for i.v. dose. For p.o. administration, compounds were mixed with 0.89% microcrystalline cellulose and 0.11% carboxymethyl cellulose sodium water solution, or 1% RC591 as suspensions. The single dose exposure levels of Bay 41-4109, Example 6, Example 11, Example 13 and Example 19 in mouse plasma and/or liver are shown as Figure 2-6.

### SYNTHESIS

The compounds of the present invention can be prepared by any conventional means. Suitable processes for synthesizing these compounds as well as their starting materials are provided in the schemes below and in the examples. All substituents, in particular, R¹ to R⁵, M and X are as defined above unless otherwise indicated. Furthermore, and unless explicitly otherwise stated, all reactions, reaction conditions, abbreviations and symbols have the meanings well known to a person of ordinary skill in organic chemistry.

General synthetic scheme for 4-methyl-5-ester-6-methyl-dihydropyrimidine based analogues **Intermediate-1** (Scheme 1)

One category of the compounds described herein relates to 4-methyl-5-ester-6-methyl-dihydropyrimidine based analogues with the formula **Intermediate-1** wherein R⁸ is C₁₋₆alkyl.

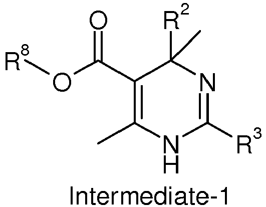

Compound of interest **Intermediate-1** can be prepared according to the general synthesis method shown in Scheme 1.

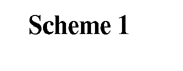

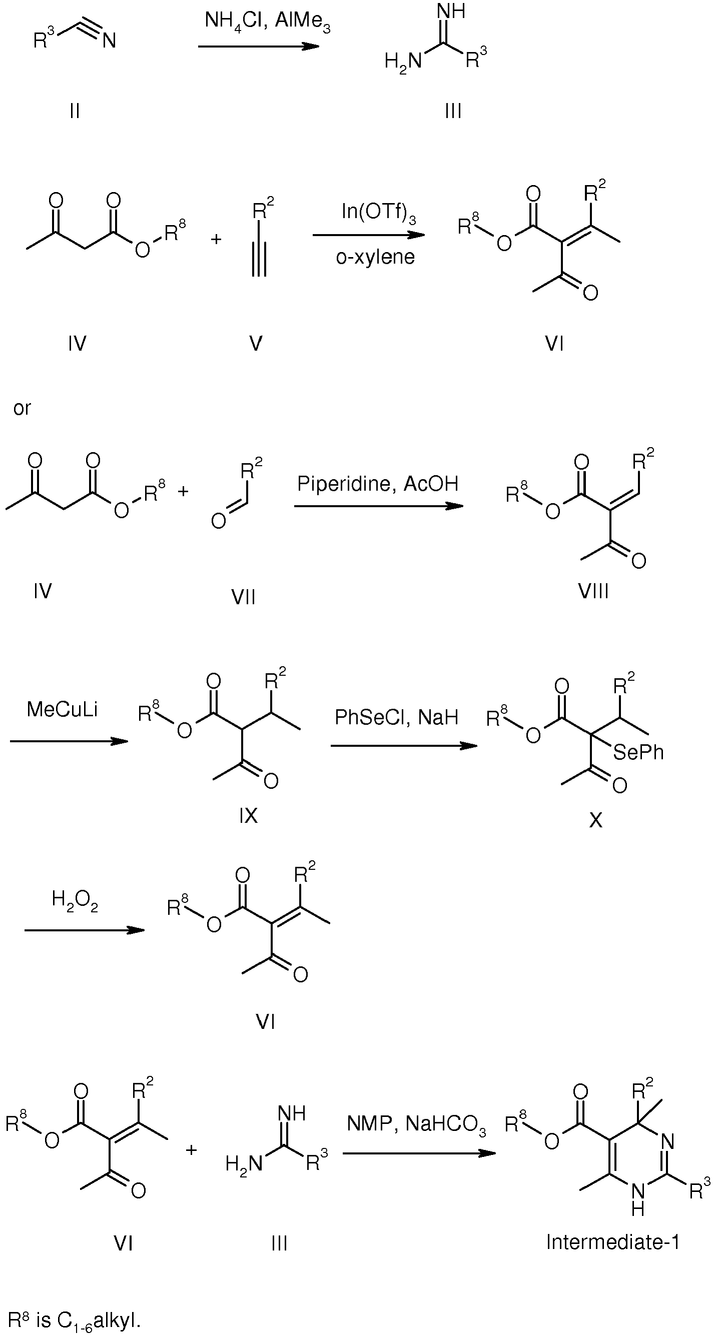

Amidine **III** can be prepared from commercial available nitrile **II,** ammonium chloride and trimethyl aluminum. The reaction is typically performed by adding trimethyl aluminum to the mixture of ammonium chloride in toluene at 0 °C. After 30 minutes, nitrile **II** is added into the flask and the reaction mixture is stirred at 80 °C overnight.

The indium triflate catalyzed condensation reaction of commercial available ester **IV** and ethynyl-benzene **V** gives α,β-unsaturated ketone **VI.** The reaction is typically performed in o-xylene at 120 °C for 2 h.

As an alternative method to synthesize tetra-substituted α,β-unsaturated ketone **VI,** especially when R⁸ is *tert*-butyl group. Ketone **VIII** can be prepared by condensation of ester **IV** with substituted benzaldehyde **VII.** The reaction is typically performed in ethanol with catalytic quantity of piperidine and acetic acid at rt overnight.

Ketone **IX** can be prepared by 1,4-Michael addition of methyl group to the α,β-unsaturated ketone **VIII.** The reaction is typically performed by adding methyl lithium solution to cuprous iodide in THF solution at 0 °C and stirred for 1 hour at 0 °C, then the solution of **VIII** in THF is added into the mixture at -78 °C and stirred for 1 hour at -78 °C.

α,β-Unsaturated ketone **VI** can be prepared by oxidative elimination of ketone **IX.** The reaction is typically performed by adding sodium hydride into the solution of ketone **IX** in THF, then phenylselenyl chloride is added and stirred at rt for 1 hour. After the mixture is treated with pentene, ether and saturated sodium bicarbonate, the organic layer is treated with N₂O₂ solution (30%) and stirred at rt for 1 hour.

Analogs with general structure **Intermediate-1** can be prepared by the condensation reaction of α,β-unsaturated ketone **VI** with amidine **III.** The reaction is typically carried out by adding a solution of **VI** in NMP dropwisely into a mixture of amidine **III** and NaHCO₃ in NMP at 120°C, after addition the mixture is stirred at 120 °C for half an hour before workup.

General synthetic scheme for 4-methyl-5-cyano-6-nitrogen-substituted-2,4-dihydro-pyrimidine based analogues **Intermediate-3** (Scheme-2)

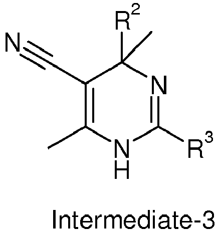

Compounds of interest **Intermediate-3** can be prepared according to the general synthesis method shown in Scheme 2.

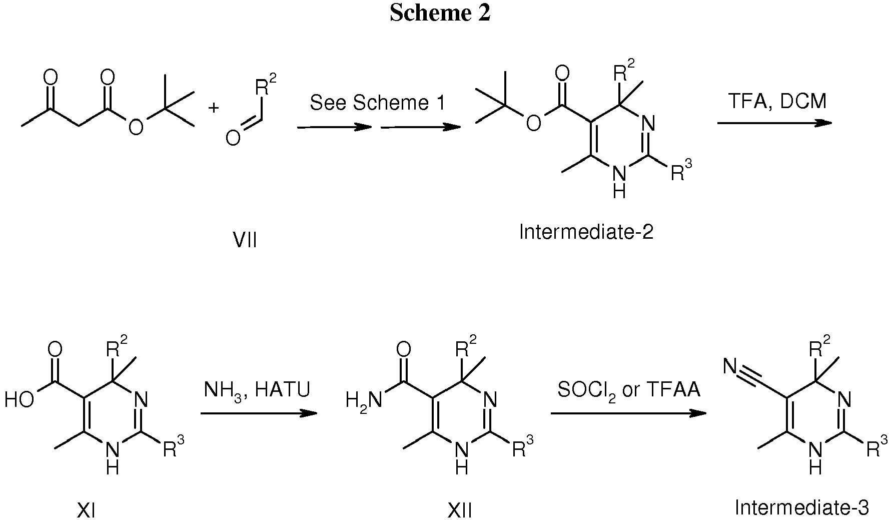

Compound **XI** can be obtained by the deprotection of **Intermediate-2.** The reaction is typically performed in DCM with TFA at rt for 2 hours.

Compound **XII** can be obtained by coupling reaction from **XI** with ammonia. The reaction is typically performed in DCM with HATU and ammonia of dioxane solution at rt for 1 hour.

Cyano compound **Intermediate-3** can be obtained by dehydrate reaction from compound **XII.** The reaction is typically performed in 1,2-dichloroethane with thionyl chloride or trifluoroacetic anhydride under refluxing for 1 hour.

General synthetic scheme for 4-methyl-5-ester or cyano-6-aminoalkyl-dihydropyrimidine based analogues **Ia** (Scheme 3)

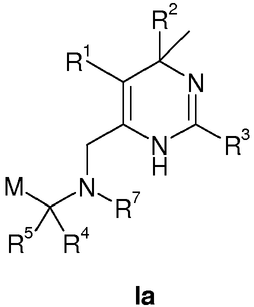

Compounds of interest **Ia** can be prepared according to the general synthesis method shown in Scheme 3.

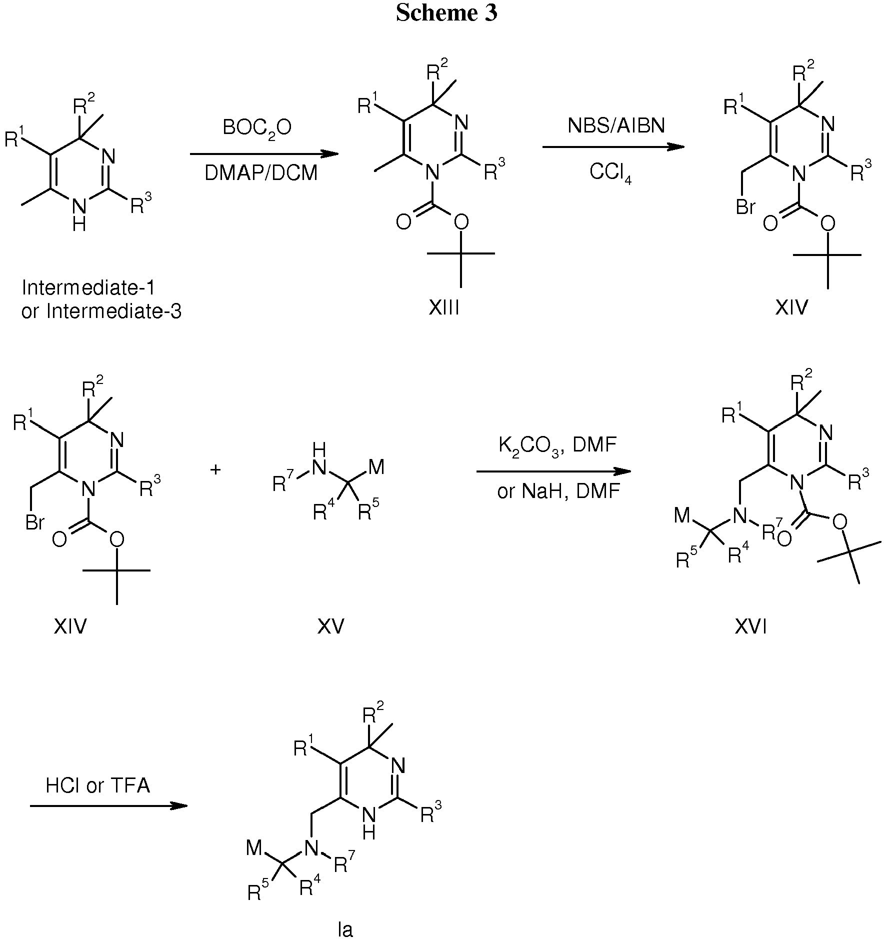

The Boc-protected compound **XIII** can be obtained by treatment of ester **Intermediate-1** or cyano **Intermediate-3** with di-*tert*-butyldicarbonate and DMAP as base in an inert organic solvent such as DCM, typically at rt for 24 hours.

The compound **XIV** can be obtained by the bromination of compound **XIII.** The reaction is typically performed in tetrachloromethane with NBS and AIBN as catalyst at 80 °C for 2 hours.

The amino substituted intermediate **XVI** can be obtained through substitution reaction of compound **XIV** with **XV.** The reaction can be carried out with a suitable organic base such as N,N-diisopropylethylamine, inorganic base such as NaH, Na₂CO₃, or *t*-BuOK in an inert organic solvent such as DCM, THF or DMF at rt or 50 °C for 1-10 hours.

Compound **Ia** can be obtained from the deprotection of **XVI** treated with TFA in DCM or HC1 in MeOH as deprotective agent at rt.

General synthetic scheme for 4-methyl-5-ester or cyano-6-alkoxymethyl-dihydropyrimidine based analogue **Ib** (Scheme 4)

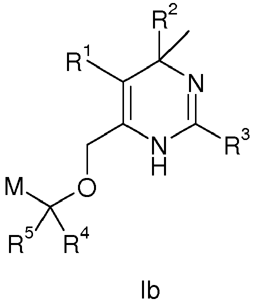

Compound of interest **Ib** can be prepared according to the general synthesis method shown in Scheme 4.

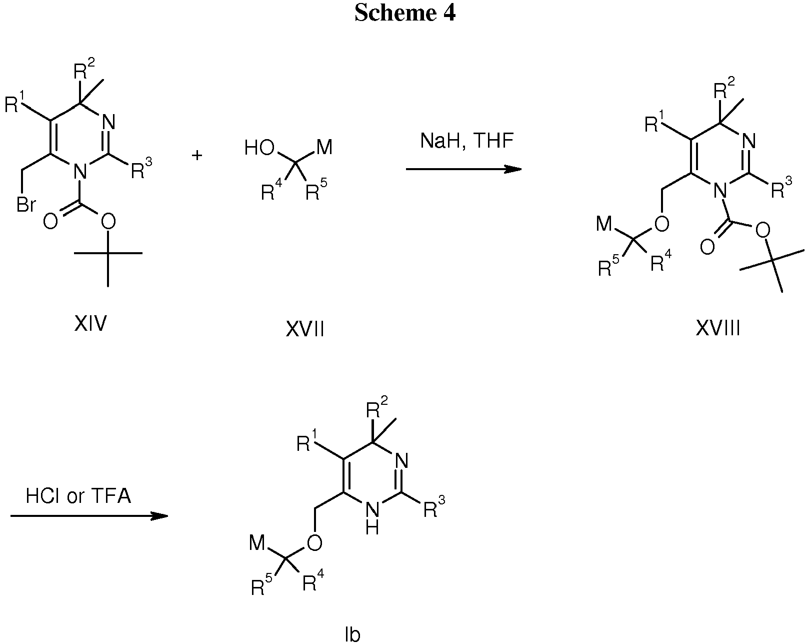

Compound **XVIII** can be obtained by substitution reaction of compound **XIV** with alcohol **XVII.** The reaction is typically performed by adding NaH to the solution of alcohol **XVII** in anhydrous THF at rt, then bromide **XIV** is added into the flask and the mixture is stirred at rt for 3 hours.

Compound **Ib** can be obtained by treating **XVIII** with TFA in DCM or HC1 in MeOH at rt.

This invention also relates to a process for the preparation of a compound of formula I comprising the reaction of
(a) a compound of formula (A) 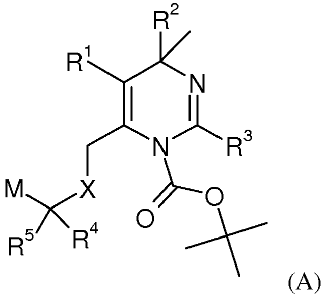
in the presence of an acid;
wherein R¹ to R⁵, M and X are defined above unless otherwise indicated.
In step (a), the acid can be for example TFA or HC1.

### PHARMACEUTICAL COMPOSITIONS AND ADMINISTRATION

The invention also relates to a compound of formula I for use as therapeutically active substance.

A compound of formula (I) when manufactured according to the above process is also an object of the invention.

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compounds of formula I are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular human being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to the suppression of serum HBV DNA levels, or HBeAg seroconversion to HBeAb, or HBsAg loss, or normalization of alanine aminotransferase levels and improvement in liver histology. For example, such amount may be below the amount that is toxic to normal cells, or the human as a whole.

In one example, the pharmaceutically effective amount of the compound of the invention administered parenterally per dose will be in the range of about 0.01 to 100 mg/kg, alternatively about 0.1 to 20 mg/kg of patient body weight per day, with the typical initial range of compound used being 0.3 to 15 mg/kg/day. In another embodiment, oral unit dosage forms, such as tablets and capsules, contain from about 0.1 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 0.1 mg to 1000 mg of the compound of the invention compounded with about 90 mg to 30 mg anhydrous lactose, about 5 mg to 40 mg sodium croscarmellose, about 5 mg to 30 mg polyvinylpyrrolidone (PVP) K30, and about 1 mg to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 mg to 400 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula I, or a stereoisomer or pharmaceutically acceptable salt thereof. In a further embodiment includes a pharmaceutical composition comprising a compound of Formula I, or a stereoisomer or pharmaceutically acceptable salt thereof, together with a pharmaceutically acceptable carrier or excipient.

### INDICATIONS AND METHODS OF TREATMENT

The compounds of the invention can inhibit HBV's de novo DNA synthesis and reduce HBV DNA levels. Accordingly, the compounds of the invention are useful for the treatment or prophylaxis of HBV infection.

The invention relates to the use of a compound of formula I for the treatment or prophylaxis of HBV infection.

The use of a compound of formula I for the preparation of medicaments useful in the treatment or prophylaxis diseases that are related to HBV infection is an object of the invention.

The invention relates in particular to the use of a compound of formula I for the preparation of a medicament for the treatment or prophylaxis of HBV infection.

Another embodiment includes the use of compounds in a method of treating or prophylaxising HBV infection in a the use of compounds in a human in need of such treatment, wherein the method comprises administering to said human a therapeutically effective amount of a compound of Formula I, a stereoisomer, tautomer, prodrug or pharmaceutically acceptable salt thereof.

### COMBINATION THERAPY

The compounds of the invention can be used together with interferon, pegylated interferons, Lamivudine, Adefovir dipivoxil, Entecavir, Telbivudine, and Tenofovir disoproxil for the treatment or prophylaxis of HBV.

### Brief description of the Figures

**Figure 1****.** Bay 41-4109 was converted to XLVI in human liver microsomes.
**Figure 2****.** Mean ± SD Plasma Concentration-Time Curve of Bay41-4109 in Male ICR Mice Following Intravenous and Oral Administration*
   * Drug exposure in liver is not available due to instability of Bay 41-4109 in liver homogenate.
**Figure 3****.** Mean ± SD Plasma and Tissue Concentration-Time Curve of Example 6 in Male ICR Mice Following Intravenous and Oral Administration
**Figure 4****.** Mean ± SD Plasma and Tissue Concentration-Time Curve of Example 11 in Male ICR Mice Following Intravenous and Oral Administration
**Figure 5****.** Mean ± SD Plasma and Tissue Concentration-Time Curve of Example 13 in Male ICR Mice Following Intravenous and Oral Administration
**Figure 6****.** Mean ± SD Plasma and Tissue Concentration-Time Curve of Example 19 in Male ICR Mice Following Intravenous and Oral Administration
**Figure 7****.** X-ray structure of compound XXVII

### EXAMPLES

The invention will be more fully understood by reference to the following examples. They should not, however, be construed as limiting the scope of the invention.

### Abbreviations used herein are as follows:

- AIBN:: azobisisobutyronitrile
- Boc:: *tert*-butoxycarbonyl
- *t*-BuOK:: potassium *tert*-butoxide
- calc'd:: calculated
- CC₅₀:: cytotoxic concentration 50%
- CC₁₄:: tetrachloromethane
- CDCl₃:: deuterated chloroform
- CCK-8:: cell counting kit-8
- CDI:: *N*,*N'*-Carbonyldiimidazole
- CMV:: cytomegalovirus
- d:: day
- DIPEA:: *N*,*N*-diisopropylethylamine
- DCM:: dichloromethylene
- DMAP:: *N*,*N'*-dimethylaminopyridine
- DMF:: dimethylformamide
- DMSO:: dimethylsulfoxide
- DNA:: deoxyribonucleic acid
- EDCI:: 1-ethyl-3-(3-dimethylaminopropyl)carbodiimide
- EDTA:: ethylenediaminetetraacetic acid
- exp:: expected
- EtOAc:: ethyl acetate
- FBS:: fetal bovine serum
- g:: gram
- EC₅₀:: concentration required for 50% induction of acetylated tubulin
- FBS:: fetal bovine serum
- h:: hour or hours
- HAP:: heteroaryldihydropyrimidine
- HATU:: 2-(7-Aza-1H-benzotriazole-1-yl)-1,1,3,3-tetramethyluronium hexafluorophosphate
- HBeAb:: hepatitis B e antibody
- HBeAg:: hepatitis B e antigen
- HBsAg:: hepatitis B surface antigen
- HCl:: hydrogen chloride
- HPLC:: high performance liquid chromatography
- Hz:: Hertz
- In(OTf)₃:: indium (III) trifluoromethanesulfonate
- IPA:: isopropanol
- KOH:: potassium
- LC/MS:: liquid chromatography mass spectrometer
- LiOH:: lithium hydroxide
- LDA:: lithium diisopropylamide
- MeOD-d4 or CD3OD:: deuterated methanol
- MeOH:: methanol
- mg:: milligram
- MHz:: megahertz
- min:: minute or minutes
- mL:: milliliter
- mM:: milliliter
- NMP:: 1-methyl-piperidin-2-one
- mmol:: millimole
- NaCl:: sodium chloride
- NaOH:: sodium hydroxide
- NBS:: N-bromosuccinimide
- NEt₃:: triethylamine
- NMR:: nuclear magnetic resonance
- PBS:: Phosphate buffered saline
- prep-HPLC:: preparative high performance liquid chromatography
- RP-HPLC:: reverse phase high performance liquid chromatography
- rt:: room temperature
- SDPK:: single dose pharmacokinetics
- SFC:: supercritical fluid chromatography
- SSC:: saline-sodium citrate buffer
- TEA:: triethylamine
- TFA:: trifluoroacetic acid
- TFAA:: trifluoroacetic acid anhydride
- THF:: tetrahydrofuran
- µl:: microliter
- µM:: micromole

### General Experimental Conditions

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µM; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge™ Prep-C₁₈ (5 µm, OBD™ 30 × 100 mm) column or SunFire™ Prep-C₁₈ (5 µm, OBD™ 30 × 100 mm) column. Waters AutoP purification System (Column: XBridge™ Prep-C₁₈, 30 × 100 mm, Sample Manager 2767, Pump 2525, Detector: Micromass ZQ and UV 2487, solvent system: acetonitrile and 0.1% ammonium hydroxide in water). For SFC chiral separation, intermediates were separated by chiral column (Daicel chiralpak IC, 5 µm, 30 × 250 mm) column using Mettler Toledo SFC-**Multigram III** system, solvent system: 95% CO₂ and 5% IPA (0.5% TEA in IPA), back pressure 100bar, detection UV@ 254nm.

LC/MS spectra of compounds were obtained using a LC/MS (Waters™ Alliance 2795-Micromass ZQ). LC/MS conditions were as follows (running time 6 min):
Acidic condition: A: 0.1% formic acid in H₂O; B: 0.1% formic acid in acetonitrile;
Basic condition: A: 0.01% NH₃·H₂O in H₂O; B: acetonitrile;
Neutral condition: A: H₂O; B: acetonitrile.

Mass spectra (MS): generally only ions which indicate the parent mass are reported, and unless otherwise stated the mass ion quoted is the positive mass ion (M+H)⁺.

LC-MS/MS instrument on liver homogenate stability test: An Agilent 1290 series LC system composited of a binary pump, a degasser, a CTCPAL autosampler and a thermostatted column was applied. The Chromatographic separation was achieved on a Chromolith Performance RP-18 endcapped (3 × 100 mm) at room temperature.

Mass spectrometric detection was performed on an Agilent 6530 Q-TOF instrument in full scan mode with an AJS ESI interface in positive ionization mode. Data processing was performed with Agilent MassHunter Workstation Data Acquisition B.04.00 and Agilent MassHunter Workstation Qualitative Analysis B.04.00.

The microwave assisted reactions were carried out in a Biotage Initiator Sixty microwave synthesizer.

NMR Spectra were obtained using Bruker Avance 400MHz.

All reactions involving air-sensitive reagents were performed under an argon atmosphere. Reagents were used as received from commercial suppliers without further purification unless otherwise noted.

The following examples were prepared by the general methods outlined in the schemes above. They are intended to illustrate the meaning of the present invention but should by no means represent a limitation within the meaning of the present invention.

### PREPARATIVE EXAMPLES

### Example 1 4-(4-Fluoro-phenyl)-6-(1-methoxycarbonyl-1-methyl-ethoxymethyl)-4-methyl-2-thiazol-2-yl-1,4-dihydro-pyrimidine-5-carboxylic acid methyl ester

The title compound was prepared according to the general synthesis methods shown in Scheme 1 and Scheme 4. A detailed synthesis route is provided as shown in Scheme 5.

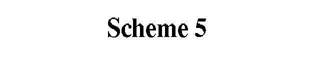

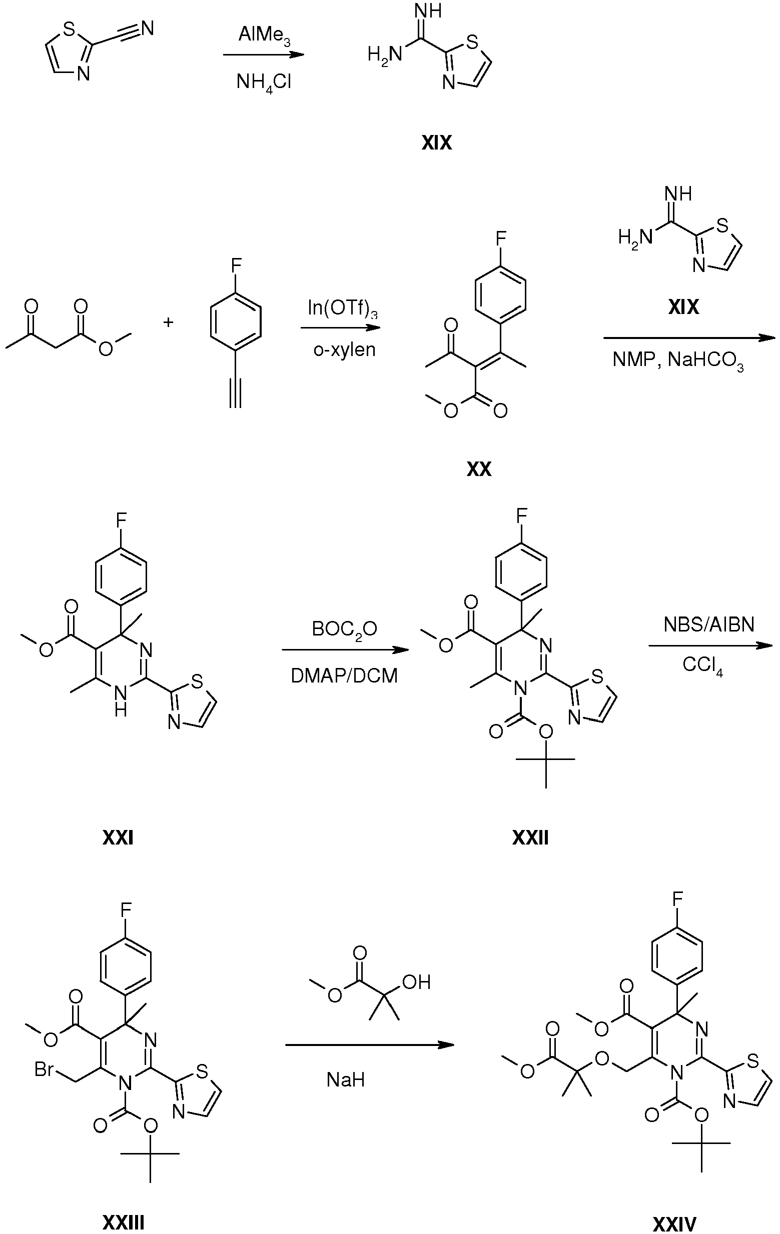

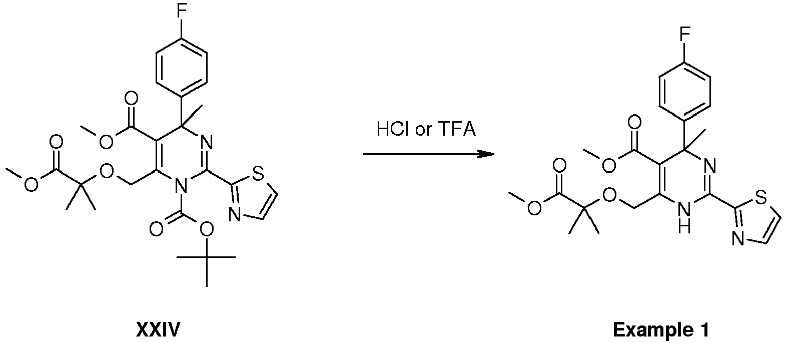

To a stirred suspension of NH₄Cl in toluene (400 mL) was added Al(CH₃)₃ solution (1.0 M, 56 mL) at 0 °C over 30 minutes. After the mixture solution was stirred at rt for 30 minutes, the solution of thiazole-2-carbonitrile (5.24 g, 47.6 mmol) in toluene (10 mL) was added into the flask. The reaction mixture was stirred at 80 °C for 16 hours before cooling to rt and then the mixture was poured into a slurry of silica gel in DCM. After stirring for 20 minutes, the slurry was filtered and washed with MeOH three times and concentrated *in vacuo* to afford crude product of thiazole-2-carboxamidine **XIX** that was used in next step reaction without further purification. MS: calc'd (MH⁺) 128.2, exp (MH⁺) 128.1.

A mixture of 3-oxo-butyric acid methyl ester (5.0 g, 43.1 mmol), 1-ethynyl-4-fluorobenzene (5.0 g, 41.7mmol) and In(OTf)₃ (400 mg, 0.71 mmol) in o-xylene (20 ml) was heated to 120°C for 1 to 2 h. After solvent removal, the residue was purified by column chromatography (EtOAc/petroleum ether: 1/10) to afford **XX** as light yellowish oil (4.0 g, yield: 40.8%).

To a mixture of thiazole-2-carboxamidine **XIX** (1.7g, 10.5 mmol) and NaHCO₃ (2.5 g, 29.8 mmol) in NMP (10 mL) which was preheated to 120°C was added dropwisely (E)-2-acetyl-3-(4-fluoro-phenyl)-but-2-enoic acid methyl ester **XX** (2.4 g, 10.2 mmol) in NMP (10 mL) in about 1 h. The mixture was stirred at 120°C for 0.5 h before reaction workup with EtOAc (200 mL) and water. The organic layer was dried and concentrated, and the residue was purified by column chromatography (EtOAc/petroleum ether 1/4 to 1/2) to afford 4-(4-fluoro-phenyl)-4,6-dimethyl-2-thiazol-2-yl-1,4-dihydro-pyrimidine-5-carboxylic acid methyl ester **XXI** as yellow viscous oil (1.7 g, yield: 48.3%). MS: calc'd (MH⁺) 346, exp (MH⁺) 346. ¹H NMR (MeOD-*d₄*, 400 MHz) 7.93 (d, 1H, *J =* 3.2 Hz), 7.72 (d, 1H, *J =* 3.2 Hz), 7.51-7.47 (m, 2H), 7.04 (t, 2H, *J* = 8.8 Hz), 3.45 (s, 3H), 2.28 (s, 3H), 1.90 (s, 3H).

To a solution of **XXI** (1.0 g, 2.9 mmol) in DCM (20 mL) was added DMAP (0.15 g, 1.2 mmol) and Boc₂O (0.94 g, 4.3 mmol), and the mixture was stirred overnight. The mixture was washed with water (15 mL) and brine (15 mL). The organic layer was separated, dried over anhydrous Na₂SO₄ and concentrated, and the residue was purified by column chromatography (EtOAc/petroleum ether from 1/4 to 1/3) to afford 4-(4-fluoro-phenyl)-4,6-dimethyl-2-thiazol-2-yl-4H-pyrimidine-1,5-dicarboxylic acid 1-*tert*-butyl ester 5-methyl ester **XXII** as yellow solid (0.91 g, yield: 70.5%).

A solution of **XXII** (0.90 g, 2.02 mmol) and NBS (0.54 g, 3.03 mmol) in CCl₄ (30 mL) was heated to 50 °C, then AIBN (30 mg) was added to initiate the reaction. The mixture was stirred for 2 h. The mixture was purified by column chromatography (EtOAc/petroleum ether from 1/4 to 1/3) to afford 6-bromomethyl-4-(4-fluoro-phenyl)-4-methyl-2-thiazol-2-yl-4H-pyrimidine-1,5-dicarboxylic acid 1-*tert*-butyl ester 5-methyl ester **XXIII** as yellow solid (1.03 g, yield: 97.3%).

Sodium hydride (12 mg, 0.5 mmol) was added to a solution of 2-hydroxy-2-methyl-propionic acid methyl ester (59 mg, 0.5 mmol) in THF at rt. Then the mixture was stirred at rt for 30 min. Compound **XXIII** (105 mg, 0.2 mmol) was added and stirred at rt overnight. Then the mixture was partitioned between water and EtOAc. The organic phase was dried, concentrated and used in the next step without further purification.

The crude product **XXIV** from above step was dissolved in DCM and then TFA was added. The mixture was stirred at rt for 2 hours. The solvent was removed and the residue was purified by prep-HPLC to afford Example 1 as yellow solid (40 mg, yield: 43% for 2 steps). MS: calc'd 462 (MH⁺), exp 462 (MH⁺). ¹H NMR (MeOD-*d₄*, 400MHz), 7.95 (d, 1H, *J* = 3.2 Hz), 7.75 (d, 1H, *J =* 3.2 Hz), 7.49-7.45 (m, 2H), 7.04 (t, 2H, *J* = 8.8 Hz), 4.71-4.62 (m, 2H), 3.80 (s, 3H), 3.43 (s, 3H), 1.92 (s, 3H), 1.57 (s, 6H).

### Example 2 6-(1-Carboxy-2,2,2-trifluoro-ethoxymethyl)-4-(4-fluoro-phenyl)-4-methyl-2-thiazol-2-yl-1,4-dihydro-pyrimidine-5-carboxylic acid methyl ester

The title compound was prepared in analogy to Example 1 in Scheme 5 by using 3,3,3-trifluoro-2-hydroxy-propionic acid methyl ester instead of 2-hydroxy-2-methyl-propionic acid methyl ester, the so-obtained methyl ester was hydrolyzed by LiOH as indicated in Scheme 3. MS: calc'd 488 (MH⁺), exp 488 (MH⁺). ¹H NMR (MeOD-*d₄*, 400MHz), 7.94 (d, 1H, *J* = 2.8 Hz), 7.93 (d, 1H, *J =* 2.8 Hz), 7.53-7.49 (m, 2H), 7.07-7.03 (m, 2H), 4.85-4.78 (m, 2H), 4.40-4.37 (m, 1H), 3.44 (s, 3H), 1.94 (s, 3H).

### Example 3 6-{[(1-Carboxy-cyclopropyl)-methyl-amino]-methyl}-4-(4-fluorophenyl)-4-methyl-2-thiazol-2-yl-1,4-dihydro-pyrimidine-5-carboxylic acid methyl ester

The title compound was prepared according to the synthesis method shown in Scheme 1 and Scheme 3. A detailed synthesis route is provided as shown in Scheme 6.

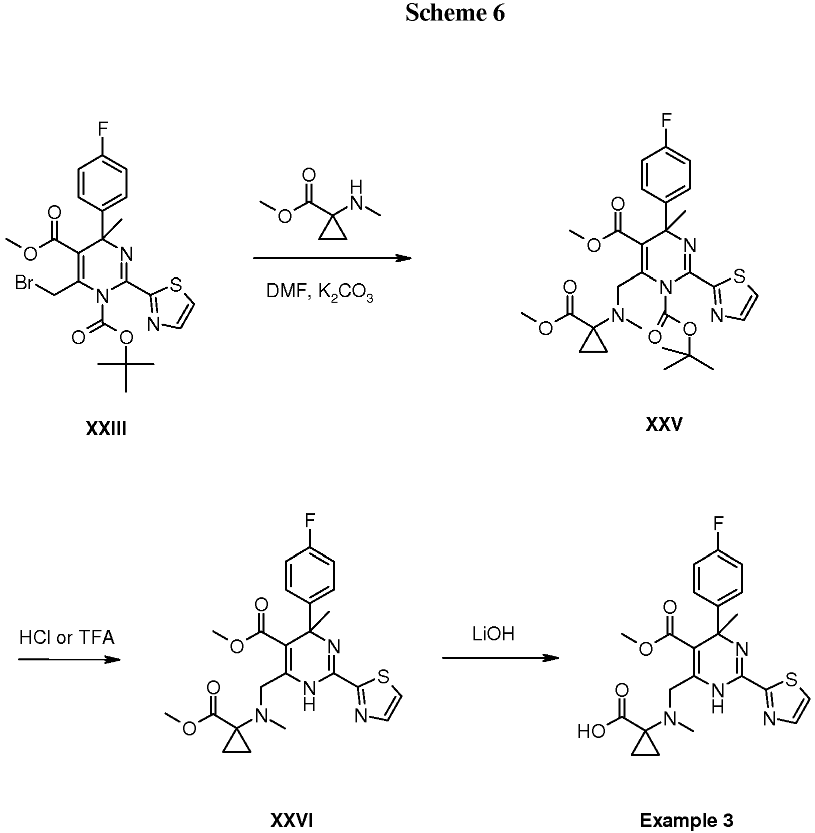

To a solution of 6-bromomethyl-4-(4-fluoro-phenyl)-4-methyl-2-thiazol-2-yl-4H-pyrimidine-1,5-dicarboxylic acid 1-*tert*-butyl ester 5-methyl ester **XXIII** (1.00 g, 1.90 mmol) and potassium carbonate in DMF was added 1-methylamino-cyclopropanecarboxylic acid methyl ester (258 mg, 2.00 mmol), the mixture was stirred at 40 °C for 3 hours and LC-MS indicated that the reaction was finished. The mixture was partitioned between water and ethyl acetate. The organic phase was dried and concentrated to afford the crude product used in the next step without further purification.

The crude product **XXV** from above step was dissolved in DCM and then TFA was added. The mixture was stirred at rt for 2 hours. LC-MS indicated that the reaction was finished. The solvent was removed and the residue was used in the next step without further purification.

The crude product **XXVI** from above step was dissolved in MeOH (5 mL) and LiOH in water (2 mL) was added to the mixture. The mixture was stirred at rt for 2 h and LC-MS indicated that the reaction was finished. The solvent was removed and the mixture was adjusted to pH (3∼5) with diluted hydrochloric acid. The mixture was purified by prep-HPLC to afford Example 3 as yellow solid (0.63 g, yield for 3 steps: 73%). MS: calc'd 459 (MH⁺), exp 459 (MH⁺). ¹H NMR (MeOD-*d₄*, 400MHz), 8.12 (d, 1H, *J =* 3.2 Hz), 8.06 (d, 1H, *J =* 3.2 Hz), 7.63-7.60 (m, 2H), 7.15 (t, 2H, J= 8.8 Hz), 4.30 (s, 2H), 3.51 (s, 3H), 2.79 (s, 3H), 2.10 (s, 3H), 1.52-1.51 (m, 2H), 1.38-1.37 (m, 2H).

### Example 4 4-[6-(4-Fluoro-phenyl)-5-methoxycarbonyl-6-methyl-2-thiazol-2-yl-3,6-dihydro-pyrimidin-4-ylmethyl]-morpholine-3-carboxylic acid

The title compound was prepared in analogy to Example 3 in Scheme 6 by using morpholine-3-carboxylic acid methyl ester instead of 1-methylamino-cyclopropanecarboxylic acid methyl ester. LC-MS: calc'd 475 (MH⁺), exp 475 (MH⁺). ¹H NMR (MeOD-*d₄*, 400 MHz) δ 8.10 (d, 1H), 8.03 (d, 1H), 7.64-7.61 (m, 2H), 7.15-7.12 (m, 2H), 4.32-4.31 (m, 2H), 4.18-4.14 (m, 2H), 3.99-3.72 (m, 3H), 3.51-3.49 (m, 4H), 3.02 (m, 1H), 2.11 (d, 3H).

### Example 5 4-[6-(4-Fluoro-phenyl)-5-methoxycarbonyl-6-methyl-2-thiazol-2-yl-3,6-dihydro-pyrimidin-4-ylmethyl]-morpholine-(S)-3-carboxylic acid methyl ester

The title compound was prepared in analogy to Example 3 in Scheme 6 by using morpholine-(S)-3-carboxylic acid methyl ester instead of 1-methylamino-cyclopropanecarboxylic acid methyl ester.

### Example 6 4-[6-(4-Fluoro-phenyl)-5-methoxycarbonyl-6-methyl-2-thiazol-2-yl-3,6-dihydro-pyrimidin-4-ylmethyl]-moipholine-(S)-3-carboxylic acid

The title compound was prepared by from the hydrolysis of Example 5 with LiOH in MeOH as shown in Scheme 6.

### Example 7 (S)-4-[6-(4-Fluoro-phenyl)-5-methoxycarbonyl-6-methyl-2-thiazol-2-yl-3,6-dihydro-pyrimidin-4-ylmethyl]-morpholine-(S)-3-carboxylic acid

The title compound was prepared according to the synthesis method shown in Scheme 1 and Scheme 3. A detailed synthesis route is provided as shown in Scheme 7.

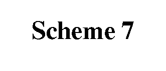

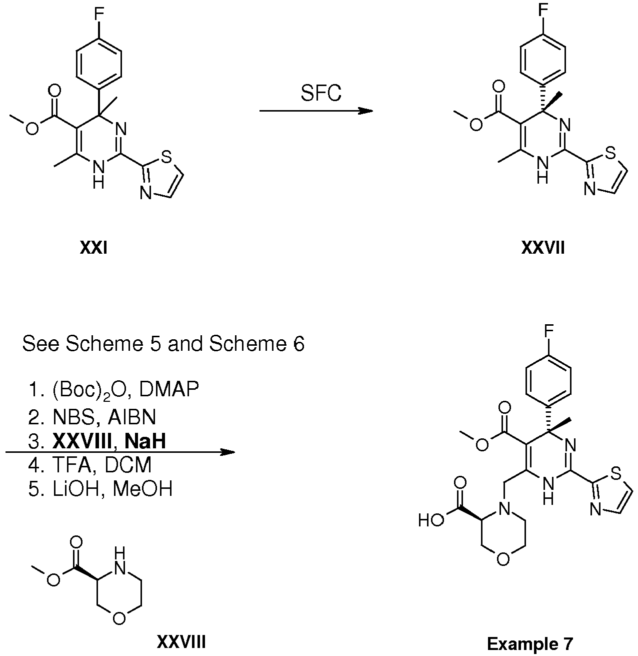

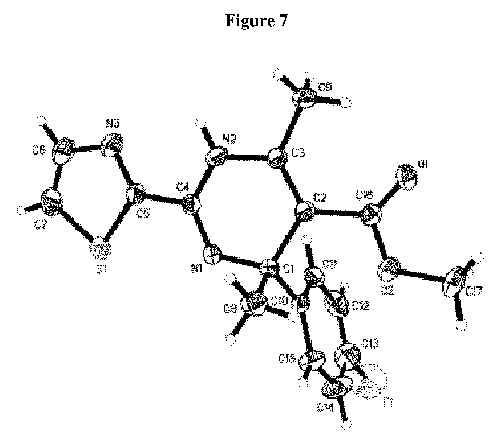

The chiral intermediate compound **XXVII** was separated from XXI by SFC and the absolute stereochemistry was determined by X-ray diffraction study (please see Figure 7). The title compound was prepared in analogy to Example 3 in Scheme 6 by using morpholine-(*S*)-3-carboxylic acid methyl ester **XXVIII** instead of 1-methylamino-cyclopropanecarboxylic acid methyl ester in the replacement reaction.

### Example 8 (S)-4-[6-(4-Fluoro-phenyl)-5-methoxycarbonyl-6-methyl-2-thiazol-2-yl-3,6-dihydro-pyrimidin-4-ylmethyl]-morpholine-(R)-3-carboxylic acid

The title compound was prepared in analogy to Example 7 in Scheme 7 by using morpholine-(*R*)-3-carboxylic acid methyl ester instead of 1-methylamino-cyclopropanecarboxylic acid methyl ester.

### Example 9 4-[6-(4-Fluoro-phenyl)-2-(5-fluoro-pyridin-2-yl)-5-methoxycarbonyl-6-methyl-3,6-dihydro-pyrimidin-4-ylmethyl]-moipholine-(R)-3-carboxylic acid

The title compound was prepared according to the methods shown in Scheme 8.

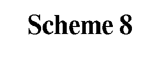

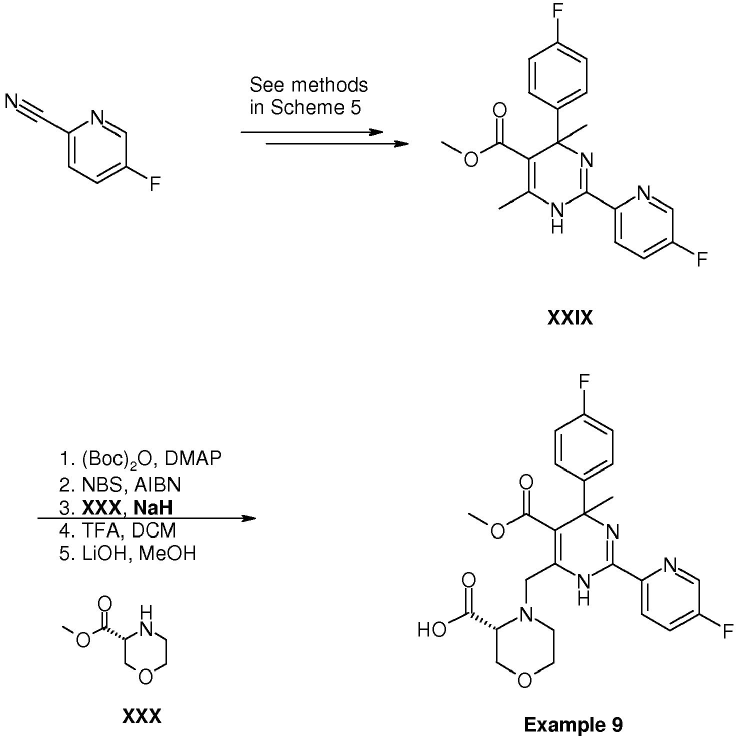

5-Fluoro-pyridine-2-carbonitrile was used in the synthesis of Compound **XXIX** in the same methods as shown in Scheme 5. Following similar procedures to Scheme 5 and Scheme 6, **XXIX** was converted to Example 9 by using morpholine-(*R*)-3-carboxylic acid methyl ester **XXX** in the replacement reaction.

### Example 10 4-[6-(4-Fluoro-phenyl)-2-(5-fluoro-pyridin-2-yl)-5-methoxycarbonyl-6-methyl-3,6-dihydro-pyrimidin-4-ylmethyl]-moipholine-(S)-3-carboxylic acid

The title compound was prepared in analogy to Example 9 in Scheme 8 by using morpholine-(*S*)-3-carboxylic acid methyl ester instead of morpholine-(*R*)-3-carboxylic acid methyl ester in the replacement reaction.

### Example 11 6-(2-(S)-Carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 3 in Scheme 6 by using 4,4-difluoro-pymolidine-(*S*)-2-carboxylic acid methyl ester instead of 1-methylamino-cyclopropanecarboxylic acid methyl ester.

### Example 12 6-(2-(R)-Carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 3 in Scheme 6 by using 4,4-difluoro-pyrrolidine-(*R*)-2-carboxylic acid methyl ester instead of 1-methylamino-cyclopropanecarboxylic acid methyl ester.

### Example 13 (S)-6-((S)-2-Carboxy-4,4-difluoro-pyrrolidin-1-ylmethyl)-4-(4-fluorophenyl)-4-methyl-2-thiazol-2-yl-1,4-dihydro-pyrimidine-5-carboxylic acid methyl ester

The title compound was prepared in analogy to Example 7 in Scheme 7 by using 4,4-difluoro-pyrrolidine-(*S*)-2-carboxylic acid methyl ester instead of using morpholine-(*S*)-3-carboxylic acid methyl ester **XXVIII** in the replacement reaction.

### Example 14 (S)-6-((S)-2-Carboxy-4,4-difluoro-pyrrolidin-1-ylmethyl)-4-(3,4-difluorophenyl)-4-methyl-2-thiazol-2-yl-1,4-dihydro-pyrimidine-5-carboxylic acid methyl ester

The title compound was prepared according to the synthesis method shown in Scheme 1 and Scheme 3. A detailed synthesis route is provided as shown in Scheme 9.

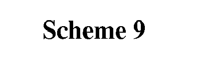

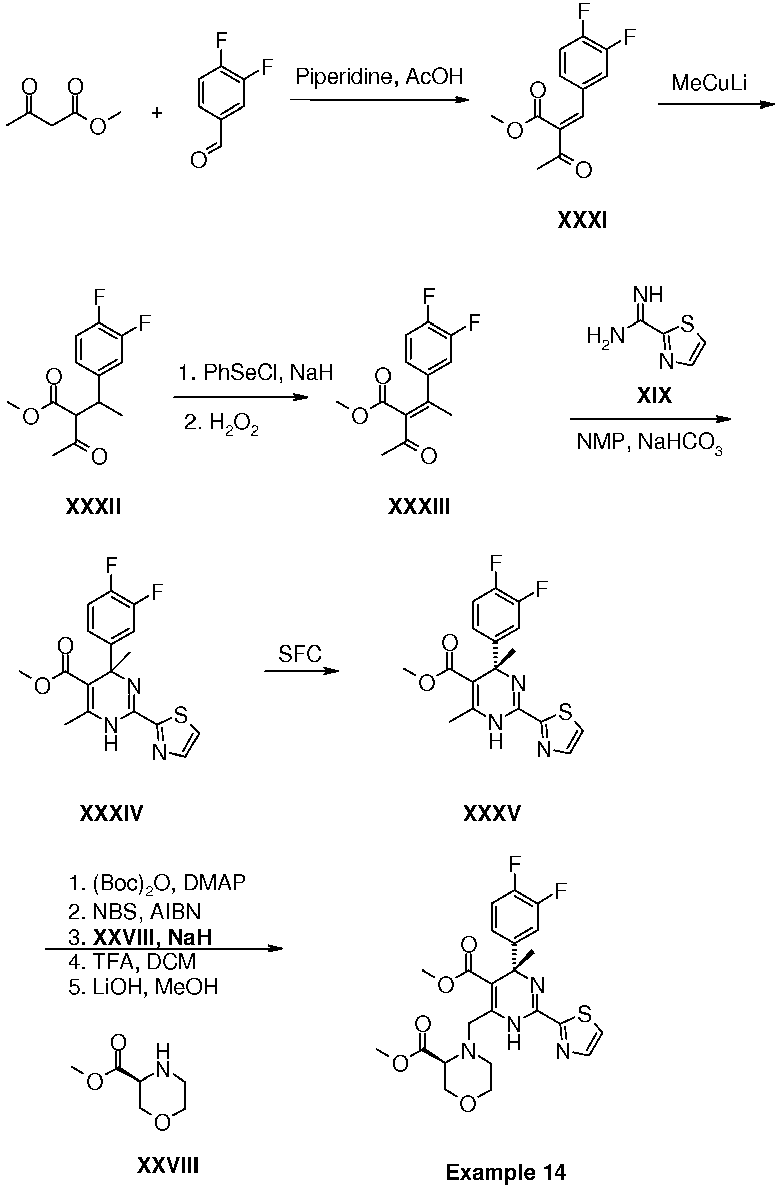

A mixture of 3,4-difluoro-benzaldehyde (8.96 g , 63.1 mmol), 3-oxo-butyric acid methyl ester (7.32 g, 63.1 mmol), piperidine (0.27 g, 3.16 mmol) and acetic acid (0.19 g, 3.16 mmol) in anhydrous ethanol (200 mL) was stirred for 12 hours at room temperature. After removal of the solvent, the residue was purified by flash column chromatography (EtOAc : hexane = 1:10) to afford the product of 2-[1-(3,4-difluoro-phenyl)-meth-(Z)-ylidene]-3-oxo-butyric acid methyl ester **XXXI** as yellow solid (13.6 g). Yield: 90%. MS: calc'd (M⁺+H) 241.0, exp (M⁺+H) 241.1.

A solution of methylithium (1.6 M in ether, 48.7 mL, 78 mmol) was added to a suspension of copper(I) iodide (14.9 g, 78 mmol) in 200 mL of anhydrous THF under argon at 0 °C and the mixture was stirred for 1 hour at 0 °C. A solution of **XXXI** (8.0 g, 31.2 mmol) in 50 mL of anhydrous THF was added dropwisely into the mixture at -78 °C. After stirring at -78 °C for 1 hour, the reaction mixture was quenched with saturated ammonium chloride solution, extracted with EtOAc, washed with brine and dried over anhydrous sodium sulfate. After removal of organic solvent, the residue was purified by flash column chromatography (EtOAc : hexane = 1:10) to afford 6.39 g of **XXXII** as oil. Yield: 80%. MS: calc'd (M⁺+H) 257.1.

NaH (60%, 1.10 g, 27.5 mmol) was added into a solution of **XXXII** (5.0 g, 18.3 mmol) in anhydrous THF (100 mL) under argon. A solution of phenylselenyl chloride (5.3 g, 27.5 mmol) in THF (20 mL) was added into the flask at rt through syringe and the mixture was stirred at rt for 1 h. 60 mL of pentene/ether mixture (v/v =1/1) and 30 mL of saturated NaHCO₃ solution were added into the reaction mixture. The organic layer was separated and washed with brine, and treated with H₂O₂ solution (30%, 4 mL) in DCM (50 mL). The mixture was stirred at rt (for 0.5 ∼2 hours) and diluted with DCM (100 mL). The organic phase was separated, washed with saturated sodium bicarbonate, sodium sulfite, water and brine in sequential and dried over anhydrous sodium sulfate. After removal of organic solvent, the residue was purified by flash column chromatography (EtOAc : hexane = 1:10) to afford 3.97 g of **XXXIII** as yellow oil. MS: calc'd (M⁺+H) 255.1, exp (M⁺+H) 255.1

A mixture of **XXXIII** (2.54 g, 10 mmol), thiazole-2-carboxamidine hydrochloride **XIX** (1.6g, 10 mmol) and sodium bicarbonate (1.68 g, 20 mmol) in NMP (15 mL) was stirred for 3 hours at 120 °C. After cooling, the mixture was separated between water and ethyl acetate. The organic phase was dried and concentrated. The residue was purified to afford the product of 4-(3,4-difluoro-phenyl)-4,6-dimethy 1-2-thiazol-2-yl-1,4-dihydro-pyrimidine-5-carboxylic acid methyl ester **XXXIV** as yellow solid (2.00 g). Yield: 55%. MS: calc'd (M⁺+H) 363.1, exp (M⁺+H) 363.1.

The chiral intermediate **XXXV** was separated from **XXXIV** by SFC and the absolute configuration was assigned through comparing its retention time on SFC with that of the stereochemistry known compound **XXVII.**

The title compound Example 14 was prepared in analogy to Example 7 in Scheme 7 from Compound **XXXV.**

### Example 15 4-[(S)-6-(3,4-Difluoro-phenyl)-5-methoxycarbonyl-6-methyl-2-thiazol-2-yl-3,6-dihydro-pyrimidin-4-ylmethyl]-morpholine-3-carboxylic acid methyl ester

The title compound was prepared by the methods shown in Scheme 10 by using **XXXIV** in the bromination and following replacement reactions, which were carried out in the same procedures as Scheme 5 and Scheme 6.

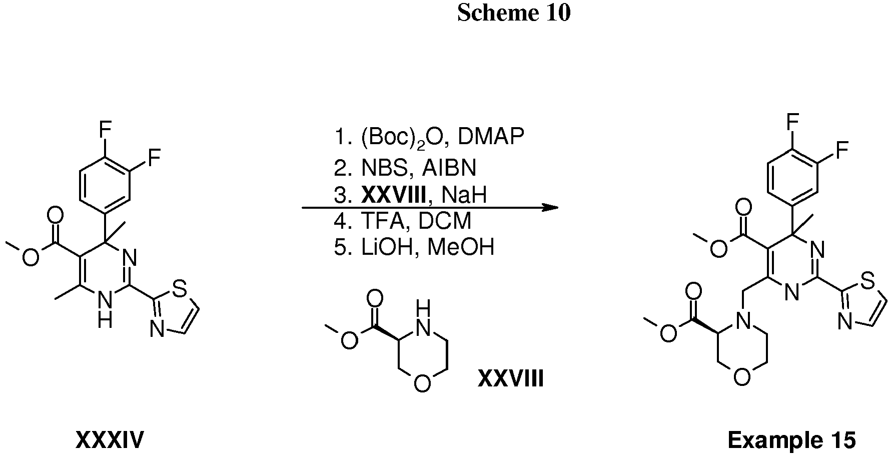

### Example 16 4-[6-(3,4-Difluoro-phenyl)-5-methoxycarbonyl-6-methyl-2-thiazol-2-yl-3,6-dihydro-pyrimidin-4-ylmethyl]-morpholine-3-carboxylic acid

The title compound was obtained by hydrolysis of Example 15 with LiOH in MeOH.

### Example 17 6-(4,4-Difluoro-2-methoxycarbonyl-pyrrolidin-1-ylmethyl)-4-(4-fluorophenyl)-2-(5-fluoro-pyridin-2-yl)-4-methyl-1,4-dihydro-pyrimidine-5-carboxylic acid methyl ester

The title compound was prepared in analogy to Example 9 in Scheme 8 by using 4,4-difluoro-pyrrolidine-(*S*)-2-carboxylic acid methyl ester instead of morpholine-(*R*)-3-carboxylic acid methyl ester **XXXVIII** in the replacement reaction.

### Example 18 (S)-6-((S)-2-Carboxy-4,4-difluoro-pyrrolidin-1-ylmethyl)-4-(4-fluorophenyl)-2-(5-fluoro-pyridin-2-yl)-4-methyl-1,4-dihydro-pyrimidine-5-carboxylic acid methyl ester

The title compound was prepared in the methods as shown in Scheme 11.

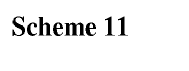

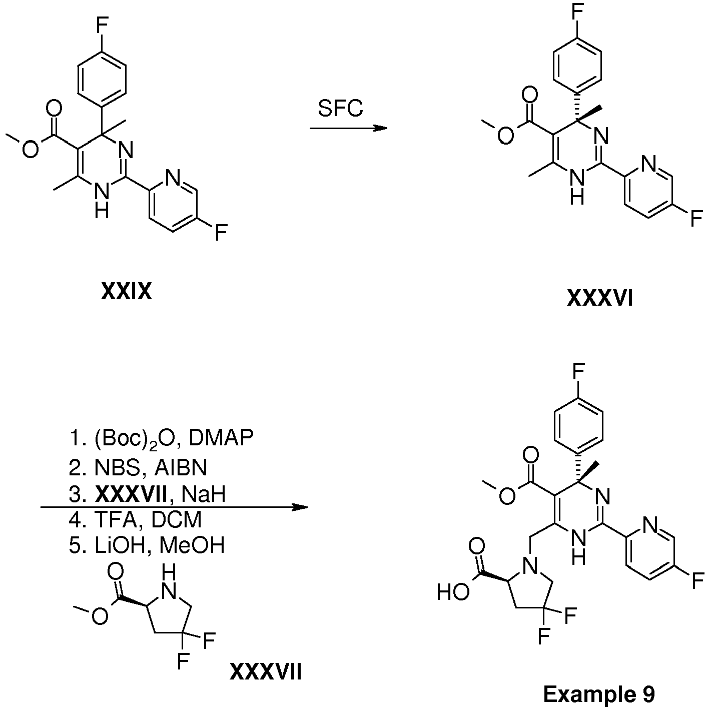

Compound **XXXVI** was obtained by SFC chiral separation of intermediate **XXIX** and the absolute configuration was assigned through comparing its retention time on SFC with that of the stereochemistry known compound **XXVII.** Example 9 was prepared from **XXXVI** by following the procedures in Scheme 5 and Scheme 6, except that 4,4-difluoro-pyrrolidine-(*S*)-2-carboxylic acid methyl ester **XXXVII** was used in the replacement reaction.

### Example 19 6-((S)-2-Carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester

The title compound was prepared in the methods as shown in Scheme 12 by using 5-methyl-thiazole-2-carbonitrile in the preparation of ammidine. Compound **XXXVIII** was obtained by following the procedures in Scheme 5. And 4,4-difluoro-pyrrolidine-(*S*)-2-carboxylic acid methyl ester **XXXVII** was used in the preparation of Example 19, in methods similar to Scheme 5 and Scheme 6.

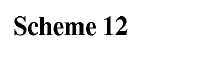

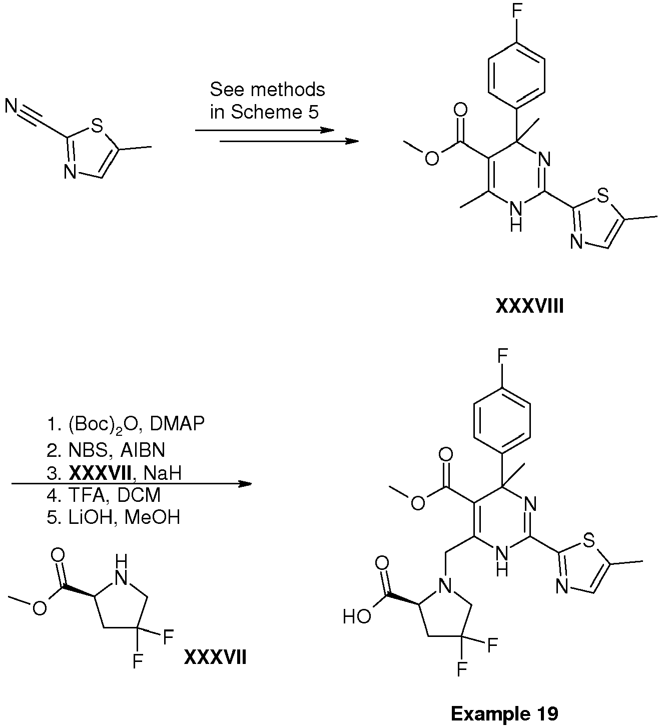

### Example 20 6-(2-Carboxy-4,4-difluoro-2-methyl-pyrrolidin-1-ylmethyl)-4-(4-fluorophenyl)-4-methyl-2-thiazol-2-yl-1,4-dihydro-pyrimidine-5-carboxylic acid methyl ester

The title compound was prepared in analogy to Example 3 in Scheme 6 by using 4,4-difluoro-2-methyl-pyrrolidine-2-carboxylic acid methyl ester instead of morpholine-3-carboxylic acid methyl ester.

### Example 21 (S)-1-[(S)-5-Cyano-6-(4-fluoro-phenyl)-6-methyl-2-thiazol-2-yl-3,6-dihydro-pyrimidin-4-ylmethyl]-4,4-difluoro-pyrrolidine-2-carboxylic acid

The title compound was prepared according to the synthesis method shown in Scheme 2 and Scheme 3. A detailed synthesis route is provided as shown in Scheme 13.

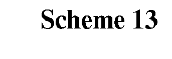

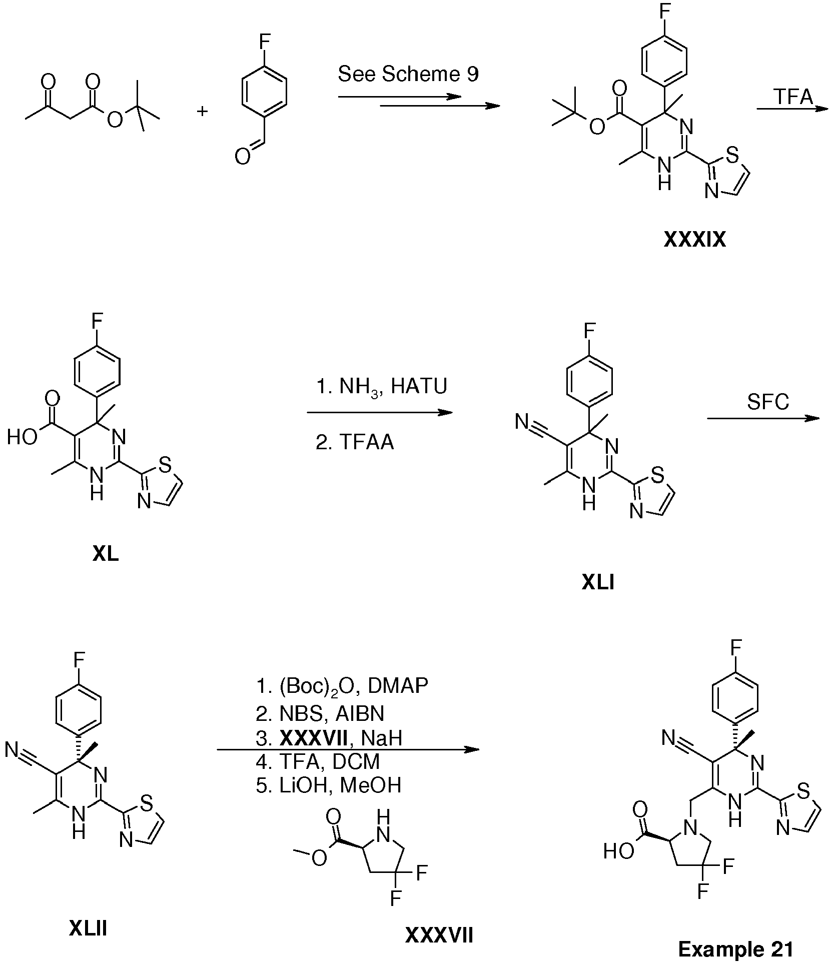

Compound 4-(4-fluoro-phenyl)-4,6-dimethyl-2-thiazol-2-yl-1,4-dihydro-pyrimidine-5-carboxylic acid *tert*-butyl ester **XXXIX** was prepared in analogy to **XXXIV** in Scheme 9 by using 3-oxo-butyric acid *tert*-butyl ester and 4-fluoro-benzaldehyde instead of 3-oxo-butyric acid methyl ester and 3,4-difluoro-benzaldehyde in the condensation reaction.

To a solution of 4-(4-fluoro-phenyl)-4,6-dimethyl-2-thiazol-2-yl-1,4-dihydro-pyrimidine-5-carboxylic acid *tert*-butyl ester **XXXIX** (1.0 g, 2.58mmol) in DCM (15 mL) was added TFA (2 mL), and the mixture was stirred for 3 hr. After that, the solvent and excess TFA was removed in vacuum. The residue **XL** was dissolved in DCM (15 mL), to which was added HATU (1.21 g,3.70 mmol) and NH₃ in dioxane (10 mL, 0.5 M), and the mixture was stirred overnight. The mixture was diluted with DCM (50 mL), and washed with aqueous NaHCO₃ and brine. The organic layer was separated, dried over anhydrous Na₂SO₄ and concentrated to give 0.89 g of 4-(4-fluoro-phenyl)-4,6-dimethyl-2-thiazol-2-yl-1,4-dihydro-pyrimidine-5-carboxylic acid amide as light yellow solid which was directly for next use without purification.

The above crude intermediate (0.89g) was dissolved in THF (10 mL), TFAA (3 mL) was added and the mixture was stirred for 3 hr. After removal of THF and excess TFAA, the residue was dissolved in MeOH (20 mL). To the solution, K₂CO₃ (2.0 g, 14.5 mmol) was added, and the mixture was stirred at rt for 3 hr. Then the mixture was filtered, the solid was washed with EtOAc (10 mL X 2). The combined filtrate was concentrated, the residue was purified by column chromatography (EtOAc/petroleum ether 1/3 to 1/2) to afford **XLI** as yellow solid (700 mg, totally yield: 87.0%).

The chiral intermediate **XLII** was separated from **XLI** by SFC.

The title compound Example 21 was prepared from **XLII** in analogy to Example 3 in Scheme 6.

### Example 22 (S)-4-[5-Cyano-6-(3,4-difluoro-phenyl)-6-methyl-2-thiazol-2-yl-3,6-dihydro-pyrimidin-4-ylmethyl]-morpholine-3-carboxylic acid

The title compound was prepared according to the synthesis method shown in Scheme 2 and Scheme 3. A detailed synthesis route is provided as shown in Scheme 14.

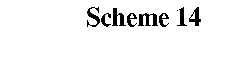

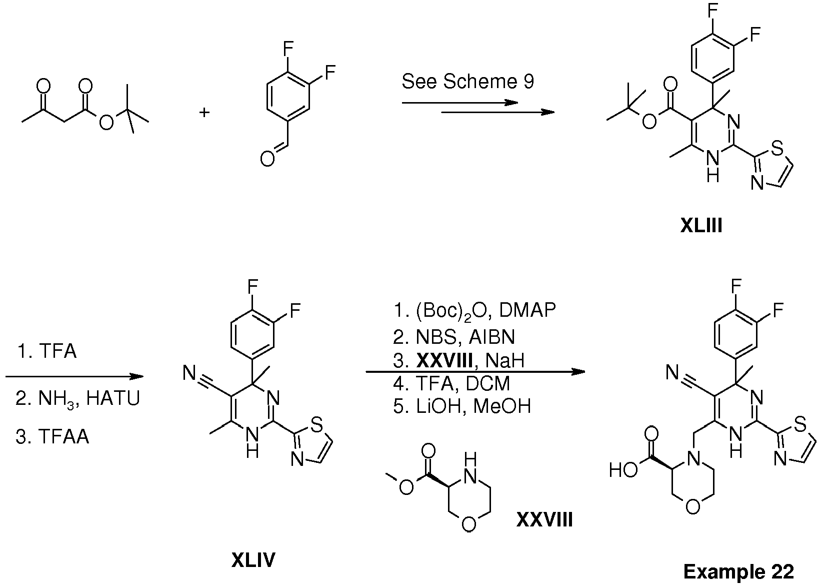

Compound 4-(3,4-difluoro-phenyl)-4,6-dimethyl-2-thiazol-2-yl-1,4-dihydro-pyrimidine-5-carboxylic acid *tert*-butyl ester **XLIII** was prepared in analogy to **XXXIV** in Scheme 9 by using 3-oxo-butyric acid *tert*-butyl ester and 3,4-difluorobenzaldehyde as starting material.

Compound 4-(3,4-difluoro-phenyl)-4,6-dimethyl-2-thiazol-2-yl-1,4-dihydro-pyrimidine-5-carbonitrile **XLIV** was prepared from *tert*-butyl ester **XLIII** in the same method as **XLI** in Scheme 13.

The title compound Example 22 was prepared in the same method as shown in Scheme 5 and Scheme 6 by using morpholine-(*S*)-3-carboxylic acid methyl ester **XXVIII** in the replacement reaction.

### Example 23 (S)-1-[(S)-5-Cyano-6-(3,4-difluoro-phenyl)-6-methyl-2-thiazol-2-yl-3,6-dihydro-pyrimidin-4-ylmethyl]-4,4-difluoro-pyrrolidine-2-carboxylic acid

The title compound was prepared according to the synthesis method shown in Scheme 2 and Scheme 3. A detailed synthesis route is provided as shown in Scheme 15.

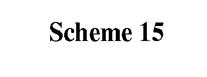

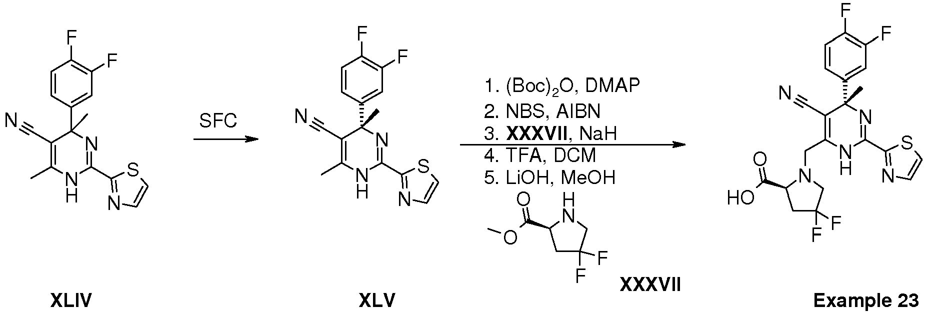

Compound **XLV** was chiral separated from racemate **XLIV** by SFC and the absolute configuration was assigned through comparing its retention time on SFC with that of the stereochemistry known compound **XXVII.** The title compound was prepared in the same method as shown in Scheme 5 and Scheme 6 by using **XXXVII** in the replacement reaction.

### Example 24 Preparation of (S)-6-((S)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester

The title compound was prepared in analogy to Example 7 in Scheme 7 by using 4-ethynyl-1,2-difluoro-benzene instead of (*S*)-morpholine-3-carboxylic acid methyl ester.

### Example 25 Preparation of (S)-6-(2-carboxy-5,5-difluoro-piperidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using5,5-difluoro-piperidine-2-carboxylic acid instead of (*S*)-morpholine-3-carboxylic acid methyl ester.

### Example 26 Preparation of (S)-6-(2-carboxy-4,4-difluoro-piperidin-1-ylmethyl) 4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 4,4-difluoro-piperidine-2-carboxylic acid instead of (*S*)-morpholine-3-carboxylic acid methyl ester.

### Example 27 Preparation of (S)-6-((S)-2-carboxy-4,4-difluoro-pyrrolidin-1-ylmethyl)-4-(4-fluoro-phenyl)-4-methyl-2-(1-methyl-1H-imidazol-2-yl)-1,4-dihydro-pyrimidine-5-carboxylic acid methyl ester

The title compound was prepared in analogy to Example 7 in Scheme 7 by using 1-methyl-1*H*-imidazole-2-carbonitrile instead of thiazole-2-carbonitrile.

### Example 28 Preparation of (R)-6-((S)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester

The title compound was prepared in analogy to Example 7 in Scheme 7 by using 4-ethynyl-1,2-difluoro-benzene instead of 1-ethynyl-4-fluoro-benzene.

### Example 29 Preparation of (S)-4-[6-(3,4-difluoro-phenyl)-5-ethoxycarbonyl-6-methyl-2-thiazol-2-yl-3,6-dihydro-pyrimidin-4-ylmethyl]-morpholine-3-carboxylic acid

The title compound was prepared in analogy to Example 28 by using (*S*)-morpholine-3-carboxylic acid instead of (*S*)-4,4-difluoro-pyrrolidine-2-carboxylic acid.

### Example 30 Preparation of (S)-4-[(S)-6-(4-fluoro-phenyl)-5-methoxycarbonyl-6-methyl-2-(1-methyl-1H-imidazol-2-yl)-3,6-dihydro-pyrimidin-4-ylmethyl]-morpholine-3-carboxylic acid

The title compound was prepared in analogy to Example 27 by using (*S*)-morpholine-3-carboxylic acid instead of (*S*)-4,4-difluoro-pyrrolidine-2-carboxylic acid.

### Example 31 Preparation of (R)-6-((S)-2-carboxy-4,4-difluoro-pyrrolidin-1-ylmethyl)-4-(4-fluoro-phenyl)-4-methyl-2-(4-methyl-thiazol-2-yl)-1,4-dihydro-pyrimidine-5-carboxylic acid methyl ester

The title compound was prepared in analogy to Example 13 by using 4-methyl-thiazole-2-carbonitrile instead of thiazole-2-carbonitrile.

### Example 32 Preparation of (S)-6-((S)-2-carboxy-4,4-difluoro-pyrrolidin-1-ylmethyl)-4-(4-fluoro-phenyl)-4-methyl-2-(4-methyl-thiazol-2-yl)-1,4-dihydro-pyrimidine-5-carboxylic acid methyl ester

The title compound was prepared in analogy to Example 13 by using 4-methyl-thiazole-2-carbonitrile instead of thiazole-2-carbonitrile.

### Example 33 Preparation of (S)-6-((S)-2-carboxy-4,4-difluoro-pyrrolidin-1-ylmethyl)-2-(5-chloro-thiazol-2-yl)-4-(4-fluoro-phenyl)-4-methyl-1,4-dihydro-pyrimidine-5-carboxylic acid methyl ester

The title compound was prepared in analogy to Example 13 by using 5-chloro-thiazole-2-carbonitrile instead of thiazole-2-carbonitrile.

### Example 34 Preparation of (S)-6-((2S,4R)-2-Carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (2*S*,4*R*)-4-fluoro-pyrrolidine-2-carboxylic acid instead of (S)-morpholine-3-carboxylic acid methyl ester.

### Example 35 Preparation of 6-((S)-2-carboxy-4,4-difluoro-pyrrolidin-1-ylmethyl)-4-(4-fluoro-phenyl)-2-isoxazol-3-yl-4-methyl-1,4-dihydro-pyrimidine-5-carboxylic acid methyl ester

The title compound was prepared in analogy to Example 11 by using isoxazole-3-carbonitrile instead of thiazole-2-carbonitrile.

### Example 36 Preparation of (R)-6-((S)-2-carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-2-methyl-pyrrolidine-2-carboxylic acid instead of (*S*)-morpholine-3-carboxylic acid methyl ester.

### Example 37 Preparation of (S)-6-((S)-2-carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester

The title compound was prepared in analogy to Example 13 by using 5-methyl-thiazole-2-carbonitrile instead of thiazole-2-carbonitrile.

### Example 38 Preparation of (S)-6-((S)-2-carboxy-4,4-difluoro-pyrrolidin-1-ylmethyl)-4-(4-fluoro-phenyl)-2-(5-fluoro-thiophen-2-yl)-4-methyl-1,4-dihydro-pyrimidine-5-carboxylic acid methyl ester

The title compound was prepared in analogy to Example 13 by using 5-fluoro-thiophene-2-carbonitrile instead of thiazole-2-carbonitrile.

### Example 39 Preparation of (S)-6-((2S,4S)-2-carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (2S,4S)-4-fluoro-pyrrolidine-2-carboxylic acid instead of (S)-morpholine-3-carboxylic acid methyl ester.

### Example 40 Preparation of 6-((S)-2-carboxy-4,4-difluoro-pyrrolidin-1-ylmethyl)-4-(4-fluoro-phenyl)-4-methyl-2-(5-methyl-isoxazol-3-yl)-1,4-dihydro-pyrimidine-5-carboxylic acid methyl ester

The title compound was prepared in analogy to Example 11 by using 5-methyl-isoxazole-3-carbonitrile instead of thiazole-2-carbonitrile.

### Example 41 Preparation of (S)-6-((S)-2-carboxy-4,4-difluoro-pyrrolidin-1-ylmethyl)-4-(4-fluoro-phenyl)-2-(3-fluoro-thiophen-2-yl)-4-methyl-1,4-dihydro-pyrimidine-5-carboxylic acid methyl ester

The title compound was prepared in analogy to Example 13 by using 3-fluoro-thiophene-2-carbonitrile instead of thiazole-2-carbonitrile.

### Example 42 Preparation of (R)-6-((S)-2-carboxy-4,4-difluoro-pyrrolidin-1-ylmethyl)-4-(4-fluoro-phenyl)-2-(3-fluoro-thiophen-2-yl)-4-methyl-1,4-dihydro-pyrimidine-5-carboxylic acid methyl ester

The title compound was prepared in analogy to Example 13 by using 3-fluoro-thiophene-2-carbonitrile instead of thiazole-2-carbonitrile.

### Example 43 Preparation of (S)-6-((S)-2-carboxy-4,4-difluoro-pyrrolidin-1-ylmethyl)-4-(4-fluoro-phenyl)-2-(4-fluoro-thiophen-2-yl)-4-methyl-1,4-dihydro-pyrimidine-5-carboxylic acid methyl ester

The title compound was prepared in analogy to Example 13 by using 4-fluoro-thiophene-2-carbonitrile instead of thiazole-2-carbonitrile.

### Example 44 Preparation of (S)-6-{[carboxymethyl-(2,2,2-trifluoro-ethyl)-amino]-methyl}-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (2,2,2-trifluoro-ethylamino)-acetic acid instead of (*S*)-morpholine-3-carboxylic acid methyl ester.

### Example 45 Preparation of (S)-6-((S)-4,4-difluoro-2-methoxycarbonyl-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid methyl ester instead of (*S*)-morpholine-3-carboxylic acid methyl ester.

### Example 46 Preparation of (S)-6-[(S)-2-(2-dimethylamino-ethoxycarbonyl)-4,4-difluoro- pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid 2-dimethylamino-ethyl ester instead of (*S*)-morpholine-3-carboxylic acid methyl ester.

### Example 47 Preparation of (S)-6-(2-carbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 4,4-difluoro-pyrrolidine-2-carboxylic acid amide instead of (*S*)-morpholine-3-carboxylic acid methyl ester.

### Example 48 Preparation of (S)-6-((S)-2-carbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid amide instead of (*S*)-morpholine-3-carboxylic acid methyl ester.

### Example 49 Preparation of (S)-6-((S)-2-dimethylcarbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid dimethylamide instead of (*S*)-morpholine-3-carboxylic acid methyl ester.

### Example 50 Preparation of 6-((S)-4,4-difluoro-2-methylcarbamoyl-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid methylamide instead of (*S*)-morpholine-3-carboxylic acid methyl ester.

### Example 51 Preparation of (S)-6-((S)-4,4-difluoro-2-methanesulfonylaminocarbonylpyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using *N*-((*S*)-4,4-difluoro-pyrrolidine-2-carbonyl)-methanesulfonamide instead of (*S*)-morpholine-3-carboxylic acid methyl ester.

### Example 52 Preparation of (S)-6-[(S)-4,4-difluoro-2-(thiazol-2-ylcarbamoyl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using (*S*)-4,4-difluoro-pyrrolidine-2-carboxylic acid thiazol-2-ylamide instead of (*S*)-morpholine-3-carboxylic acid methyl ester.

### Example 53 Preparation of 4-(4-fluoro-phenyl)-6-((R)-3-hydroxymethyl-morpholin-4-ylmethyl)-4-methyl-2-thiazol-2-yl-1,4-dihydro-pyrimidine-5-carboxylic acid methyl ester

The title compound was prepared in analogy to Example 7 in Scheme 7 by using (*R*)-1-morpholin-3-yl-methaol instead of (*S*)-morpholine-3-carboxylic acid methyl ester.

### Example 54 Preparation of (S)-6-[(S)-4,4-difluoro-2-(1-hydroxy-1-methyl-ethyl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 2-((*S*)-4,4-difluoro-pyrrolidin-2-yl)-propan-2-ol instead of (*S*)-morpholine-3-carboxylic acid methyl ester.

### Example 55 Preparation of (S)-6-((S)-4,4-difluoro-2-hydroxymethyl-pyrrolidin-1-ylmethyl)-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using ((*S*)-4,4-difluoro-pyrrolidin-2-yl)-methanol instead of (*S*)-morpholine-3-carboxylic acid methyl ester.

### Example 56 Preparation of (S)-6-[4,4-difluoro-2-(3-hydroxy-propyl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 3-(4,4-difluoro-pyrrolidin-2-yl)-propan-1-ol instead of (*S*)-morpholine-3-carboxylic acid methyl ester.

### Example 57 Preparation of (S)-6-[(S)-4,4-difluoro-2-(5-methyl-[1,3,4]oxadiazol-2-yl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 2-((*S*)-4,4-difluoro-pyrrolidin-2-yl)-5-methyl-[1,3,4]oxadiazole instead of (*S*)-morpholine-3-carboxylic acid methyl ester.

### Example 58 Preparation of (S)-6-[(S)-4,4-difluoro-2-(1H-tetrazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 5-((*S*)-4,4-difluoro-pyrrolidin-2-yl)-1*H*-tetrazole instead of (*S*)-morpholine-3-carboxylic acid methyl ester.

### Example 59 Preparation of (S)-6-[(S)-4,4-difluoro-2-(3-methyl-[1,2,4]oxadiazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester

The title compound was prepared in analogy to Example 7 in Scheme 7 by using 5-((*S*)-4,4-difluoro-pyrrolidin-2-yl)-3-methyl-[1,2,4]oxadiazole instead of (*S*)-morpholine-3-carboxylic acid methyl ester.

### BIOLOGICAL EXAMPLES

### Example 60 HBV inhibition assays (biochemical assay)

Cells and culture conditions: HepDE19 (Haitao Guo et al, Journal of Virology, 81, Nov. 2007, 12472-12484; Richeng Mao etal, Journal of Virology, 85, Jan. 2011, 1048-1057) cells were derived from HepG2 (ATCC, American Type Culture Collection) cells through transfection with pTet-off plasmid (Clontech) that expresses the Tet-responsive transcriptional activator and pTREHBVDE plasmid in which HBV pgRNA expression is controlled by a CMV early promoter with a tetracycline-responsive element. The transfected cells were selected with G418 (also known as *Genticin,* purchased from Invitrogen). In tetracycline-free medium, cells support high levels of HBV DNA replication and HBV virus secretion. These cells were maintained in Dulbecco's modified Eagle's medium (DMEM)-F12 medium (Invitrogen) supplemented with 10% fetal bovine serum, 100 U/ml penicillin, and 100 µg/ml streptomycin, 0.5 mg/ml of G418 and µg/ml tetracycline.

Anti-HBV activity and cytotoxicity: HepDE19 cells were seeded into 96-well plates (3×10⁴ cells/well) with tetracycline-free medium and incubated overnight at 37 °C. The test or control compounds were serially half-log diluted with medium and added into the plates (the final concentration of DMSO kept at 0.5% in each well). Five days after compound treatment, cells were washed with PBS and lysed with 50 mM Tris-1mM EDTA-0.2% CA-630 (pH 8.0) at 37°C for 20 min. After centrifugation to remove nuclei and other debris, the supernatant was transferred into a new plate and incubated with 2M NaOH/20×SSC (3M NaCl, 0.3M Sodium citrate, pH7.0) at rt for 30 min. Then the samples were transferred to nylon membrane and neutralized with1M Tris (pH7.4)/2M NaCl. The presence of HBV DNA was detected by dot-blot with DIG-labeled HBV specific DNA probe and quantified by dot density. The compound concentrations that inhibited HBV DNA by 50% (EC₅₀) were determined (See Table 1).

To determine if the anti-HBV effect of compound is due to cytotoxicity, HepDE19 cells (5×10³ cells/well) were seeded into 96-well plates and compounds were treated as described above. Five days after treatment, cell viability was measured by addition of 20 µl of CCK-8 reagent. Four hours after incubation at 37 °C, the absorbance at wavelengths of 450 nm and 630 nm (OD₄₅₀ and OD₆₃₀) was recorded by a plate reader. The 50% cytotoxic concentration (CC₅₀) of each compounds were determined accordingly.

The compounds of the present invention were tested for their capacity to inhibit a HBV activity and activation as described herein. The Examples were tested in the above assay and found to have EC₅₀ of about 0.01 µM to about 50 µM. Particular compounds of formula I were found to have EC₅₀ of about 0.1µM to about 30 µM.

Results of HepDe19 EC₅₀ (µM) and CC₅₀ (µM) are given in Table 1.

### Example A

A compound of formula I can be used in a manner known per se as the active ingredient for the production of tablets of the following composition:

**Per tablet**

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula I can be used in a manner known per se as the active ingredient for the production of capsules of the following composition:

**Per capsule**

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. Compounds of formula (I) 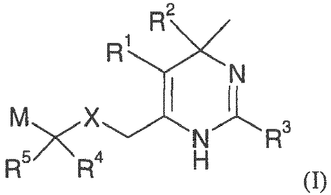 wherein,
R¹ is C₁₋₂alkoxycarbonyl or cyano;
R² is phenyl, which is substituted by halogen;
R³ is thiazolyl, thienyl, imidazolyl, isoxazolyl or pyridinyl; which is unsubstituted or substituted by halogen or C₁₋₆alkyl;
X is oxygen or -NR⁷;
R⁴ and R⁵ are independently selected from hydrogen, C₁₋₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷, form a pyrrolidinyl, morpholinyl or piperidinyl ring, which ring is unsubstituted or substituted by fluoro;
M is C₁₋₆alkoxycarbonyl, carboxy, diC₁₋₆alkylaminoC₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 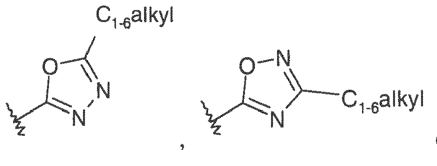 or 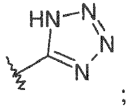
R⁷ is C₁₋₆alkyl or trifluoroC₁₋₆alkyl;
y is 1-6;
or pharmaceutically acceptable salts, or tautomerism isomers thereof.

2. A compound according to claim 1, wherein,
R¹ is C₁₋₂alkoxycarbonyl or cyano;
R² is phenyl, which is once or twice substituted by halogen;
R³ is 2-thiazolyl, which is unsubstituted or once substituted by C₁₋₆alkyl or halogen; or 2-thienyl or 2-pyridinyl, which is once substituted by halogen; or 2-imidazolyl, which is once substituted by C₁₋₆alkyl; or 3-isoxazolyl, which is unsubstituted or once substituted by C₁₋₆alkyl;
X is oxygen or -NR⁷;
R⁴ and R⁵ are independently selected from hydrogen, C₁₋₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷ together form a morpholinyl; or pyrrolidinyl or piperidinyl, which is substituted by fluoro;
M is C₁₋₆alkoxycarbonyl, carboxy, diC₁₋₆alkylamino-C₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 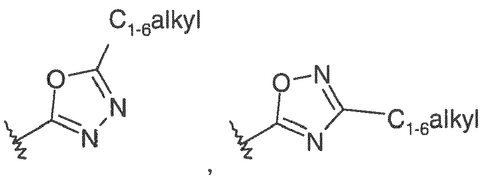 or 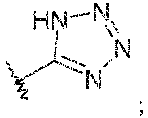
R⁷ is C₁₋₆alkyl or trifluoroC₁₋₆alkyl;
y is 1-6;
or pharmaceutically acceptable salts, or tautomerism isomers thereof.

3. A compound according to claim 1 or 2, wherein
R¹ is methoxycarbonyl, ethoxycarbonyl or cyano;
R² is phenyl substituted once or twice by fluoro;
R^{3 is} 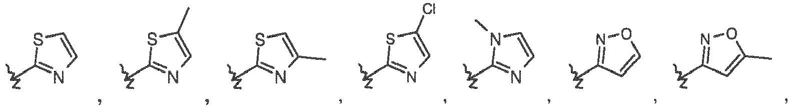 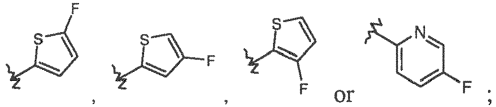
X is oxygen or -NR⁷;
R⁴ and R⁵ are independently selected from hydrogen, methyl and trifluoromethyl; or
R⁴ and R⁵ together with the carbon atom to which they are attached form cyclopropyl; or when X is -NR⁷, one of R⁴ and R⁵ is hydrogen or methyl, and the other one with the carbon atom to which it is attached and -NR⁷ form 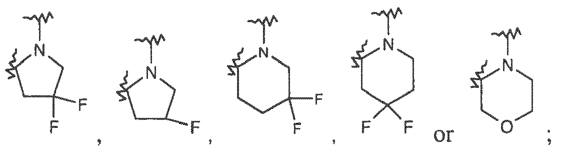
M is methoxycarbonyl, carboxy, dimethylaminoethoxycarbonyl, aminocarbonyl, dimethylaminocarbonyl, methylaminocarbonyl, methylsulfonylaminocarbonyl, 2-thiazolylaminocarbonyl, hydroxymethyl, hydroxypropyl, 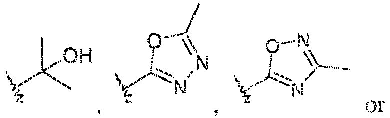 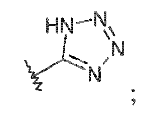
R⁷ is methyl or trifluoroethyl;
or pharmaceutically acceptable salts, or tautomerism isomers thereof.

4. A compound according to claim 1 or 2, wherein.
R¹ is C₁₋₂alkoxycarbonyl;
R² is phenyl which is once substituted by halogen;
R³ is 2-thiazolyl;
X is oxygen;
R⁴ and R⁵ are independently selected from hydrogen, C₁₋₆alkyl and trifluoroC₁₋₆alkyl;
M is C₁₋₆alkoxycarbonyl or carboxy.

5. A compound according to any one of claims 1 to 4, wherein
R¹ is methyoxycarbonyl;
R² is 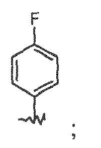
R³ is 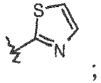
X is oxygen;
R⁴ and R⁵ are independently selected from hydrogen, methyl and trifluoromethyl;
M is methoxycarbonyl or carboxy.

6. A compound according to claim 1 or 2, wherein
R¹ is C₁₋₂alkoxycarbonyl;
R² is phenyl which is once substituted by halogen;
R³ is 2-thiazolyl;
X is -N-C₁₋₆alkyl or -N-trifluoroC₁₋₆alkyl;
R⁴ is hydrogen;
R⁵ is hydrogen;
or R⁴ and R⁵, together with the carbon atom to which they are attached, form a 3 to 7 membered cycloalkyl;
M is carboxy.

7. A compound according to any one of claims 1, 2, 3 or 6, wherein
R¹ is methoxycarbonyl;
R² is 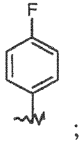
R³ is 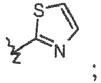
X is -NCH₃ or 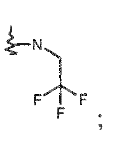
R⁴ is hydrogen;
R⁵ is hydrogen;
or R⁴ and R⁵ together with the carbon atom to which they are attached, form cyclopropyl;
M is carboxy.

8. A compound according to claim 1 or 2, wherein
R¹ is C₁₋₂alkoxycarbonyl or cyano;
R² is phenyl which is once or twice substituted by halogen;
R³ is 2-thiazolyl; or 2-pyridinyl, which is once substituted by halogen; or 2-imidazolyl, which is once substituted by C₁₋₆alkyl;
X is -NR⁷;
one of R⁴ and R⁵ is hydrogen, and the other one with the carbon atom to which it is attached and -NR⁷ together form a morpholinyl;
M is C₁₋₆alkoxycarbonyl, carboxy or hydroxy-C_{y}H_{2y}-;
y is 1-6.

9. A compound according to any one of claims 1, 2, 3 or 8, wherein
R¹ is methoxycarbonyl, ethoxycarbonyl or cyano;
R² is 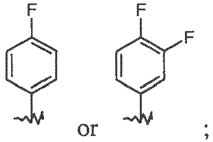
R³ is 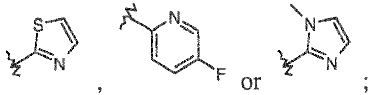
one of R⁴ and R⁵ is hydrogen, and the other one with the carbon atom to which it is attached and -NR⁷ form 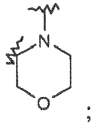
M is methoxycarbonyl, carboxy or hydroxymethyl-.

10. A compound according to claim 1 or 2, wherein
R¹ is C₁₋₂alkoxycarbonyl or cyano;
R² is phenyl which is once or twice substituted by halogen;
R³ is 2-thiazolyl, which is unsubstituted or once substituted by C₁₋₆alkyl or halogen; or 2-thienyl or 2-pyridinyl, which is once substituted by halogen; or 2-imidazolyl, which is once substituted by C₁₋₆alkyl; or 3-isoxazolyl, which is unsubstituted or once substituted by C₁₋₆alkyl;
X is -NR⁷;
one of R⁴ and R⁵ is hydrogen or C₁₋₆alkyl, and the other one with the carbon atom to which it is attached and -NR⁷ together form a pyrrolidinyl or piperidinyl, which is substituted by fluoro;
M is C₁₋₆alkoxycarbonyl, carboxy, diC₁₋₆alkylaminoC₂₋₆alkoxycarbonyl, aminocarbonyl, C₁₋₆alkylaminocarbonyl, diC₁₋₆alkylaminocarbonyl, C₁₋₆alkylsulfonylaminocarbonyl, 2-thiazolylaminocarbonyl, hydroxy-C_{y}H_{2y}-, 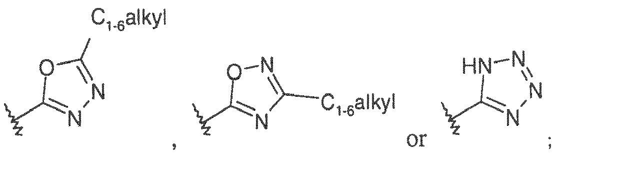
y is 1-6.

11. A compound according to any one of claims 1, 2, 3 or 10, wherein
R¹ is methoxycarbonyl, ethoxycarbonyl or cyano;
R² is 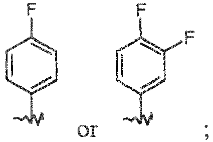
R³ is 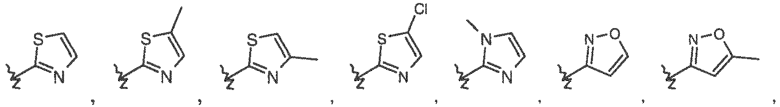 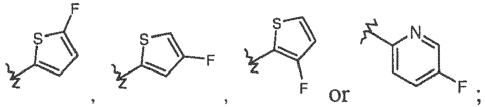
X is -NR⁷;
one of R⁴ and R⁵ is hydrogen or methyl, and the other one with the carbon atom to which it is attached and -NR⁷ form 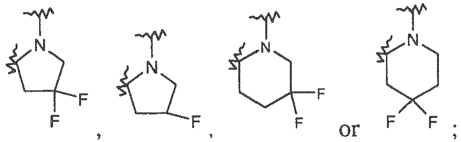
M is methoxycarbonyl, carboxy, dimethylaminoethoxycarbonyl, aminocarbonyl, dimethylaminocarbonyl, methylaminocarbonyl, methylsulfonylaminocarbonyl, 2-thiazolylaminocarbonyl, hydroxymethyl, hydroxypropyl,

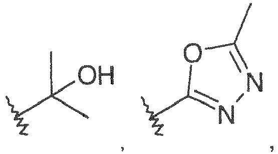

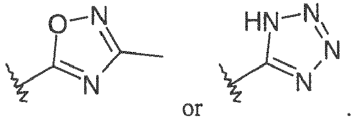

12. A compound according to claim 1 of formula (I') 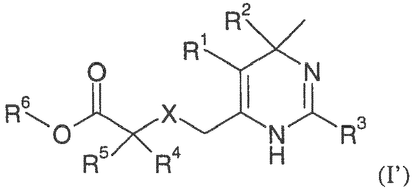 wherein,
R¹ is C₁₋₂alkoxycarbonyl or cyano;
R² is phenyl, which is substituted by halogen;
R³ is 2-thiazolyl which is unsubstituted or substituted by C₁₋₆alkyl or 2-pyridinyl, which is substituted by halogen;
X is oxygen or -NR⁷;
R⁴ and R⁵ are independently selected from hydrogen, C₁₋₆alkyl and trifluoroC₁₋₆alkyl; or
R⁴ and R⁵, together with the carbon atom to which they are attached, form a 3 to 7 membered cycloalkyl; or
when X is -NR⁷, one of R⁴ and R⁵ is hydrogen or C₁₋₆alkyl, and the other one with the carbon atom to which it is attached and -NR⁷ together form a morpholinyl; or pyrrolidinyl substituted by fluoro;
R⁶ is hydrogen or C₁₋₆alkyl;
R⁷ is C₁₋₆alkyl;
or pharmaceutically acceptable salts, or tautomerism isomers thereof.

13. A compound according to claim 12, wherein
R¹ is methoxycarbonyl or cyano;
R² is phenyl substituted once or twice by fluoro;
R³ is 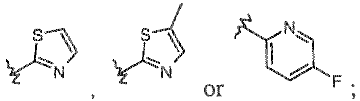
X is oxygen or -NR⁷;
R⁴ and R⁵ are independently selected from hydrogen, methyl and trifluoromethyl; or
R⁴ and R⁵ together with the carbon atom to which they are attached form cyclopropyl; or when X is -NR⁷, one of R⁴ and R⁵ is hydrogen or methyl, and the other one with the carbon atom to which it is attached and -NR⁷ form 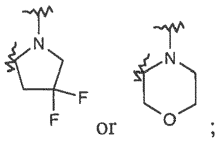
R⁶ is hydrogen or methyl;
R⁷ is methyl;
or pharmaceutically acceptable salts, or tautomerism isomers thereof.

14. A compound according to claim 12, wherein
R¹ is C₁₋₂alkoxycarbonyl or cyano;
R² is phenyl which is substituted by halogen;
R³ is 2-thiazolyl or 2-pyridinyl, which is substituted by halogen;
X is -NR⁷;
one of R⁴ and R⁵ is hydrogen or C₁₋₆alkyl, and the other one with the carbon atom to which it is attached and -NR⁷ together form a morpholinyl;
R⁶ is hydrogen or C₁₋₆alkyl.

15. A compound according to any one of claims 12, 13 or 14, wherein
R¹ is methoxycarbonyl or cyano;
R² is 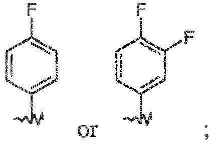
R³ is 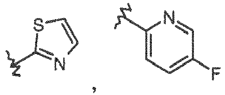
one of R⁴ and R⁵ is hydrogen or methyl, and the other one with the carbon atom to which it is attached and -NR⁷ form 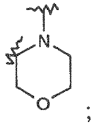
R⁶ is hydrogen or methyl.

16. A compound according to claim 12, wherein
R¹ is C₁₋₂alkoxycarbonyl or cyano;
R² is phenyl which is substituted by halogen;
R³ is 2-thiazolyl which is unsubstituted or substituted by C₁₋₆alkyl or 2-pyridinyl, which is substituted by halogen;
X is -NR⁷;
one of R⁴ and R⁵ is hydrogen or C₁₋₆alkyl, and the other one with the carbon atom to which it is attached and -NR⁷ together form a pyrrolidinyl which is substituted by fluoro;
R⁶ is hydrogen or C₁₋₆alkyl.

17. A compound according to any one of claims 12, 13 and 16, wherein
R¹ is methoxycarbonyl or cyano;
R² is 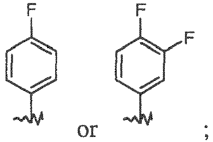
R³ is 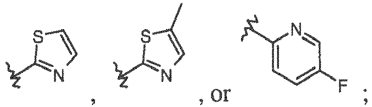
X is -NR⁷;
one of R⁴ and R⁵ is hydrogen or methyl, and the other one with the carbon atom to which it is attached and -NR⁷ form 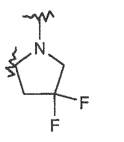
R⁶ is hydrogen or methyl.

18. A compound according to any one of claims 1 to 17, selected from 4-(4-fluoro-phenyl)-6-(1-methoxycarbonyl-1-methyl-ethoxymethyl)-4-methyl-2-thiazol-2-yl-1,4-dihydro-pyrimidine-5-carboxylic acid methyl ester; 6-(1-carboxy-2,2,2-trifluoro-ethoxymethyl)-4-(4-fluoro-phenyl)-4-methyl-2-thiazol-2-yl-1,4-dihydro-pyrimidine-5-carboxylic acid methyl ester; 6-{[(1-carboxy-cyclopropyl)-methyl-amino]-methyl}-4-(4-fluoro-phenyl)-4-methyl-2-thiazol-2-yl-1,4-dihydro-pyrimidine-5-carboxylic acid methyl ester; 4-[6-(4-fluoro-phenyl)-5-methoxycarbonyl-6-methyl-2-thiazol-2-yl-3,6-dihydro-pyrimidin-4-ylmethyl]-morpholine-3-carboxylic acid; 4-[6-(4-fluoro-phenyl)-5-methoxycarbonyl-6-methyl-2-thiazol-2-yl-3,6-dihydro-pyrimidin-4-ylmethyl]-morpholine-(*S*)-3-carboxylic acid methyl ester; 4-[6-(4-fluoro-phenyl)-5-methoxycarbonyl-6-methyl-2-thiazol-2-yl-3,6-dihydro-pyrimidin-4-ylmethyl]-morpholine-(*S*)-3-carboxylic acid; (*S*)-4-[6-(4-fluoro-phenyl)-5-methoxycarbonyl-6-methyl-2-thiazol-2-yl-3,6-dihydro-pyrimidin-4-ylmethyl]-morpholine-(*S*)-3-carboxylic acid; (*S*)-4-[6-(4-fluoro-phenyl)-5-methoxycarbonyl-6-methyl-2-thiazol-2-yl-3,6-dihydro-pyrimidin-4-ylmethyl]-morpholine-(*R*)-3-carboxylic acid; 4-[6-(4-fluoro-phenyl)-2-(5-fluoro-pyridin-2-yl)-5-methoxycarbonyl-6-methyl-3,6-dihydro-pyrimidin-4-ylmethyl]-morpholine-(*R*)-3-carboxylic acid; 4-[6-(4-fluoro-phenyl)-2-(5-fluoro-pyridin-2-yl)-5-methoxycarbonyl-6-methyl-3,6-dihydro-pyrimidin-4-ylmethyl]-morpholine-(*S*)-3-carboxylic acid; 6-(2-(*S*)-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; 6-(2-(*R*)-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid methyl ester; 4-[(*S*)-6-(3,4-difluoro-phenyl)-5-methoxycarbonyl-6-methyl-2-thiazol-2-yl-3,6-dihydro-pyrimidin-4-ylmethyl]-morpholine-3-carboxylic acid methyl ester; 4-[6-(3,4-difluoro-phenyl)-5-methoxycarbonyl-6-methyl-2-thiazol-2-yl-3,6-dihydro-pyrimidin-4-ylmethyl]-morpholine-3-carboxylic acid; 6-(4,4-difluoro-2-methoxycarbonyl-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester; 6-(2-carboxy-4,4-difluoro-pyrrolidin-1-ylmethyl)-4-(4-fluoro-phenyl)-2-(5-fluoro-pyridin-2-yl)-4-methyl-1,4-dihydro-pyrimidine-5-carboxylic acid methyl ester; 6-((*S*)-2-carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester; 6-(2-carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-1-[(*S*)-5-cyano-6-(4-fluoro-phenyl)-6-methyl-2-thiazol-2-yl-3,6-dihydro-pyrimidin-4-ylmethyl]-4,4-difluoro-pyrrolidine-2-carboxylic acid; (*S*)-4-[5-cyano-6-(3,4-difluoro-phenyl)-6-methyl-2-thiazol-2-yl-3,6-dihydro-pyrimidin-4-ylmethyl]-morpholine-3-carboxylic acid; (*S*)-1-[(*S*)-5-cyano-6-(3,4-difluoro-phenyl)-6-methyl-2-thiazol-2-yl-3,6-dihydro-pyrimidin-4-ylmethyl]-4,4-difluoro-pyrrolidine-2-carboxylic acid; (*S*)-6-((*S*)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester; (*S*)-6-(2-carboxy-5,5-difluoro-piperidin-1-ylmethyl)-4-(4-fluoro-phenyl)-4-methyl-2-thiazol-2-yl-1,4-dihydro-pyrimidine-5-carboxylic acid methyl ester; (*S*)-6-(2-carboxy-4,4-difluoro-piperidin-1-ylmethyl)4-(4-fluoro-phenyl)-4-methyl-2-thiazol-2-yl-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-4-methyl-2-(1-methyl-1*H*-imidazol-2-yl)-1,4-dihydro-pyrimidine-5-carboxylic acid methyl ester; (*R*)-6-((*S*)-2-carboxy-4,4-difluoro-pyrrolidin-1-ylmethyl)-4-(3,4-difluoro-phenyl)-4-methyl-2-thiazol-2-yl-1,4-dihydro-pyrimidine-5-carboxylic acid ethyl ester; (*S*)-4-[6-(3,4-difluoro-phenyl)-5-ethoxycarbonyl-6-methyl-2-thiazol-2-yl-3,6-dihydro-pyrimidin-4-ylmethyl] -morpholine-3-carboxylic acid; (*S*)-4-[(*S*)-6-(4-fluoro-phenyl)-5-methoxycarbonyl-6-methyl-2-(1-methyl-1*H*-imidazol-2-yl)-3,6-dihydro-pyrimidin-4-ylmethyl]-morpholine-3-carboxylic acid; (*R*)-6-((*S*)-2-carboxy-4,4-difluoro-pyrrolidin-1-ylmethyl)-4-(4-fluoro-phenyl)-4-methyl-2-(4-methyl-thiazol-2-yl)-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-4-methyl-2-(4-methyl-thiazol-2-yl)-1,4-dihydro-pyrimidine-5-carboxylic acid methyl ester; (*S*)-6-((*S*)-2-carboxy-4,4-difluoro-pyrrolidin-1-ylmethyl)-2-(5-chloro-thiazol-2-yl)-4-(4-fluoro-phenyl)-4-methyl-1,4-dihydro-pyrimidine-5-carboxylic acid methyl ester; (*S*)-6-((2*S*,4*R*)-2-carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester; 6-((*S*)-2-carboxy-4,4-difluoro-pyrrolidin-1-ylmethyl)-4-(4-fluoro-phenyl)-2-isoxazol-3-yl-4-methyl-1,4-dihydro-pyrimidine-5-carboxylic acid methyl ester; (*R*)-6-((*S*)-2-carboxy-4,4-difluoro-2-methyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-4-methyl-2-(5-methyl-thiazol-2-yl)-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-2-(5-fluoro-thiophen-2-yl)-4-methyl-1,4-dihydro-pyrimidine-5-carboxylic acid methyl ester; (*S*)-6-((2*S*,4*S*)-2-carboxy-4-fluoro-pyrrolidin-1-ylmethyl)-4-(4-fluoro-phenyl)-4-methyl-2-thiazol-2-yl-1,4-dihydro-pyrimidine-5-carboxylic acid methyl ester; 6-((*S*)-2-carboxy-4,4-difluoro-pyrrolidin-1-ylmethyl)-4-(4-fluoro-phenyl)-4-methyl-2-(5-methyl-isoxazol-3-yl)-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-2-(3-fluoro-thiophen-2-yl)-4-methyl-1,4-dihydro-pyrimidine-5-carboxylic acid methyl ester; (*R*)-6-((*S*)-2-carboxy-4,4-difluoro-pyrrolidin-1-ylmethyl)-4-(4-fluoro-phenyl)-2-(3-fluoro-thiophen-2-yl)-4-methyl-1,4-dihydro-pyrimidine-5-carboxylic acid methyl ester; (*S*)-6-((*S*)-2-carboxy-4,4-difluoro-pyrrolidin-1-ylmethyl)-4-(4-fluoro-phenyl)-2-(4-fluoro-thiophen-2-yl)-4-methyl-1,4-dihydro-pyrimidine-5-carboxylic acid methyl ester; (*S*)-6-{[carboxymethyl-(2,2,2-trifluoro-ethyl)-amino]-methyl}-4-(4-fluoro-phenyl)-4-methyl-2-thiazol-2-yl-1,4-dihydro-pyrimidine-5-carboxylic acid methyl ester; (*S*)-6-((*S*)-4,4-difluoro-2-methoxycarbonyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-[(*S*)-2-(2-dimethylamino-ethoxycarbonyl)-4,4-difluoro-pyrrolidin-1-ylmethyl]-4-(4-fluoro-phenyl)-4-methyl-2-thiazol-2-yl-1,4-dihydro-pyrimidine-5-carboxylic acid methyl ester; (*S*)-6-(2-carbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-2-carbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-2-dimethylcarbamoyl-4,4-difluoro-pyrrolidin-1-ylmethyl)-4-(4-fluoro-phenyl)-4-methyl-2-thiazol-2-yl-1,4-dihydro-pyrimidine-5-carboxylic acid methyl ester; 6-((*S*)-4,4-difluoro-2-methylcarbamoyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-((*S*)-4,4-difluoro-2-methanesulfonylaminocarbonyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-[(*S*)-4,4-difluoro-2-(thiazol-2-ylcarbamoyl)-pyrrolidin-1-ylmethyl]-4-(4-fluoro-phenyl)-4-methyl-2-thiazol-2-yl-1,4-dihydro-pyrimidine-5-carboxylic acid methyl ester; 4-(4-fluoro-phenyl)-6-((*R*)-3-hydroxymethyl-morpholin-4-ylmethyl)-4-methyl-2-thiazol-2-yl-1,4-dihydro-pyrimidine-5-carboxylic acid methyl ester; (*S*)-6-[(*S*)-4,4-difluoro-2-(1-hydroxy-1-methyl-ethyl)-pyrrolidin-1-ylmethyl]-4-(4-fluoro-phenyl)-4-methyl-2-thiazol-2-yl-1,4-dihydro-pyrimidine-5-carboxylic acid methyl ester; (*S*)-6-((*S*)-4,4-difluoro-2-hydroxymethyl-pyrrolidin-1-ylmethyl)-4-(4-fluoro-phenyl)-4-methyl-2-thiazol-2-yl-1,4-dihydro-pyrimidine-5-carboxylic acid methyl ester; (*S*)-6-[4,4-difluoro-2-(3-hydroxy-propyl)-pyrrolidin-1-ylmethyl]-4-(4-fluoro-phenyl)-4-methyl-2-thiazol-2-yl-1,4-dihydro-pyrimidine-5-carboxylic acid methyl ester; (*S*)-6-[(*S*)-4,4-difluoro-2-(5-methyl-[1,3,4]oxadiazol-2-yl)-pyrrolidin-1-ylmethyl]-4-(4-fluoro-phenyl)-4-methyl-2-thiazol-2-yl-1,4-dihydro-pyrimidine-5-carboxylic acid methyl ester; (*S*)-6-[(*S*)-4,4-difluoro-2-(1*H*-tetrazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester; and (*S*)-6-[(*S*)-4,4-difluoro-2-(3-methyl-[1,2,4]oxadiazol-5-yl)-pyrrolidin-1-ylmethyl]-4-(4-fluoro-phenyl)-4-methyl-2-thiazol-2-yl-1,4-dihydro-pyrimidine-5-carboxylic acid methyl ester.

19. A process for the preparation of a compound according to any one of claims 1 to 18 comprising the reaction of
(a) a compound of formula (A) 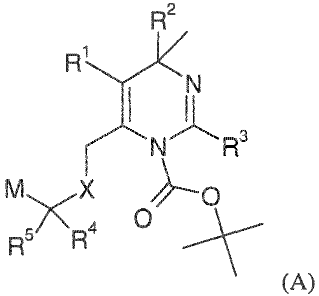 in the presence of an acid;
wherein R¹ to R⁵, M and X are defined as in any one of claims 1 to 17.

20. A compound according to any one of claims 1 to 18 for use as therapeutically active substance.

21. A pharmaceutical composition comprising a compound in accordance with any one of claims 1 to 18 and a therapeutically inert carrier.

22. The use of a compound according to any one of claims 1 to 18 for the preparation of a medicament for the treatment or prophylaxis of hepatitis B virus infection.

23. A compound according to any one of claims 1 to 18 for use in the treatment or prophylaxis of hepatitis B virus infection.

## Patentansprüche

1. Verbindungen der Formel (I) 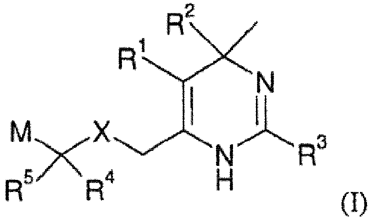 wobei
R¹ für C₁₋₂-Alkoxycarbonyl oder Cyano steht;
R² für Phenyl steht, welches mit Halogen substituiert ist;
R³ für Thiazolyl, Thienyl, Imidazolyl, Isoxazolyl oder Pyridinyl steht, welches unsubstituiert oder mit Halogen oder C₁₋₆-Alkyl substituiert ist;
X für Sauerstoff oder -NR⁷ steht;
R⁴ und R⁵ unabhängig ausgewählt sind aus Wasserstoff, C₁₋₆-Alkyl und Tufluor-C₁₋₆-alkyl; oder
R⁴ und R⁵ zusammen mit dem Kohlenstoffatom, an welches sie gebunden sind, ein 3- bis 7-gliedriges Cycloalkyl bilden; oder
wenn X gleich -NR⁷ ist, einer der Reste R⁴ und R⁵ für Wasserstoff oder C₁₋₆-Alkyl steht und der andere zusammen mit dem Kohlenstoffatom, an welches er gebunden ist, und -NR⁷ einen Pyrrolidinyl-, Morpholinyl- oder Piperidinylring bildet, wobei der Ring unsubstituiert oder mit Fluor substituiert ist;
M für C₁₋₆-Alkoxycarbonyl, Carboxy, Di-C₁₋₆-alkylamino-C₂₋₆-alkoxycarbonyl, Aminocarbonyl, C₁₋₆-Alkylaminocarbonyl, Di-C₁₋₆-alkylaminocarbonyl, C₁₋₆-Alkylsulfonylaminocarbonyl, 2-Thiazolylaminocarbonyl, Hydroxy-CyH_{2y-}, 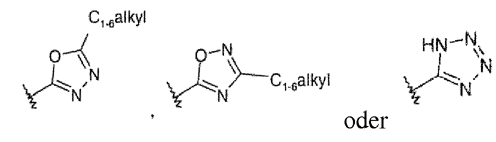 steht;
R⁷ für C₁₋₆-Alkyl oder Trifluor-C₁₋₆-alkyl steht;
y gleich 1-6 ist;
oder pharmazeutisch verträgliche Salze oder Tautomerie-Isomere davon.

2. Eine Verbindung gemäß Anspruch 1, wobei
R¹ für C₁₋₂-Alkoxycarbonyl oder Cyano steht;
R² für Phenyl steht, welches einmal oder zweimal mit Halogen substituiert ist;
R³ für 2-Thiazolyl steht, welches unsubstituiert oder einmal mit C₁₋₆-Alkyl oder Halogen substituiert ist; oder 2-Thienyl oder 2-Pyridinyl steht, welches einmal mit Halogen substituiert ist; oder 2-Imidazolyl steht, welches einmal mit C₁₋₆-Alkyl substituiert ist; oder 3-Isoxazolyl, welches unsubstituiert oder einmal mit C₁₋₆-Alkyl substituiert ist;
X für Sauerstoff oder -NR⁷ steht;
R⁴ und R⁵ unabhängig ausgewählt sind aus Wasserstoff, C₁₋₆-Alkyl und Tufluor-C₁₋₆-alkyl; oder
R⁴ und R⁵, zusammen mit dem Kohlenstoffatom, an welches sie gebunden sind, ein 3-bis 7-gliedriges Cycloalkyl bilden; oder
wenn X gleich -NR⁷ ist, einer der Reste R⁴ und R⁵ für Wasserstoff oder C₁₋₆-Alkyl steht und der andere zusammen mit dem Kohlenstoffatom, an welches er gebunden ist, und -NR⁷ Morpholinyl bildet; oder Pyrrolidinyl oder Piperidinyl, welches mit Fluor substituiert ist;
M für C₁₋₆-Alkoxycarbonyl, Carboxy, Di-C₁₋₆-alkylamino-C₂₋₆-alkoxycarbonyl, Aminocarbonyl, C₁₋₆-Alkylaminocarbonyl, Di-C₁₋₆-alkylaminocarbonyl, C₁₋₆-Alkylsulfonylaminocarbonyl, 2-Thiazolylaminocarbonyl, Hydroxy-CyH_{2y}-, 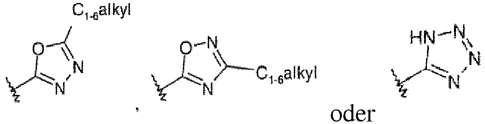 steht;
R⁷ für C₁₋₆-Alkyl oder Trifluor-C₁₋₆-alkyl steht;
y gleich 1-6 ist;
oder pharmazeutisch verträgliche Salze oder Tautomerie-Isomere davon.

3. Eine Verbindung gemäß Anspruch 1 oder 2, wobei
R¹ für Methoxycarbonyl, Ethoxycarbonyl oder Cyano steht;
R² Phenyl darstellt, welches einmal oder zweimal mit Fluor substituiert ist;
R³ für 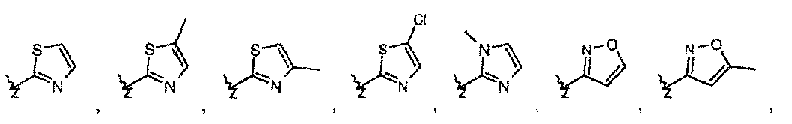 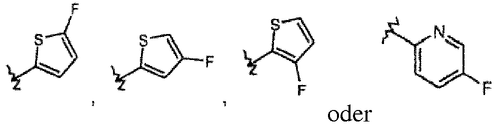 steht;
X für Sauerstoff oder -NR⁷ steht;
R⁴ und R⁵ unabhängig ausgewählt sind aus Wasserstoff, Methyl und Trifluormethyl; oder
R⁴ und R⁵ zusammen mit dem Kohlenstoffatom, an welches sie gebunden sind, Cyclopropyl bilden; oder
wenn X gleich -NR⁷ ist, einer der Reste R⁴ und R⁵ für Wasserstoff oder Methyl steht, und der andere zusammen mit dem Kohlenstoffatom, an welches er gebunden ist, und-NR⁷ 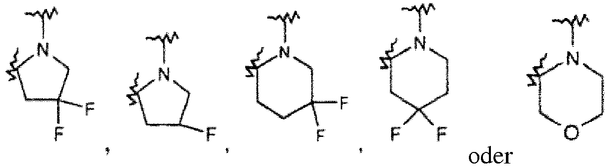 bildet;
M für Methoxycarbonyl, Carboxy, Dimethylaminoethoxycarbonyl, Aminocarbonyl, Dimethylaminocarbonyl, Methylaminocarbonyl, Methylsulfonylaminocarbonyl, 2-Thiazolylaminocarbonyl, Hydroxymethyl, Hydroxypropyl, 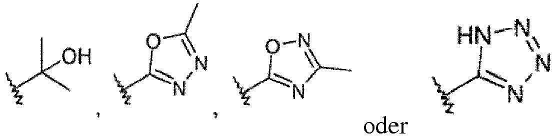 steht;
R⁷ für Methyl oder Trifluorethyl steht;
oder pharmazeutisch verträgliche Salze oder Tautomerie-Isomere davon.

4. Eine Verbindung gemäß Anspruch 1 oder 2, wobei
R¹ für C₁₋₂-Alkoxycarbonyl steht;
R² für Phenyl steht, welches einmal mit Halogen substituiert ist;
R³ für 2-Thiazolyl steht;
X für Sauerstoff steht;
R⁴ und R⁵ unabhängig ausgewählt sind aus Wasserstoff, C₁₋₆-Alkyl und Trifluor-C₁₋₆-alkyl;
M für C₁₋₆-Alkoxycarbonyl oder Carboxy steht.

5. Eine Verbindung gemäß einem der Ansprüche 1 bis 4, wobei R¹ für Methoxycarbonyl steht;
R₂ für 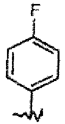 steht;
R³ für 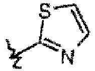 steht;
X für Sauerstoff steht;
R⁴ und R⁵ unabhängig ausgewählt sind aus Wasserstoff, Methyl und Trifluormethyl;
M für Methoxycarbonyl oder Carboxy steht.

6. Eine Verbindung gemäß Anspruch 1 oder 2, wobei
R¹ für C₁₋₂-Alkoxycarbonyl steht;
R² für Phenyl steht, welches einmal mit Halogen substituiert ist;
R³ für 2-Thiazolyl steht;
X für -N-C₁₋₆-Alkyl oder -N-Trifluor-C₁₋₆-alkyl steht;
R⁴ für Wasserstoff steht;
R⁵ für Wasserstoff steht;
oder R⁴ und R⁵ zusammen mit dem Kohlenstoffatom, an welches sie gebunden sind, ein 3- bis 7-gliedriges Cycloalkyl bilden;
M für Carboxy steht.

7. Eine Verbindung gemäß einem der Ansprüche 1, 2, 3 oder 6, wobei
R¹ für Methoxycarbonyl steht;
R² für 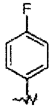 steht;
R³ für 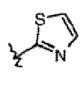 steht;
X für -NCH₃ oder 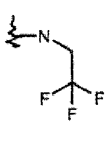 steht;
R⁴ für Wasserstoff steht;
R⁵ für Wasserstoff steht;
oder R⁴ und R⁵ zusammen mit dem Kohlenstoffatom, an welches sie gebunden sind, Cyclopropyl bilden;
M für Carboxy steht.

8. Eine Verbindung gemäß Anspruch 1 oder 2, wobei
R¹ für C₁₋₂-Alkoxycarbonyl oder Cyano steht;
R² für Phenyl steht, welches einmal oder zweimal mit Halogen substituiert ist;
R³ für 2-Thiazolyl steht; oder 2-Pyridinyl, welches einmal mit Halogen substituiert ist; oder 2-Imidazolyl, welches einmal mit C₁₋₆-Alkyl substituiert ist;
X für -NR⁷ steht;
einer der Reste R⁴ und R⁵ für Wasserstoff steht und der andere zusammen mit dem Kohlenstoffatom, an welches er gebunden ist, und -NR⁷ Morpholinyl bildet;
M für C₁₋₆-Alkoxycarbonyl, Carboxy oder Hydroxy-C_{y}H_{2y}- steht;
Y gleich 1-6 ist.

9. Eine Verbindung gemäß einem der Ansprüche 1, 2, 3 oder 8, wobei
R¹ für Methoxycarbonyl, Ethoxycarbonyl oder Cyano steht;
R² für 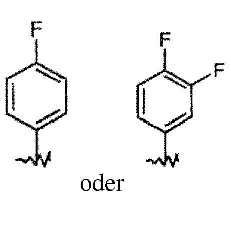 steht;
R³ für 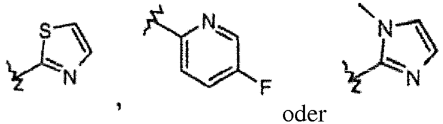 steht;
einer der Reste R⁴ und R⁵ für Wasserstoff steht und der andere zusammen mit dem Kohlenstoffatom, an welches er gebunden ist, und -NR⁷ 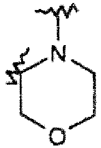 bildet;
M für Methoxycarbonyl, Carboxy oder Hydroxymethyl steht.

10. Eine Verbindung gemäß Anspruch 1 oder 2, wobei
R¹ für C₁₋₂-Alkoxycarbonyl oder Cyano steht;
R² für Phenyl steht, welches einmal oder zweimal mit Halogen substituiert ist;
R³ für Thiazolyl steht, welches unsubstituiert oder einmal mit C₁₋₆-Alkyl oder Halogen substituiert ist; oder 2-Thienyl oder 2-Pyridinyl, welches einmal mit Halogen substituiert ist; oder 2-Imidazolyl, welches einmal mit C₁₋₆-Alkyl substituiert ist; oder 3-Isoxazolyl, welches unsubstituiert oder einmal mit C₁₋₆-Alkyl substituiert ist;
X für -NR⁷ steht;
einer der Reste R⁴ und R⁵ für Wasserstoff oder C₁₋₆-Alkyl steht und der andere zusammen mit dem Kohlenstoffatom, an welches er gebunden ist, und -NR⁷ Pyrrolidinyl oder Piperidinyl bildet, welches mit Fluor substituiert ist;
M für C₁₋₆-Alkoxycarbonyl, Carboxy, Di-C₁₋₆-alkylamino-C₂₋₆-alkoxycarbonyl, Aminocarbonyl, C₁₋₆-Alkylaminocarbonyl, Di-C₁₋₆-alkylaminocarbonyl, C₁₋₆-Alkylsulfonylaminocarbonyl, 2-Thiazolylaminocarbonyl, Hydroxy-C_{y}H_{2y}-, 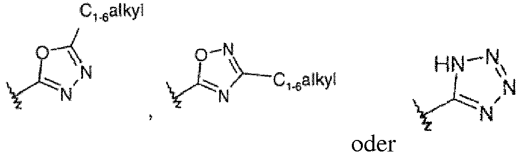 bildet;
y gleich 1-6 ist.

11. Eine Verbindung gemäß einem der Ansprüche 1, 2, 3 oder 10, wobei
R¹ für Methoxycarbonyl, Ethoxycarbonyl oder Cyano steht;
R² für 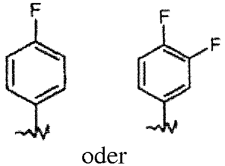 steht;
R³ für 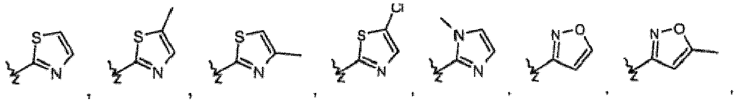 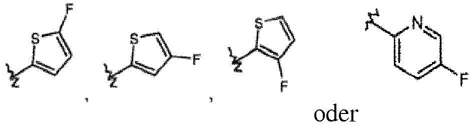 steht;
X für -NR⁷ steht;
einer der Reste R⁴ und R⁵ für Wasserstoff oder Methyl steht und der andere mit dem Kohlenstoffatom, an welches er gebunden ist, und -NR⁷ 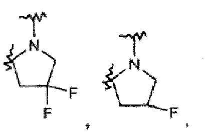 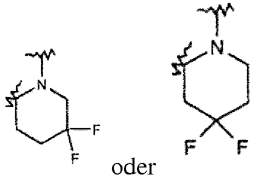 bildet;
M für Methoxycarbonyl, Carboxy, Dimethylaminoethoxycarbonyl, Aminocarbonyl, Dimethylaminocarbonyl, Methylaminocarbonyl, Methylsulfonylaminocarbonyl,
2-Thiazolylaminocarbonyl, Hydroxymethyl, Hydroxypropyl, 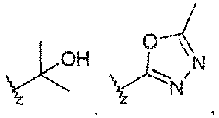 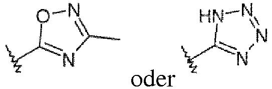 steht.

12. Eine Verbindung gemäß Anspruch 1 der Formel (I') 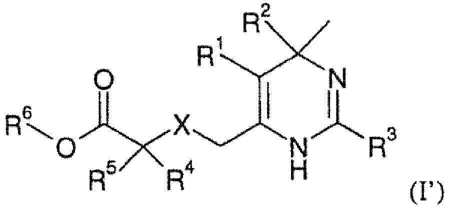 wobei
R¹ für C₁₋₂-Alkoxycarbonyl oder Cyano steht;
R² für Phenyl steht, welches mit Halogen substituiert ist;
R³ für 2-Thiazolyl steht, welches unsubstituiert oder mit C₁₋₆-Alkyl substituiert ist, oder 2-Pyridinyl, welches mit Halogen substituiert ist;
X für Sauerstoff oder -NR⁷ steht;
R⁴ und R⁵ unabhängig ausgewählt sind aus Wasserstoff, C₁₋₆-Alkyl und Tufluor-C₁₋₆-alkyl; oder
R⁴ und R⁵ zusammen mit dem Kohlenstoffatom, an welches sie gebunden sind, 3- bis 7-gliedrige Cycloalkyl bilden; oder
falls X gleich -NR⁷ ist, einer der Reste R⁴ und R⁵ für Wasserstoff oder C₁₋₆-Alkyl steht und der andere zusammen mit dem Kohlenstoffatom, an welches er gebunden ist, und -NR⁷ Morpholinyl bildet; oder Pyrrolidinyl, welches mit Fluor substituiert ist;
R⁶ für Wasserstoff oder C₁₋₆-Alkyl steht;
R⁷ für C₁₋₆-Alkyl steht;
oder pharmazeutisch verträgliche Salze oder Tautomerie-Isomere davon.

13. Eine Verbindung gemäß Anspruch 12, wobei
R¹ für Methoxycarbonyl oder Cyano steht;
R² für Phenyl steht, welches einmal oder zweimal mit Fluor substituiert ist;
R³ für 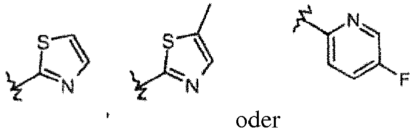 steht;
X für Sauerstoff oder -NR⁷ steht;
R⁴ und R⁵ unabhängig ausgewählt sind aus Wasserstoff, Methyl und Trifluormethyl; oder R⁴ und R⁵ zusammen mit dem Kohlenstoffatom, an welches sie gebunden sind, Cyclopropyl bilden; oder
wenn X gleich -NR⁷ ist, einer der Reste R⁴ und R⁵ für Wasserstoff oder Methyl steht und der andere mit dem Kohlenstoffatom, an welches er gebunden ist, und -NR⁷ 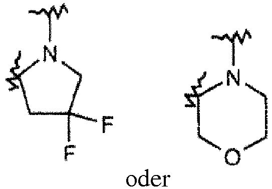 bildet;
R⁶ für Wasserstoff oder Methyl steht;
R⁷ für Methyl steht;
oder pharmazeutisch verträgliche Salze oder Tautomerie-Isomere davon.

14. Eine Verbindung gemäß Anspruch 12, wobei
R¹ für C₁₋₂-Alkoxycarbonyl oder Cyano steht;
R² für Phenyl steht, welches mit Halogen substituiert ist;
R³ für 2-Thiazolyl oder 2-Pyridinyl steht, welches mit Halogen substituiert ist;
X für -NR⁷ steht;
einer der Reste R⁴ und R⁵ für Wasserstoff oder C₁₋₆-Alkyl steht und der andere zusammen mit dem Kohlenstoffatom, an welches er gebunden ist, und -NM⁷ Morpholinyl bildet;
R⁶ für Wasserstoff oder C₁₋₆-Alkyl steht.

15. Eine Verbindung gemäß einem der Ansprüche 12, 13 oder 14, wobei
R¹ für Methoxycarbonyl oder Cyano steht;
R² für 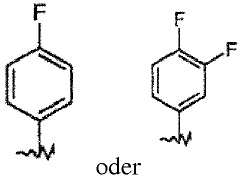 steht;
R³ für 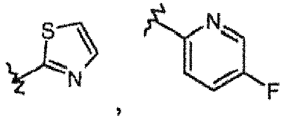 steht;
einer der Reste R⁴ und R⁵ für Wasserstoff oder Methyl steht und der andere mit dem Kohlenstoffatom, an welches er gebunden ist, und -NR⁷ 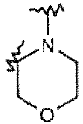 bildet;
R⁶ für Wasserstoff oder Methyl steht;

16. Eine Verbindung gemäß Anspruch 12, wobei
R¹ für C₁₋₂-Alkoxycarbonyl oder Cyano steht;
R² für Phenyl steht, welches mit Halogen substituiert ist;
R³ für 2-Thiazolyl steht, welches unsubstituiert oder mit C₁₋₆-Alkyl substituiert ist, oder 2-Pyridinyl, welches mit Halogen substituiert ist;
X für -NR⁷ steht;
einer der Reste R⁴ und R⁵ für Wasserstoff oder C₁₋₆-Alkyl steht und der andere zusammen mit dem Kohlenstoffatom, an welches er gebunden ist, und -NR⁷ Pyrrolidinyl bildet, welches mit Fluor substituiert ist;
R⁶ für Wasserstoff oder C₁₋₆-Alkyl steht.

17. Eine Verbindung gemäß einem der Ansprüche 12, 13 und 16, wobei
R¹ für Methoxycarbonyl oder Cyano steht;
R² für 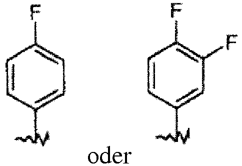 steht;
R³ für 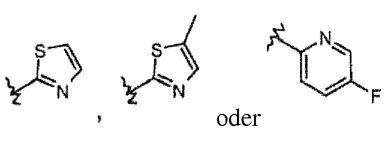 steht;
X für -NR⁷ steht;
einer der Reste R⁴ und R⁵ für Wasserstoff oder Methyl steht und der andere zusammen mit dem Kohlenstoffatom, an welches er gebunden ist, und -NR⁷ 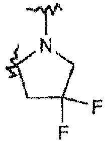 bildet;
R für Wasserstoff oder Methyl steht.

18. Eine Verbindung gemäß einem der Ansprüche 1 bis 17, ausgewählt aus 4-(4-Fluor-phenyl)-6-(1-methoxycarbonyl-1-methyl-ethoxymethyl)-4-methyl-2-thiazol-2-yl-1,4-dihydro-pyrimidin-5-carbonsäure-methylester; 6-(1-Carboxy-2,2,2-trifluor-ethoxymethyl)-4-(4-fluor-phenyl)-4-methyl-2-thiazol-2-yl-1,4-dihydro-pyrimidin-5-carbonsäure-methylester; 6-{[(1-Carboxy-cyclopropyl)-methyl-amino]-methyl}-4-(4-fluor-phenyl)-4-methyl-2-thiazol-2-yl-1,4-dihydro-pyrimidin-5-carbonsäure-methylester; 4-[6-(4-Fluor-phenyl)-5-methoxycarbonyl-6-methyl-2-thiazol-2-yl-3,6-dihydro-pyrimidin-4-ylmethyl]-morpholin-3-carbonsäure; 4-[6-(4-Fluor-phenyl)-5-methoxycarbonyl-6-methyl-2-thiazol-2-yl-3,6-dihydro-pyrimidin-4-ylmethyl]-morpholin-(S)-3-carbonsäure-methylester; 4-[6-(4-Fluor-phenyl)-5-methoxycarbonyl-6-methyl-2-thiazol-2-yl-3,6-dihydro-pyrimidin-4-ylmethyl]-morpholin-(S)-3-carbonsäure; (S)-4-[6-(4-Fluor-phenyl)-5-methoxycarbonyl-6-methyl-2-thiazol-2-yl-3,6-dihydro-pyrimidin-4-ylmethyl]-morpholin-(S)-3-carbonsäure; (S)-4-[6-(4-Fluor-phenyl)-5-methoxycarbonyl-6-methyl-2-thiazol-2-yl-3,6-dihydro-pyrimidin-4-ylmethyl]-morpholin-(R)-3-carbonsäure; 4-[6-(4-Fluor-phenyl)-2-(5-fluor-pyridin-2-yl)-5-methoxycarbonyl-6-methyl-3,6-dihydro-pyrimidin-4-ylmethyl]-morpholin-(R)-3-carbonsäure; 4-[6-(4-Fluor-phenyl)-2-(5-fluor-pyridin-2-yl)-5-methoxycarbonyl-6-methyl-3,6-dihydro-pyrimidin-4-ylmethyl]-morpholin-(S)-3-carbonsäure; 6-(2-(S)-Carboxy-4,4-difluor-pyrrolidin-1-ylmethyl)-4-(4-fluor-phenyl)-4-methyl-2-thiazol-2-yl-1,4-dihydro-pyrimidin-5-carbonsäure-methylester; 6-(2-(R)-Carboxy-4,4-difluor-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-2-Carboxy-4,4-difluor-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-2-Carboxy-4,4-difluor-pyrrolidin-1-ylmethyl)-4-(3,4-difluor-phenyl)-4-methyl-2-thiazol-2-yl-1,4-dihydro-pyrimidin-5-carbonsäure-methylester; 4-[(S)-6-(3,4-Difluor-phenyl)-5-methoxycarbonyl-6-methyl-2-thiazol-2-yl-3,6-dihydro-pyrimidin-4-ylmethyl]-morpholin-3-carbonsäure-methylester; 4-[6-(3,4-Difluor-phenyl)-5-methoxycarbonyl-6-methyl-2-thiazol-2-yl-3,6-dihydro-pyrimidin-4-ylmethyl]-morpholin-3-carbonsäure; 6-(4,4-Difluor-2-methoxycarbonyl-pyrrolidin-1-ylmethyl)-4-(4-fluor-phenyl)-2-(5-fluor-pyridin-2-yl)-4-methyl-1,4-dihydro-pyrimidin-5-carbonsäure-methylester; 6-(2-Carboxy-4,4-difluor-pyrrolidin-1-ylmethyl)-4-(4-fluor-phenyl)-2-(5-fluor-pyridin-2-yl)-4-methyl-1,4-dihydro-pyrimidin-5-carbonsäure-methylester; 6-((S)-2-Carboxy-4,4-difluor-pyrrolidin-1-ylmethyl)-4-(4-fluor-phenyl)-4-methyl-2-(5-methyl-thiazol-2-yl)-1,4-dihydro-pyrimidin-5-carbonsäure-methylester; 6-(2-Carboxy-4,4-difluor-2-methyl-pyrrolidin-1-ylmethyl)-4-(4-fluor-phenyl)-4-methyl-2-thiazol-2-yl-1,4-dihydro-pyrimidin-5-carbonsäure-methylester; (S)-1-[(S)-5-Cyano-6-(4-fluor-phenyl)-6-methyl-2-thiazol-2-yl-3,6-dihydro-pyrimidin-4-ylmethyl]-4,4-difluor-pyrrolidin-2-carbonsäure; (S)-4-[5-Cyano-6-(3,4-difluor-phenyl)-6-methyl-2-thiazol-2-yl-3,6-dihydro-pyrimidin-4-ylmethyl]-morpholin-3-carbonsäure; (S)-1-[(S)-5-Cyano-6-(3,4-difluor-phenyl)-6-methyl-2-thiazol-2-yl-3,6-dihydro-pyrimidin-4-ylmethyl]-4,4-difluor-pyrrolidin-2-carbonsäure; (S)-6-((S)-2-Carboxy-4,4-difluor-pyrrolidin-1-ylmethyl-4-(3,4-difluor-phenyl)-4-methyl-2-thiazol-2-yl-1,4-dihydro-pyrimidin-5-carbonsäure-ethylester; (S)-6-(2-Carboxy-5,5-difluor-piperidin-1-ylmethyl)-4-(4-fluorphenyl)-4-methyl-2-thiazol-2-yl-1,4-dihydro-pyrimidin-5-carbonsäure-methylester; (S)-6-(2-Carboxy-4,4-difluor-piperidin-1-ylmethyl)4-(4-fluor-phenyl)-4-methyl-2-thiazol-2-yl-1,4-dihydro-pyrimidin-5-carbonsäure-methylester; (S)-6-((S)-2-Carboxy-4,4-difluor-pyrrolidin-1-ylmethyl)-4-(4-fluor-phenyl)-4-methyl-2-(1-methyl-1H-imidazol-2-yl)-1,4-dihydro-pyrimidin-5-carbonsäure-methylester; (R)-6-((S)-2-Carboxy-4,4-difluor-pyrrolidin-1-ylmethyl)-4-(3,4-difluor-phenyl)-4-methyl-2-thiazol-2-yl-1,4-dihydro-pyrimidin-5-carbonsäure-ethylester; (S)-4-[6-(3,4-Difluor-phenyl)-5-ethoxycarbonyl-6-methyl-2-thiazol-2-yl-3,6-dihydro-pyrimidin-4-ylmethyl]-morpholin-3-carbonsäure; (S)-4-[(S)-6-(4-Fluor-phenyl)-5-methoxycarbonyl-6-methyl-2-(1-methyl-1H-imidazol-2-yl)-3,6-dihydro-pyrimidin-4-ylmethyl]-morpholin-3-carbonsäure; (R)-6-((S)-2-Carboxy-4,4-difluor-pyrrolidin-1-ylmethyl)-4-(4-fluor-phenyl)-4-methyl-2-(4-methyl-thiazol-2-yl)-1,4-dihydro-pyrimidin-5-carbonsäure-methylester; (S)-6-((S)-2-Carboxy-4,4-difluor-pyrrolidin-1-ylmethyl)-4-(4-fluor-phenyl)-4-methyl-2-(4-methyl-thiazol-2-yl)-1,4-dihydro-pyrimidin-5-carbonsäure-methylester; (S)-6-((S)-2-Carboxy-4,4-difluor-pyrrolidin-1-ylmethyl)-2-(5-chlor-thiazol-2-yl)-4-(4-fluor-phenyl)-4-methyl-1,4-dihydro-pyrimidin-5-carbonsäure-methylester; (S)-6-((2S,4R)-2-Carboxy-4-fluor-pyrrolidin-1-ylmethyl)-4-(4-fluor-phenyl)-4-methyl-2-thiazol-2-yl-1,4-dihydro-pyrimidin-5-carbonsäure-methylester; 6-((S)-2-Carboxy-4,4-difluor-pyrrolidin-1-ylmethyl)-4-(4-fluor-phenyl)-2-isoxazol-3-yl-4-methyl-1,4-dihydro-pyrimidin-5-carbonsäure-methylester; (R)-6-((S)-2-Carboxy-4,4-difluor-2-methyl-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-2-Carboxy-4,4-difluor-pyrrolidin-1-ylmethyl)-4-(4-fluor-phenyl)-4-methyl-2-(5-methyl-thiazol-2-yl)-1,4-dihydro-pyrimidin-5-carbonsäure-methylester; (S)-6-((S)-2-Carboxy-4,4-difluor-pyrrolidin-1-ylmethyl)-4-(4-fluor-phenyl)-2-(5-fluor-thiophen-2-yl)-4-methyl-1,4-dihydro-pyrimidin-5-carbonsäure-methylester; (S)-6-((2S,4S)-2-Carboxy-4-fluor-pyrrolidin-1-ylmethyl)-4-(4-fluor-phenyl)-4-methyl-2-thiazol-2-yl-1,4-dihydro-pyrimidin-5-carbonsäure-methylester; 6-((S)-2-Carboxy-4,4-difluor-pyrrolidin-1-ylmethyl)-4-(4-fluor-phenyl)-4-methyl-2-(5-methyl-isoxazol-3-yl)-1,4-dihydro-pyrimidin-5-carbonsäure-methylester; (S)-6-((S)-2-Carboxy-4,4-difluor-pyrrolidin-1-ylmethyl)-4-(4-fluor-phenyl)-2-(3-fluor-thiophen-2-yl)-4-methyl-1,4-dihydro-pyrimidin-5-carbonsäure-methylester; (R)-6-((S)-2-Carboxy-4,4-difluor-pyrrolidin-1-ylmethyl)-4-(4-fluor-phenyl)-2-(3-fluor-thiophen-2-yl)-4-methyl-1,4-dihydro-pyrimidin-5-carbonsäure-methylester; (S)-6-((S)-2-Carboxy-4,4-difluor-pyrrolidin-1-ylmethyl)-4-(4-fluor-phenyl)-2-(4-fluor-thiophen-2-yl)-4-methyl-1,4-dihydro-pyrimidin-5-carbonsäure-methylester; (S)-6-{[Carboxymethyl-(2,2,2-trifluor-ethyl)-amino]-methyl}-4-(4-fluorphenyl)-4-methyl-2-thiazol-2-yl-1,4-dihydro-pyrimidin-5-carbonsäure-methylester; (S)-6-((S)-4,4-Difluor-2-methoxycarbonyl-pyrrolidin-1-ylmethyl)-4-(4-fluor-phenyl)-4-methyl-2-thiazol-2-yl-1,4-dihydro-pyrimidin-5-carbonsäure-methylester; (S)-6-[(S)-2-(2-Dimethylamino-ethoxycarbonyl)-4,4-difluor-pyrrolidin-1-ylmethyl]-4-(4-fluor-phenyl)-4-methyl-2-thiazol-2-yl-1,4-dihydro-pyrimidin-5-carbonsäure-methylester; (S)-6-(2-Carbamoyl-4,4-difluor-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-2-Carbamoyl-4,4-difluor-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-2-Dimethylcarbamoyl-4,4-difluor-pyrrolidin-1-ylmethyl)-4-(4-fluor-phenyl)-4-methyl-2-thiazol-2-yl-1,4-dihydro-pyrimidin-5-carbonsäure-methylester; 6-((S)-4,4-Difluor-2-methylcarbamoyl-pyrrolidin-1-ylmethyl)-4-(4-fluor-phenyl)-4-methyl-2-thiazol-2-yl-1,4-dihydro-pyrimidin-5-carbonsäure-methylester; (S)-6-((S)-4,4-Difluor-2-methansulfonylaminocarbonyl-pyrrolidin-1-ylmethyl)-4-(4-fluor-phenyl)-4-methyl-2-thiazol-2-yl-1,4-dihydro-pyrimidin-5-carbonsäure-methylester; (S)-6-[(S)-4,4-Difluor-2-(thiazol-2-ylcarbamoyl)-pyrrolidin-1-ylmethyl]-4-(4-fluor-phenyl)-4-methyl-2-thiazol-2-yl-1,4-dihydro-pyrimidin-5-carbonsäure-methylester; 4-(4-Fluor-phenyl)-6-((R)-3-hydroxymethyl-morpholin-4-ylmethyl)-4-methyl-2-thiazol-2-yl-1,4-dihydro-pyrimidin-5-carbonsäure-methylester; (S)-6-[(S)-4,4-Difluor-2-(1-hydroxy-1-methyl-ethyl)-pyrrolidin-1-ylmethyl]-4-(4-fluorphenyl)-4-methyl-2-thiazol-2-yl-1,4-dihydro-pyrimidin-5-carbonsäure-methylester; (S)-6-((S)-4,4-Difluor-2-hydroxymethyl-pyrrolidin-1-ylmethyl)-4-(4-fluor-phenyl)-4-methyl-2-thiazol-2-yl-1,4-dihydro-pyrimidin-5-carbonsäure-methylester; (S)-6-[4,4-Difluor-2-(3-hydroxy-propyl)-pyrrolidin-1 -ylmethyl] -4-(4-fluor-phenyl)-4-methyl-2-thiazol-2-yl-1,4-dihydro-pyrimidin-5-carbonsäure-methylester; (S)-6-[(S)-4,4-Difluor-2-(5-methyl-[1,3,4]oxadiazol-2-yl)-pyrrolidin-1-ylmethyl]-4-(4-fluor-phenyl)-4-methyl-2-thiazol-2-yl-1,4-dihydro-pyrimidin-5-carbonsäure-methylester; (S)-6-[(S)-4,4-difluor-2-(1H-tetrazol-5-yl)-pyrrolidin-1-ylmethyl]-4-(4-fluor-phenyl)-4-methyl-2-thiazol-2-yl-1,4-dihydro-pyrimidin-5-carbonsäure-methylester; und (S)-6-[(S)-4,4-Difluor-2-(3-methyl-[1,2,4]oxadiazol-5-yl)-pyrrolidin-1-ylmethyl]-4-(4-fluor-phenyl)-4-methyl-2-thiazol-2-yl-1,4-dihydro-pyrimidin-5-carbonsäure-methylester.

19. Ein Verfahren zur Herstellung einer Verbindung gemäß einem der Ansprüche 1 bis 18, umfassend die Umsetzung von
(a) einer Verbindung der Formel (A) 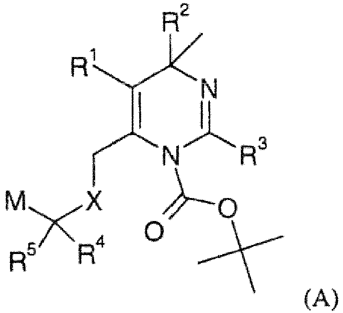 in Gegenwart einer Säure;
wobei R¹ bis R⁵, M und X wie in einem der Ansprüche 1 bis 17 definiert sind.

20. Eine Verbindung gemäß einem der Ansprüche 1 bis 18 zur Verwendung als therapeutischer Wirkstoff.

21. Ein Arzneimittel, umfassend eine Verbindung gemäß einem der Ansprüche 1 bis 18 und einen therapeutisch inerten Träger.

22. Die Verwendung einer Verbindung gemäß einem der Ansprüche 1 bis 18 zur Herstellung eines Medikaments zur Behandlung oder Vorbeugung einer Hepatitis-B-Virusinfektion.

23. Eine Verbindung gemäß einem der Ansprüche 1 bis 18 zur Verwendung bei der Behandlung oder Vorbeugung einer Hepatitis-B-Virusinfektion.

## Revendications

1. Composés de formule (I) 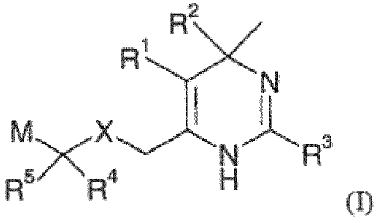 dans laquelle,
R¹ est un (alcoxy en C₁ à C₂)carbonyle ou un cyano ;
R² est phényle, qui est substitué avec un halogène ;
R³ est un thiazolyle, un thiényle, un imidazolyle, un isoxazolyle ou un pyridinyle ; qui est non substitué ou substitué avec un halogène ou un alkyle en C₁ à C₆ ;
X est un oxygène ou -NR⁷ ;
R⁴ et R⁵ sont choisis indépendamment parmi un hydrogène, un alkyle en C₁ à C₆ et trifluoroalkyle en C₁ à C₆ ; ou
R⁴ et R⁵, ensemble avec l'atome de carbone auquel ils sont liés, forment un cycloalkyle de 3 à 7 chaînons ; ou
quand X est -NR⁷, un parmi R⁴ et R⁵ est un hydrogène ou un alkyle en C₁ à C₆, et l'autre avec l'atome de carbone auquel il est lié et -NR⁷ forme un cycle pyrrolidinyle, morpholinyle ou pipéridinyle, ledit cycle étant non substitué ou substitué avec un fluoro ;
M est un (alcoxy en C₁ à C₆)carbonyle, un carboxy, un di(alkyle en C₁ à C₆)amino-(alcoxy en C₂ à C₆)carbonyle, un aminocarbonyle, un (alkyle en C₁ à C₆)amino-carbonyle, un di(alkyle en C₁ à C₆)aminocarbonyle, un (alkyle en C₁ à C₆)sulfonyl-aminocarbonyle, un 2-thiazolylaminocarbonyle, un hydroxy-C_{y}H_{2y}-, 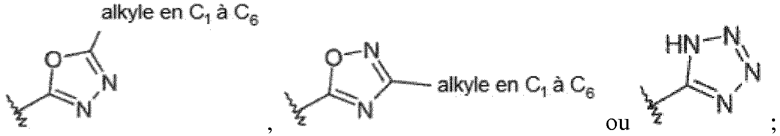
R⁷ est un alkyle en C₁ à C₆ ou un trifluoroalkyle en C₁ à C₆ ;
y est 1 à 6 ;
ou leurs sels pharmaceutiquement acceptables ou isomères de tautomérisme.

2. Composé selon la revendication 1, dans lequel,
R¹ est un (alcoxy en C₁ à C₂)carbonyle ou un cyano ;
R² est phényle, qui est substitué une fois ou deux fois avec un halogène ;
R³ est un 2-thiazolyle, qui est non substitué ou substitué une fois avec un alkyle en C₁ à C₆ ou un halogène ; ou un 2-thiényle ou un 2-pyridinyle, qui est substitué une fois avec un halogène ; ou un 2-imidazolyle, qui est substitué une fois avec un alkyle en C₁ à C₆ ; ou un 3-isoxazolyle, qui est non substitué ou substitué une fois avec un alkyle en C₁ à C₆ ;
X est un oxygène ou -NR⁷ ;
R⁴ et R⁵ sont choisis indépendamment parmi un hydrogène, un alkyle en C₁ à C₆ et un trifluoroalkyle en C₁ à C₆ ; ou
R⁴ et R⁵, ensemble avec l'atome de carbone auquel ils sont liés, forment un cycloalkyle de 3 à 7 chaînons ; ou
quand X est -NR⁷, un parmi R⁴ et R⁵ est un hydrogène ou un alkyle en C₁ à C₆, et l'autre avec l'atome de carbone auquel il est lié et -NR⁷ forme un morpholinyle ; ou un pyrrolidinyle ou un pipéridinyle, qui est substitué avec un fluoro ;
M est un (alcoxy en C₁ à C₆)carbonyle, un carboxy, un di(alkyle en C₁ à C₆)amino-(alcoxy en C₂ à C₆)carbonyle, un aminocarbonyle, un (alkyle en C₁ à C₆)amino-carbonyle, un di(alkyle en C₁ à C₆)aminocarbonyle, un (alkyle en C₁ à C₆)sulfonyl-aminocarbonyle, un 2-thiazolylaminocarbonyle, un hydroxy-C_{y}H_{2y}-, 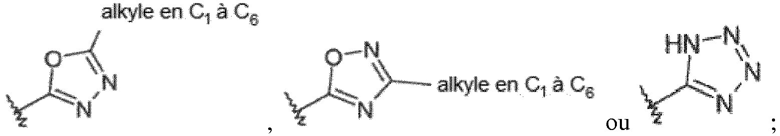
R⁷ est un alkyle en C₁ à C₆ ou un trifluoroalkyle en C₁ à C₆ ;
y est 1 à 6 ;
ou ses sels pharmaceutiquement acceptables ou isomères de tautomérisme.

3. Composé selon la revendication 1 ou 2, dans lequel
R¹ est un méthoxycarbonyle, un éthoxycarbonyle ou un cyano ;
R² est un phényle substitué une fois ou deux fois avec un fluoro ;
R³ est 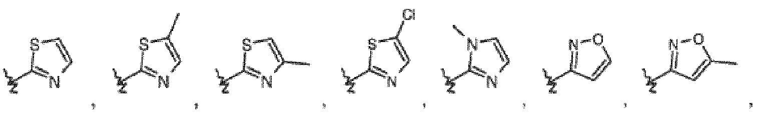 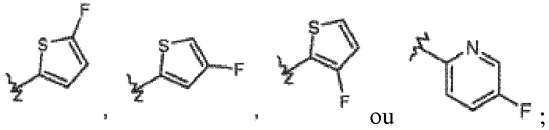
X est un oxygène ou -NR⁷;
R⁴ et R⁵ sont choisis indépendamment parmi un hydrogène, un méthyle et un trifluorométhyle ; ou
R⁴ et R⁵ ensemble avec l'atome de carbone auquel ils sont liés forment un cyclopropyle ; ou quand X est -NR⁷, un parmi R⁴ et R⁵ est un hydrogène ou un méthyle, et l'autre avec l'atome de carbone auquel il est lié et -NR⁷ forme 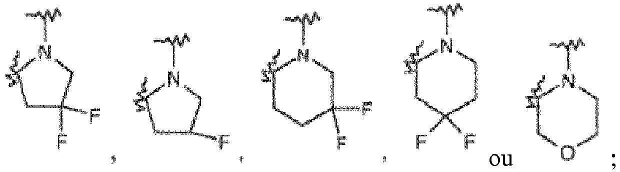
M est un méthoxycarbonyle, un carboxy, un diméthylaminoéthoxycarbonyle, un aminocarbonyle, un diméthylaminocarbonyle, un méthylaminocarbonyle, un méthyl-sulfonylaminocarbonyle, un 2-thiazolylaminocarbonyle, un hydroxyméthyle, un hydroxypropyle, 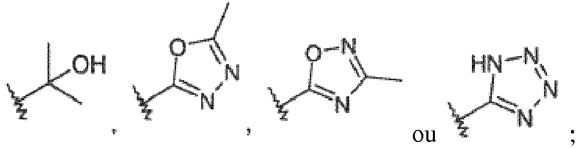
R⁷ est un méthyle ou un trifluoroéthyle ;
ou ses sels pharmaceutiquement acceptables ou isomères de tautomérisme.

4. Composé selon la revendication 1 ou 2, dans lequel,
R¹ est un (alcoxy en C₁ à C₂)carbonyle ;
R² est phényle qui est substitué une fois avec un halogène ;
R³ est un 2-thiazolyle ;
X est un oxygène ;
R⁴ et R⁵ sont choisis indépendamment parmi un hydrogène, un alkyle en C₁ à C₆ et un trifluoroalkyle en C₁ à C₆ ;
M est un (alcoxy en C₁ à C₆)carbonyle ou un carboxy.

5. Composé selon l'une quelconque des revendications 1 à 4, dans lequel
R¹ est un méthoxycarbonyle ;
R² est 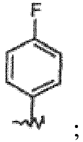
R³ est 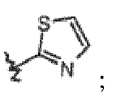
X est un oxygène ;
R⁴ et R⁵ sont choisis indépendamment parmi un hydrogène, un méthyle et un trifluorométhyle ;
M est un méthoxycarbonyle ou un carboxy.

6. Composé selon la revendication 1 ou 2, dans lequel
R¹ est un (alcoxy en C₁ à C₂)carbonyle
R² est un phényle qui est substitué une fois avec un halogène ;
R³ est un 2-thiazolyle ;
X est un -N-alkyle en C₁ à C₆ ou un -N-trifluoroalkyle en C₁ à C₆ ;
R⁴ est un hydrogène ;
R⁵ est un hydrogène ;
ou R⁴ et R⁵, ensemble avec l'atome de carbone auquel ils sont liés, forment un cycloalkyle de 3 à 7 chaînons ;
M est un carboxy.

7. Composé selon l'une quelconque des revendications 1, 2, 3 ou 6, dans lequel
R¹ est un méthoxycarbonyle ;
R² est 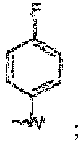
R³ est 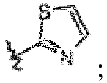
X est -NCH₃ ou 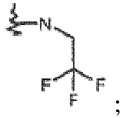
R⁴ est un hydrogène ;
R⁵ est un hydrogène ;
ou R⁴ et R⁵, ensemble avec l'atome de carbone auquel ils sont liés, forment un cyclopropyle ;
M est un carboxy.

8. Composé selon la revendication 1 ou 2, dans lequel
R¹ est un (alcoxy en C₁ à C₂)carbonyle ou un cyano ;
R² est un phényle qui est substitué une fois ou deux fois avec un halogène ;
R³ est un 2-thiazolyle ; ou un 2-pyridinyle, qui est substitué une fois avec un halogène ; ou un 2-imidazolyle, qui est substitué une fois avec un alkyle en C₁ à C₆ ; X est -NR⁷ ;
un parmi R⁴ et R⁵ est un hydrogène, et l'autre avec l'atome de carbone auquel il est lié et -NR⁷ forment ensemble un morpholinyle ;
M est un (alcoxy en C₁ à C₆)carbonyle, un carboxy ou un hydroxy-C_{y}H_{2y}- ; y est 1 à 6.

9. Composé selon l'une quelconque des revendications 1, 2, 3 ou 8, dans lequel
R¹ est un méthoxycarbonyle, un éthoxycarbonyle ou un cyano ;
R² est 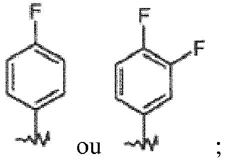
R³ est 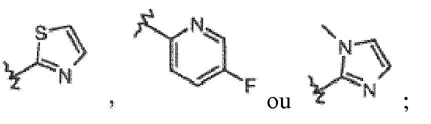
un parmi R⁴ et R⁵ est un hydrogène, et l'autre avec l'atome de carbone auquel il est lié et -NR⁷ forment 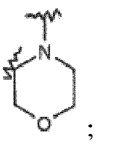
M est un méthoxycarbonyle, un carboxy ou un hydroxyméthyle.

10. Composé selon la revendication 1 ou 2, dans lequel
R¹ est un (alcoxy en C₁ à C₂)carbonyle ou un cyano ;
R² est un phényle qui est substitué une fois ou deux fois avec un halogène ;
R³ est un 2-thiazolyle, qui est non substitué ou substitué une fois avec un alkyle en C₁ à C₆ ou un halogène ; ou un 2-thiényle ou un 2-pyridinyle, qui est substitué une fois avec un halogène ; ou un 2-imidazolyle, qui est substitué une fois avec un alkyle en C₁ à C₆ ; ou un 3-isoxazolyle, qui est non substitué ou substitué une fois avec un alkyle en C₁ à C₆ ;
X est -NR⁷ ;
un parmi R⁴ et R⁵ est un hydrogène ou un alkyle en C₁ à C₆, et l'autre avec l'atome de carbone auquel il est lié et -NR⁷ forment ensemble un pyrrolidinyle ou un pipéridinyle, qui est substitué avec un fluoro ;
M est un (alcoxy en C₁ à C₆)carbonyle, un carboxy, un di(alkyle en C₁ à C₆)amino-(alcoxy en C₂ à C₆)carbonyle, un aminocarbonyle, un (alkyle en C₁ à C₆)amino-carbonyle, un di(alkyle en C₁ à C₆)aminocarbonyle, un (alkyle en C₁ à C₆)sulfonyl-aminocarbonyle, un 2-thiazolylaminocarbonyle, un hydroxy-C_{y}H_{2y}-, 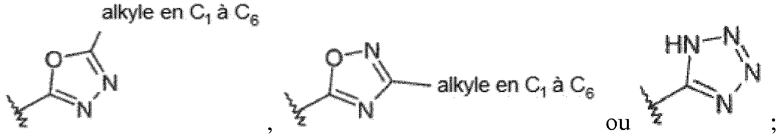
y est 1 à 6.

11. Composé selon l'une quelconque des revendications 1, 2, 3 ou 10, dans lequel
R¹ est un méthoxycarbonyle, un éthoxycarbonyle ou un cyano ;
R² est 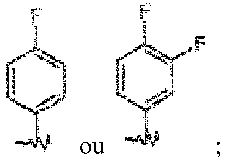
R³ est 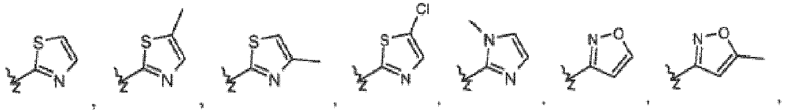 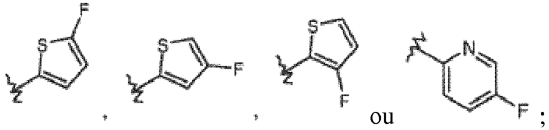
X est -NR⁷ ;
un parmi R⁴ et R⁵ est un hydrogène ou un méthyle, et l'autre avec l'atome de carbone auquel il est lié et -NR⁷ forment 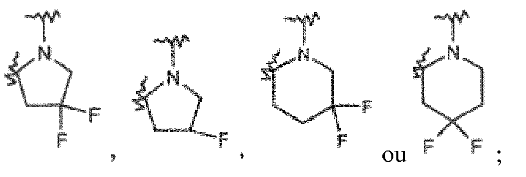
M est un méthoxycarbonyle, un carboxy, un diméthylaminoéthoxycarbonyle, un aminocarbonyle, un diméthylaminocarbonyle, un méthylaminocarbonyle, un méthyl-sulfonylaminocarbonyle, un 2-thiazolylaminocarbonyle, un hydroxyméthyle, un hydroxypropyle,

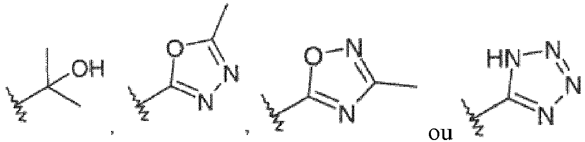

12. Composé selon la revendication 1 de formule (I') 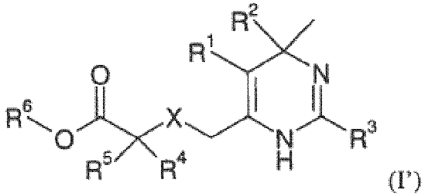 dans laquelle,
R¹ est un (alcoxy en C₁ à C₂)carbonyle ou un cyano ;
R² est un phényle, qui est substitué avec un halogène ;
R³ est un 2-thiazolyle qui est non substitué ou substitué avec un alkyle en C₁ à C₆ ou un 2-pyridinyle, qui est substitué avec un halogène ;
X est un oxygène ou -NR⁷ ;
R⁴ et R⁵ sont choisis indépendamment parmi un hydrogène, un alkyle en C₁ à C₆ et un trifluoroalkyle en C₁ à C₆ ; ou
R⁴ et R⁵, ensemble avec l'atome de carbone auquel ils sont liés, forment un cycloalkyle de 3 à 7 chaînons ; ou
quand X est -NR⁷, un parmi R⁴ et R⁵ est un hydrogène ou un alkyle en C₁ à C₆, et l'autre avec l'atome de carbone auquel il est lié et -NR⁷ forment ensemble un morpholinyle ; ou un pyrrolidinyle substitué avec un fluoro ;
R⁶ est un hydrogène ou un alkyle en C₁ à C₆ ;
R⁷ est un alkyle en C₁ à C₆ ;
ou ses sels pharmaceutiquement acceptables ou isomères de tautomérisme.

13. Composé selon la revendication 12, dans lequel
R¹ est un méthoxycarbonyle ou un cyano ;
R² est un phényle substitué une fois ou deux fois avec un fluoro ;
R³ est 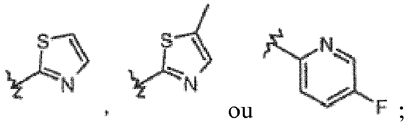
X est un oxygène ou -NR⁷;
R⁴ et R⁵ sont choisis indépendamment parmi un hydrogène, un méthyle et un trifluorométhyle ; ou
R⁴ et R⁵ ensemble avec l'atome de carbone auquel ils sont liés forment un cyclopropyle ; ou quand X est -NR⁷, un parmi R⁴ et R⁵ est un hydrogène ou un méthyle, et l'autre avec l'atome de carbone auquel il est lié et -NR⁷ forment 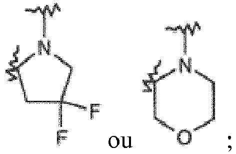
R⁶ est un hydrogène ou un méthyle ;
R⁷ est méthyle ;
ou ses sels pharmaceutiquement acceptables ou isomères de tautomérisme.

14. Composé selon la revendication 12, dans lequel
R¹ est un (alcoxy en C₁ à C₂)carbonyle ou un cyano ;
R² est un phényle qui est substitué avec un halogène ;
R³ est un 2-thiazolyle ou un 2-pyridinyle, qui est substitué avec un halogène ; X est -NR⁷ ;
un parmi R⁴ et R⁵ est un hydrogène ou un alkyle en C₁ à C₆, et l'autre avec l'atome de carbone auquel il est lié et -NR⁷ forment ensemble un morpholinyle ;
R⁶ est un hydrogène ou un alkyle en C₁ à C₆.

15. Composé selon l'une quelconque des revendications 12, 13 ou 14, dans lequel
R¹ est un méthoxycarbonyle ou un cyano ;
R² est 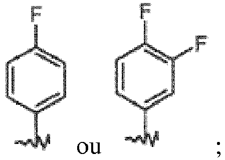
R³ est 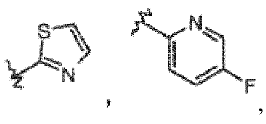
un parmi R⁴ et R⁵ est un hydrogène ou un méthyle, et l'autre avec l'atome de carbone auquel il est lié et -NR⁷ forment 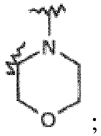
R⁶ est un hydrogène ou un méthyle.

16. Composé selon la revendication 12, dans lequel
R¹ est un (alcoxy en C₁ à C₂)carbonyle ou un cyano ;
R² est un phényle qui est substitué avec un halogène ;
R³ est un 2-thiazolyle qui est non substitué ou substitué avec un alkyle en C₁ à C₆ ou un 2-pyridinyle, qui est substitué avec un halogène ;
X est -NR⁷ ;
un parmi R⁴ et R⁵ est un hydrogène ou un alkyle en C₁ à C₆, et l'autre avec l'atome de carbone auquel il est lié et -NR⁷ forment ensemble un pyrrolidinyle qui est substitué avec un fluoro ;
R⁶ est un hydrogène ou un alkyle en C₁ à C₆.

17. Composé selon l'une quelconque des revendications 12, 13 et 16, dans lequel
R¹ est un méthoxycarbonyle ou un cyano ;
R² est 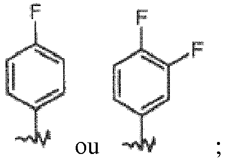
R³ est 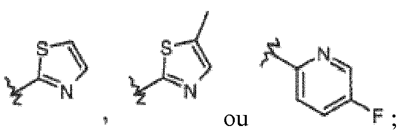
X est -NR⁷ ;
un parmi R⁴ et R⁵ est un hydrogène ou un méthyle, et l'autre avec l'atome de carbone auquel il est lié et NR⁷ forment 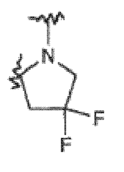
R⁶ est un hydrogène ou méthyle.

18. Composé selon l'une quelconque des revendications 1 à 17, choisi parmi le 4-(4-fluoro-phényl)-6-(1-méthoxycarbonyl-1-méthyl-éthoxyméthyl)-4-méthyl-2-thiazol-2-yl-1,4-dihydro-pyrimidine-5-carboxylate de méthyle ; le 6-(1-carboxy,-2,2,2-trifluoro-éthoxyméthyl)-4-(4-fluoro-phényl)-4-méthyl-2-thiazol-2-yl-1,4-dihydro-pyrimidine-5-carboxylate de méthyle; le 6-{[(1-carboxy-cyclopropyl)-méthyl-amino]-méthyl}-4-(4-fluoro-phényl)-4-méthyl-2-thiazol-2-yl-1,4-dihydro-pyrimidine-5-carboxylate de méthyle ; l'acide 4-[6-(4-fluoro-phényl)-5-méthoxy-carbonyl-6-méthyl-2-thiazol-2-yl-3,6-dihydro-pyrimidin-4-ylméthyl]-morpholine-3-carboxylique ; le 4-[6-(4-fluoro-phényl)-5-méthoxycarbonyl-6-méthyl-2-thiazol-2-yl-3,6-dihydro-pyrimidin-4-ylméthyl]-morpholine-(S)-3-carboxylate de méthyle ; l'acide 4-[6-(4-fluoro-phényl)-5-méthoxycarbonyl-6-méthyl-2-thiazol-2-yl-3,6-dihydro-pyrimidin-4-ylméthyl]-moipholine-(S)-3-carboxylique ; l'acide (S)-4-[6-(4-fluoro-phényl)-5-méthoxycarbonyl-6-méthyl-2-thiazol-2-yl-3,6-dihydro-pyrimidin-4-ylméthyl]-morpholine-(S)-3-carboxylique ; l'acide (S)-4-[6-(4-fluoro-phényl)-5-méthoxycarbonyl-6-méthyl-2-thiazol-2-yl-3,6-dihydro-pyrimidin-4-ylméthyl]-morpholine-(R)-3-carboxylique ; l'acide 4-[6-(4-fluoro-phényl)-2-(5-fluoro-pyridin-2-yl)-5-méthoxycarbonyl-6-méthyl-3,6-dihydro-pyrimidin-4-ylméthyl]-morpholine-(R)-3-carboxylique ; l'acide 4-[6-(4-fluoro-phényl)-2-(5-fluoro-pyridin-2-yl)-5-méthoxycarbonyl-6-méthyl-3,6-dihydro-pyumidin-4-ylméthyl]-morpholine-(S)-carboxylique ; le 6-(2-(S)-carboxy-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le 6-(2-(R)-carboxy-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-2-carboxy-4,4-difluoro-pyrrolidin-1-ylméthyl)-4-(3,4-difluoro-phényl)-4-méthyl-2-thiazol-2-yl-1,4-dihydro-pyrimidine-5-carboxylate de méthyle ; le 4-[(S)-6-(3,4-difluoro-phényl)-5-méthoxycarbonyl-6-méthyl-2-thiazol-2-yl-3,6-dihydro-pyumidin-4-ylméthyl]-moipholine-3-carboxylate de méthyle ; l'acide 4-[6-(3,4-difluoro-phényl)-5-méthoxycarbonyl-6-méthyl-2-thiazol-2-yl-3,6-dihydro-pyrimidin-4-yl-méthyl]-morpholine-3-carboxylique ; le 6-(4,4-difluoro-2-méthoxycarbonyl-pyrrolidin-1-ylméthyl)-4-(4-fluoro-phényl)-2-(5-fluoro-pyridin-2-yl)-4-méthyl-1,4-dihydro-pyrimidine-5-carboxylate de méthyle ; le 6-(2-carboxy-4,4-difluoro-pyrrolidin-1-ylméthyl)-4-(4-fluoro-phényl)-2-(5-fluoro-pyridin-2-yl)-4-méthyl-1,4-dihydro-pyrimidine-5-carboxylate de méthyle ; le 6-((S)-2-carboxy-4,4-difluoro-pyrrolidin-1-ylméthyl)-4-(4-fluoro-phényl)-4-méthyl-2-(5-méthyl-thiazol-2-yl)-1,4-dihydro-pyrimidine-5-carboxylate de méthyle ; le 6-(2-carboxy-4,4-difluoro-2-méthyl-pyrrolidin-1-ylméthyl)-4-(4-fluoro-phényl)-4-méthyl-2-thiazol-2-yl-1,4-dihydro-pyrimidine-5-carboxylate de méthyle ; l'acide (S)-1-[(S)-5-cyano-6-(4-fluoro-phényl)-6-méthyl-2-thiazol-2-yl-3,6-dihydro-pyrimidin-4-ylméthyl]-4,4-difluoro-pyrrolidine-2-carboxylique ; l'acide (S)-4-[5-cyano-6-(3,4-difluoro-phényl)-6-méthyl-2-thiazol-2-yl-3,6-dihydro-pyrimidin-4-ylméthyl]-morpholine-3-carboxylique ; l'acide (S)-1-[(S)-5-cyano-6-(3,4-difluoro-phényl)-6-méthyl-2-thiazol-2-yl-3,6-dihydro-pyrimidin-4-ylméthyl]-4,4-difluoro-pyrrolidine-2-carboxylique ; le (S)-6-((S)-2-carboxy-4,4-difluoro-pyrrolidin-1-ylméthyl)-4-(3,4-difluoro-phényl)-4-méthyl-2-thiazol-2-yl-1,4-dihydro-pyrimidine-5-carboxylate d'éthyle ; le (S)-6-(2-carboxy-5,5-difluoro-pipéridin-1-ylméthyl)-4-(4-fluoro-phényl)-4-méthyl-2-thiazol-2-yl-1,4-dihydro-pyrimidine-5-carboxylate de méthyle ; le (S)-6-(2-carboxy-4,4-difluoro-pipéridin-1-ylméthyl)4-(4-fluoro-phényl)-4-méthyl-2-thiazol-2-yl-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-4-méthyl-2-(1-méthyl-1H-imidazol-2-yl)-1,4-dihydro-pyrimidine-5-carboxylate de méthyle ; le (R)-6-((S)-2-carboxy-4,4-difluoro-pyrrolidin-1-ylméthyl)-4-(3,4-difluoro-phényl)-4-méthyl-2-thiazol-2-yl-1,4-dihydro-pyrimidine-5-carboxylate d'éthyle ; l'acide (S)-4-[6-(3,4-difluoro-phényl)-5-éthoxycarbonyl-6-méthyl-2-thiazol-2-yl-3,6-dihydro-pyrimidin-4-ylméthyl]-moipholine-3-carboxylique ; l'acide (S)-4-[(S)-6-(4-fluoro-phényl)-5-méthoxycarbonyl-6-méthyl-2-(1-méthyl-1H-imidazol-2-yl)-3,6-dihydro-pyrimidin-4-ylméthyl]-morpholine-3-carboxylique ; le (R)-6-((S)-2-carboxy-4,4-difluoro-pymolidin-1-ylméthyl)-4-(4-fluoro-phényl)-4-méthyl-2-(4-méthyl-thiazol-2-yl)-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-4-méthyl-2-(4-méthyl-thiazol-2-yl)-1,4-dihydro-pyrimidine-5-carboxylate de méthyle ; le (S)-6-((S)-2-carboxy-4,4-difluoro-pyrrolidin-1-ylméthyl)-2-(5-chloro-thiazol-2-yl)-4-(4-fluoro-phényl)-4-méthyl-1,4-dihydro-pyrimidine-5-carboxylate de méthyle ; le (S)-6-((2S,4R)-2-carboxy-4-fluoro-pyrrolidin-1-ylméthyl)-4-(4-fluoro-phényl)-4-méthyl-2-thiazol-2-yl-1,4-dihydro-pyrimidine-5-carboxylate de méthyle ; le 6-((S)-2-carboxy-4,4-difluoro-pymolidin-1-ylméthyl)-4-(4-fluoro-phényl)-2-isoxazol-3-yl-4-méthyl-1,4-dihydro-pyrimidine-5-carboxylate de méthyle ; le (R)-6-((S)-2-carboxy-4,4-difluoro-2-méthyl-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-4-méthyl-2-(5-méthyl-thiazol-2-yl)-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-2-(5-fluoro-thiophén-2-yl)-4-méthyl-1,4-dihydro-pyrimidine-5-carboxylate de méthyle ; le (S)-6-((2S,4S)-2-carboxy-4-fluoro-pyrrolidin-1-ylméthyl)-4-(4-fluoro-phényl)-4-méthyl-2-thiazol-2-yl-1,4-dihydro-pyrimidine-5-carboxylate de méthyle ; le 6-((S)-2-carboxy-4,4-difluoro-pyrrolidin-1-ylméthyl)-4-(4-fluoro-phényl)-4-méthyl-2-(5-méthyl-isoxazol-3-yl)-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-2-(3-fluoro-thiophén-2-yl)-4-méthyl-1,4-dihydro-pyrimidine-5-carboxylate de méthyle ; le (R)-6-((S)-2-carboxy-4,4-difluoro-pyrrolidin-1-ylméthyl)-4-(4-fluoro-phényl)-2-(3-fluoro-thiophén-2-yl)-4-méthyl-1,4-dihydro-pyrimidine-5-carboxylate de méthyle ; le (S)-6-((S)-2-carboxy-4,4-difluoro-pyrrolidin-1-ylméthyl)-4-(4-fluoro-phényl)-2-(4-fluoro-thiophén-2-yl)-4-méthyl-1,4-dihydro-pyrimidine-5-carboxylate de méthyle; le (S)-6-{[carboxyméthyl-(2,2,2-tiifluoro-éthyl)-amino]-méthyl}-4-(4-fluoro-phényl)-4-méthyl-2-thiazol-2-yl-1,4-dihydro-pyrimidine-5-carboxylate de méthyle ; le (S)-6-((S)-4,4-difluoro-2-méthoxycarbonyl-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-[(S)-2-(2-diméthyl-amino-éthoxycarbonyl)-4,4-difluoro-pyrrolidin-1-ylméthyl]-4-(4-fluoro-phényl)-4-méthyl-2-thiazol-2-yl-1,4-dihydro-pyrimidine-5-carboxylate de méthyle ; le (S)-6-(2-carbamoyl-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-2-carbamoyl-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-2-diméthylcarbamoyl-4,4-difluoro-pyrrolidin-1-ylméthyl)-4-(4-fluoro-phényl)-4-méthyl-2-thiazol-2-yl-1,4-dihydro-pyrimidine-5-carboxylate de méthyle ; le 6-((S)-4,4-difluoro-2-méthylcarbamoyl-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-((S)-4,4-difluoro-2-méthanesulfonylaminocarbonyl-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-[(S)-4,4-difluoro-2-(thiazol-2-ylcarbamoyl)-pyrrolidin-1-yl-méthyl]-4-(4-fluoro-phényl)-4-méthyl-2-thiazol-2-yl-1,4-dihydro-pyrimidine-5-carboxylate de méthyle ; le 4-(4-fluoro-phényl)-6-((R)-3-hydroxyméthyl-morpholin-4-ylméthyl)-4-méthyl-2-thiazol-2-yl-1,4-dihydro-pyrimidine-5-carboxylate de méthyle ; le (S)-6-[(S)-4,4-difluoro-2-(1-hydroxy-1-méthyl-éthyl)-pyrrolidin-1-ylméthyl]-4-(4-fluoro-phényl)-4-méthyl-2-thiazol-2-yl-1,4-dihydro-pyrimidine-5-carboxylate de méthyle ; le (S)-6-((S)-4,4-difluoro-2-hydroxyméthyl-pyrrolidin-1-ylméthyl)-4-(4-fluoro-phényl)-4-méthyl-2-thiazol-2-yl-1,4-dihydro-pyrimidine-5-carboxylate de méthyle ; le (S)-6-[4,4-difluoro-2-(3-hydroxy-propyl)-pyrrolidin-1-ylméthyl]-4-(4-fluoro-phényl)-4-méthyl-2-thiazol-2-yl-1,4-dihydro-pyrimidine-5-carboxylate de méthyle ; le (S)-6-[(S)-4,4-difluoro-2-(5-méthyl-[1,3,4]oxadiazol-2-yl)-pyrrolidin-1-ylméthyl]-4-(4-fluoro-phényl)-4-méthyl-2-thiazol-2-yl-1,4-dihydro-pyrimidine-5-carboxylate de méthyle ; le (S)-6-[(S)-4,4-difluoro-2-(1H-tétrazol-5-yl)-pyrrolidin-1-ylméthyl]-4-(4-fluoro-phényl)-4-méthyl-2-thiazol-2-yl-1,4-dihydro-pyrimidine-5-carboxylate de méthyle ; et le (S)-6-[(S)-4,4-difluoro-2-(3-méthyl-[1,2,4]oxadiazol-5-yl)-pyrrolidin-1-ylméthyl]-4-(4-fluoro-phényl)-4-méthyl-2-thiazol-2-yl-1,4-dihydro-pyrimidine-5-carboxylate de méthyle.

19. Procédé de préparation d'un composé selon l'une quelconque des revendications 1 à 18, comprenant la réaction
(a) d'un composé de formule (A) 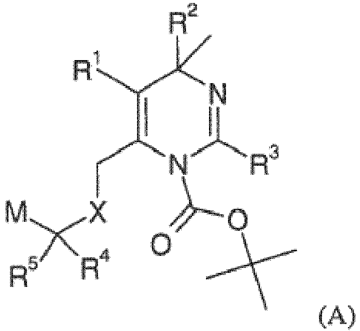 en présence d'un acide ;
dans laquelle R¹ à R⁵, M et X sont tels que définis dans l'une quelconque des revendications 1 à 17.

20. Composé selon l'une quelconque des revendications 1 à 18, pour son utilisation comme substance thérapeutiquement active.

21. Composition pharmaceutique comprenant un composé selon l'une quelconque des revendications 1 à 18 et un support thérapeutiquement inerte.

22. Utilisation d'un composé selon l'une quelconque des revendications 1 à 18, pour la préparation d'un médicament destiné au traitement ou à la prophylaxie d'une infection par le virus de l'hépatite B.

23. Composé selon l'une quelconque des revendications 1 à 18, pour son utilisation dans le traitement ou la prophylaxie d'une infection par le virus de l'hépatite B.
